# DEMANDE DE BREVET EUROPEEN

(11) **EP 2 295 428 A2**
(43) Date de publication de la demande: **16.03.2011**
(21) Numéro de dépôt: 10182543.8
(22) Date de dépôt: 26.07.2007
(51) Int. Cl.: C07D 401/14, C07D 401/04, C07D 403/04, A61K 31/501

(54) **Composés pyridaziniques et pyrroliques liénaires, procédés d'obtention et applications**

(30) Priorité: 26.07.2006 FR 0606841
(62) Demande divisionnaire de: 07823345.9
(71) Demandeur: Centre National de la Recherche Scientifique (CNRS), 75794 Paris Cedex 16 (FR)
(72) Inventeur: Dubreuil, Didier Max, 44710, Port Saint Pere (FR); Pipelier, Muriel, Geneviève, 44300, Nantes (FR); Pradere, Jean-Paul, 44880, Sautron (FR); Bakkali, Hicham, 44300, Nantes (FR); Thobie, Christine, Genviève, Juliette, 44119, Treillieres (FR); Leonel, Eric, Frédéric, 92320, Chatillon (FR); Nedelec, Jean-Yves, Joseph, Parie, 94240, l'Hay-les roses (FR); Sengmany, Stéphane, 94400, Vitry sur Seine (FR); Delaunay, Thierry, 72300, Sable-sur-Sarthe (FR); Tabatchnik, Alexandra, 44500, La Baule (FR)
(74) Mandataire: Peaucelle, Chantal

(57) **Abrégé**

La présente invention est relative à des composés pyridaziniques linéaires et plus particulièrement à ceux de ces composés qui sont oligopyridaziniques, à leurs procédés d'obtention, à leurs applications, ainsi qu'à leur réduction en pyrroles et aux applications des composés pyrroliques, pyridazinyl-pyrroliques et oligopyrroliques obtenus. L'invention vise en particulier les applications en tant que médicaments, notamment pour traiter des pathologies telles que le cancer, les infections bactériennes ou parasitaires, ainsi que les applications dans le domaine des matériaux, de l'environnement, de l'électronique et de l'optique.

## Description

La présente invention est relative à des composés pyridaziniques linéaires et plus particulièrement à ceux de ces composés qui sont oligopyridaziniques, à leurs procédés d'obtention, à leurs applications, ainsi qu'à leur régression en pyrroles et aux applications des composés pyrroliques, pyridazinyl-pyrroliques et oligopyrroliques obtenus. Les termes « oligopyridazine » et « oligopyrrole » désignent des composés comportant une pluralité de cycles azotés adjacents.

Depuis la fin des années 80, la chimie de coordination des composés azotés connaît un essor important en raison de la diversité des propriétés chimiques et catalytiques des complexes organométalliques contenant dans leur sphère de coordination une ou plusieurs fonctions azotées. Cette diversité est essentiellement liée à celle des fonctions azotées impliquées dans ces complexes : amine, imine, nitrile, azide, ...

La chimie de coordination joue un rôle fondamental dans la chimie supramoléculaire et, dans ce domaine, les oligopyridines ont, les premières, attiré une attention particulière. Les oligopyridines sont des ligands polydentés qui peuvent aussi être classés selon le nombre d'atomes d'azote participant à la chélation du métal dans le complexe : bidentates (bipyridines), tridentates (terpyridines), tétradentates (quaterpyridines), etc., de structure

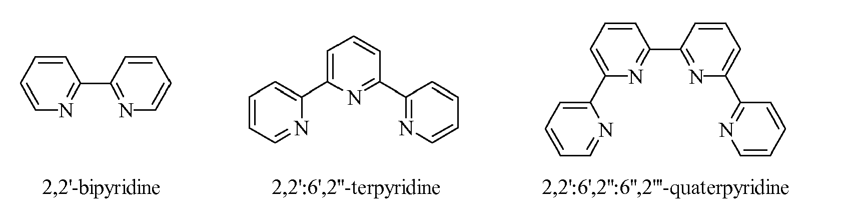

Les 2,2'-bipyridines sont depuis longtemps les ligands les plus utilisés en chimie de coordination, notamment lorsqu'ils expriment des propriétés d'induction asymétrique liées à la présence de groupements induisant un facteur de chiralité. Plus récemment, les 2,2': 6',2"-terpyridines (tpy) ont ouvert un champ d'investigation par l'expression de sites polydentates favorisant la formation de complexes avec des métaux de transition de degré d'oxydation supérieur. Cette propriété a été exploitée, par exemple, pour l'oxydation d'alcools et la carbonylation des composés aromatiques. Plus récemment encore, la chimie de ce type de ligands polydentates a été développée pour l'activation catalytique dans le cadre de la dépollution de déchets radioactifs.

Mis à part les structures hétérogènes constituées d'unités pyridiniques et pyrimidiniques, qui expriment une variété de sites de coordination polydentés, relativement peu d'investigations ont concerné des ligands oligohétérocycliques incorporant des unités diaziniques.

Des exemples de telles structures ligands sont donnés ci-dessous :

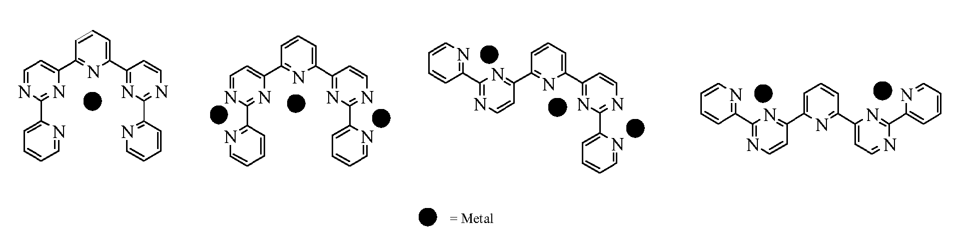

A l'exemple des travaux mentionnés sur les ligands bidentés 3,6-bis(pyridin-2-yl)-pyridazines **80** (Hoogenboom, R. et al., Eur. J. Org. Chem., 2003, p.4887 ; voir schéma ci-dessous), les ligands polydentés à base d'hétérocycles pyridaziniques n'ont été développés que très récemment en raison de leurs approches synthétiques délicates, bien que leur potentiel dans le domaine de la chimie de coordination apparaisse maintenant évident.

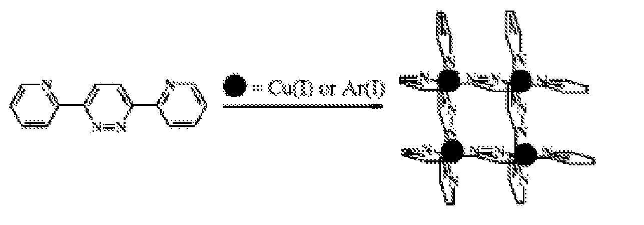

La 6,6'-bis(6-méthyl-pyridin-2-yl)-3,3'-bipyridazine **2** a par ailleurs montré un fort potentiel d'organisation supramoléculaire en présence de divers métaux, tel que l'argent(I) (Baxter, P. N. W. ; Lehn, J.-M., Fisher,J. ; Youinou, M, -T Angew. Chem. 1994, 106, 2432).

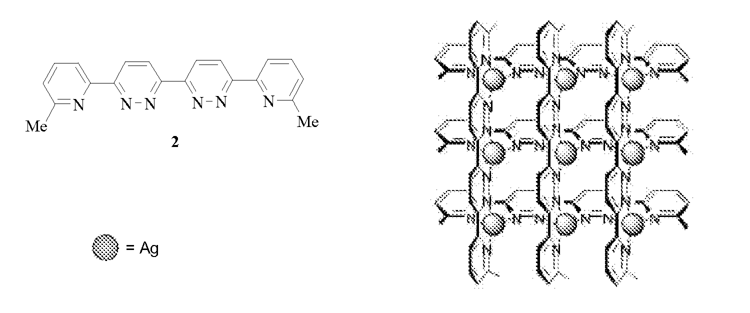

En 2000, les mêmes auteurs, exploitant les réactions d'homocouplage de bipyridazines halogénées, ont réalisé la synthèse d'un tétramère pyridazinique à partir d'un précurseur dichloro-bipyridazine **17** (Baxter, P. N. W ; Lehn, J. -M ; Baum, G. ; Fenske, D. Chem Eur. J. 2000, 6, 4510).

Comme son homologue dimérique, ce ligand α,α'-tétrapyridazinique possède une géométrie linéaire et ne peut donc exprimer que des enchaînements de sites de coordination poly-bidentés pour une même molécule. En présence d'argent, ce tétramère (quaterpyridazinique) conduit préférentiellement à un arrangement supramoléculaire de type grille carrée, comme c'est le cas pour la bipyridazine **2.** Cependant, l'élongation de la chaîne pyridazinique permet également dans ce cas un auto-assemblage de quatre monomères conduisant à une organisation hélicoïdale formée d'un tétramère en équilibre avec la grille carrée.

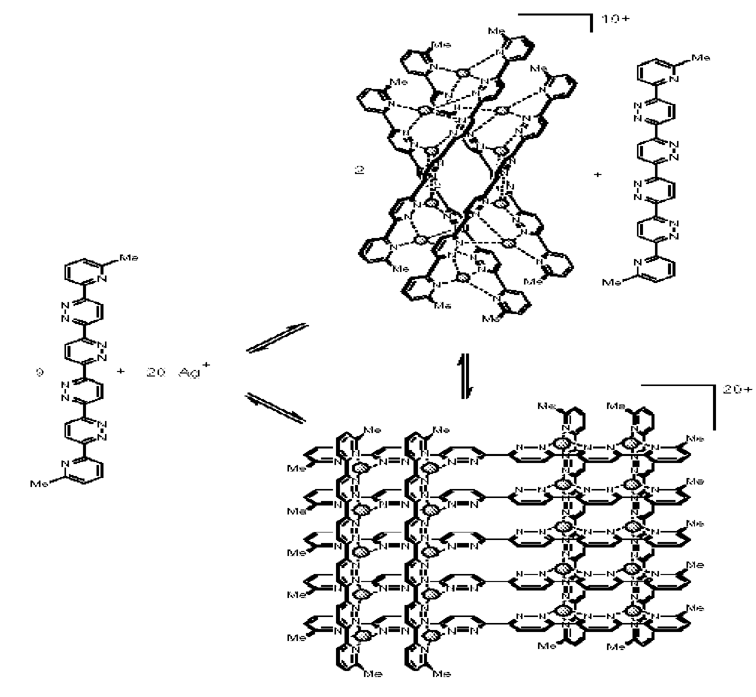

Au vu du fort potentiel des composés ci-dessus décrits et de l'absence de voie de synthèse généralisable vers ces composés et leurs analogues, qui par ailleurs, sont peu nombreux, les inventeurs ont décidé de tenter d'ouvrir la voie à une nouvelle génération de ligands pyridaziniques et pyrroliques.

Or, lors de travaux antérieurs, les inventeurs avaient mis au point une méthode de réduction électrochimique permettant de réduire des composés monopyridaziniques en monopyrroles (Manh G.T. et al, Electrochimica Acta, 2002, 2833).
Les inventeurs ont donc posé le postulat qu'une réduction électrochimique de composés oligopyridaziniques pouvait être réalisable dans des conditions à déterminer, et ce malgré la présence de plusieurs cycles pyridaziniques adjacents, susceptibles de modifier considérablement la structure et les propriétés électroniques de la molécule.

Afin de valider ce postulat, les inventeurs ont dû dans un premier temps mettre au point des voies de synthèse des composés oligopyridaziniques. Ces voies de synthèses mises au point sont multiples. L'une d'entre elles en particulier est relative à une optimisation de la voie de synthèse proposée par Lehn et al., dans la publication mentionnée ci-avant. Cette dernière synthèse est purement organique, mais d'autres ont été obtenues par voie électrochimique.

Ces recherches de synthèse chimiques ont par ailleurs permis de préparer de nouveaux composés bispyridinyl-pyridazine substitués, en particulier des composés dissymétriques.

Une fois les composés oligopyridaziniques préparés, la réduction en oligopyrroles a pu être tentée. De manière inattendue, cette réduction est non seulement effective dans des conditions spécifiques mises au point et optimisées par les inventeurs, mais en outre, elle ne donne pas lieu à une cyclisation entre les résidus pyridaziniques ou à toutes autres réactions secondaires potentielles. De plus, la réduction peut aussi avoir lieu cycle pyridazinique par cycle pyridazinique permettant d'obtenir un seul ou plusieurs site(s) de réduction sur la molécule, ouvrant ainsi la voie à la préparation de composés mixtes pyridazinyl-pyrroliques.

Les inventeurs ont également cherché à identifier les applications biologiques potentielles de ces nouveaux composés. Ces composés se sont alors révélés avoir des propriétés thérapeutiques de grand intérêt, en particulier anti-parasitaires, anti-cancéreuses et antibactériennes.

La présente invention est donc relative à des enchaînements pyridaziniques linéaires, plus particulièrement des bispyridinyl-pyridazines substituées et des oligopyridazines, à leurs procédés d'obtention et à leurs applications ; au procédé électrochimique de réduction des oligopyridazines en oligopyrroles ; ainsi qu'aux pyrroles obtenus, plus particulièrement les bispyridinyl-pyrroles et les oligopyrroles et leurs applications.

Selon un premier aspect, l'invention est relative à des composés de formule 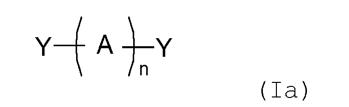 dans laquelle
- si n=1
   - si A représente un groupement de formule 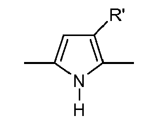 dans laquelle R' représente un hydrogène, une chaîne alkyle, hydroxyalkyle, alkylamine, alkyloxy de 1 à 6 carbones, un groupement -COOH, -COOR1, -CONH₂, -CONHR1, dans lesquels R1 est une chaîne alkyle de 1 à 6 carbones,
   - les groupements Y, identiques ou différents, représentent un groupement de formule 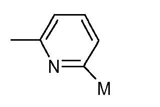 dans lequel M représente un hydrogène, un halogène, une chaîne alkyle, hydroxyalkyle, alkylamine ou alkyloxy de 1 à 6 carbones, un groupement -COOH, -COOR1, -CONH₂, -CONHR1, dans lesquels R1 est tel que défini ci-avant,
   - si A représente un groupement de formule 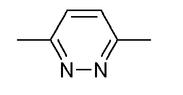
   - les groupements Y, identiques, représentent un groupement de formule 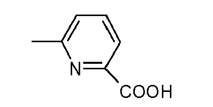 ou, les groupements Y, différents, représentent un groupement de formule 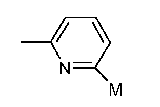 dans lequel M représente un hydrogène, un halogène, une chaîne alkyle, hydroxyalkyle, alkylamine ou alkyloxy de 1 à 6 carbones, un groupement -COOH, -COOR1, -CONH₂, -CONHR1, dans lesquels R1 est tel que défini ci-avant,
- si n est un entier compris entre 2 et 4 bornes incluses,
   - les groupements A, identiques ou différents, représentent un groupement 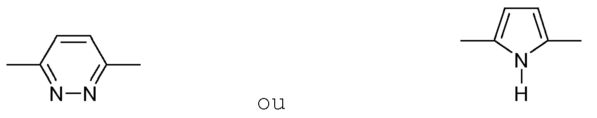
   - les groupements Y, identiques ou différents, représentent un halogène, un hydroxy, un mercapto, une chaîne alkyle, hydroxyalkyle, alkylamine ou alkyloxy de 1 à 6 carbones, éventuellement cyclique, un groupement -COOH, -COOR1, - CONH₂, -CONHR1, dans lesquels R1 est tel que défini précédemment, ou un groupement choisi parmi les groupes suivants : 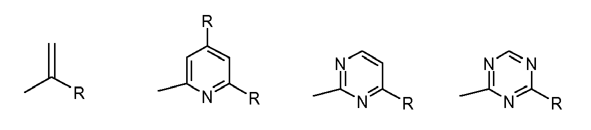 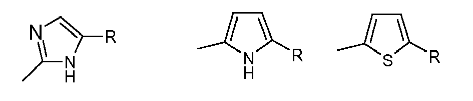 dans lesquels les groupes R, identiques ou différents, représentent un hydrogène, une chaîne alkyle ou alkyloxy de 1 à 6 carbones, un groupement -COOH, -COOR1, -CONH₂, - CONHR1, dans lesquels R1 est tel que défini ci- avant,
   à l'exception des composés suivants :
- 2,5-bis(pyridin-2-yl)pyrrole,
- 6,6'-bis(6-méthyl-pyridin-2-yl)-3,3'-bipyridazine,
- 6,6"'-bis-(6-méthyl-pyridin-2-yl)-[3,3':6',6":3",3"']quaterpyridazine,
- 6,6'-dimethoxy-3,3'-bipyridazine,
- 6,6'-dichloro-3,3'-bipyridazine.

La préparation des composés bispyridinyl-mono et oligo pyridazines sera décrite ci-après. Les composés mono et oligopyrroliques ou pyridazinyl-pyrroliques correspondants sont obtenus par une optimisation du procédé de réduction décrit dans Manh G.T. et al, Electrochimica Acta, 2002, 2833.

Les composés monopyrroliques substitués par le groupement R' sont également préparés par réduction électrochimique, à partir de composés pyridaziniques substitués par R' déjà décrits dans l'état de la technique. Dans ce cas, ces composés pyridaziniques sont obtenus classiquement par une réaction de Diels-Alder entre une bipyridyl-tétrazine et un acétylène (voir par exemple, Hoogenboom et al., cité plus haut).

En revanche, certaines monopyridazines et les oligopyridazines ainsi que les oligopyrroles correspondants sont intégralement préparés à l'aide des procédés selon l'invention, décrits ci-après.

Préférentiellement, les chaînes alkyles, hydroxyalkyles, alkylamines et alkyloxy sont des méthyles, hydroxyméthyles, méthylamines et méthoxy, et les groupements A sont identiques.

L'invention vise plus particulièrement les composés dans lesquels, lorsque n=1, M représente un hydrogène, un halogène, une chaîne alkyle de 1 à 6 carbones ou un groupement -COOH, et préférentiellement les composés suivants :
- 3-(2-carboxy-pyridin-6-yl)-6-(pyridin-2-yl)-pyridazine,
- 3,6-bis(2-carboxy-pyridin-6-yl)-pyridazine,
- 3-(6-méthyl-pyridin-2-yl)-6-(pyridin-2-yl)-pyridazine,
- 3-(2-bromo-pyridin-6-yl)-6-(pyridin-2-yl)-pyridazine.

Ces composés sont plus amplement décrits dans les exemples qui suivent, en particulier, les exemples 6-7, 9-11.

Lorsque n=2, les groupements Y, identiques ou différents, sont préférentiellement des groupements 2-pyridinyles éventuellement substitués ou des groupements -C(CH₂)OR1, dans lesquels R1 est une chaîne alkyle de 1 à 6 carbones, de préférence éthyle.

Les composés suivants sont par ailleurs spécifiquement décrits dans les exemples 1 à 5, 8 :
- 5,5'-bis(6-méthyl-pyridin-2-yl)-2,2'-bipyrrole,
- 6,6'-di-(1-éthoxyvinyl)-3,3'-bipyridazine,
- 6,6'-bis(pyridin-2-yl)-3,3'-bipyridazine,
- 5,5'-bis(pyridin-2-yl)-2,2'-bipyrrole,
- 3-[5-(6-méthyl-pyridin-2-yl)-pyrrol-2-yl]-6-(6-méthyl-pyridin-2-yl)-pyridazine,
- 6-(pyridin-2-yl)-3-((5-pyridin-2-yl)-pyrrol-2-yl)-pyridazine,
- 6,6'-bis(4,6-diméthylpyridin-2-yl)-3,3'-bipyridazine,
- 5,5'-bis(4,6-diméthylpyridin-2-yl)-2,2'-bipyrrole),
- 6-(4,6-dimethylpyridin-2-yl)-3-((5-(4,6-diméthylpyridin-2-yl)-pyrrol-2-yl)-pyridazine).

Dans toute la demande, les termes « oligopyridazines » et « composés oligopyridaziniques » d'une part, « oligopyrroles » et « composés oligopyrroliques » d'autre part, sont utilisés indifféremment. Par ces termes, on désigne des composés comportant une pluralité de cycles adjacents, de préférence 2 à 4 cycles.

Selon un second aspect, l'invention est relative aux procédés d'obtention des composés décrits ci-avant.

Le premier procédé couvert par l'invention est un procédé de préparation des composés de formule 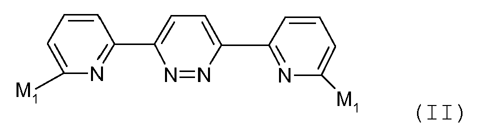 dans laquelle les substituants M₁, identiques ou différents, représentent un hydrogène, un halogène, une chaîne alkyle ou alkyloxy de 1 à 6 carbones,
par couplage de Stille entre un composé de formule 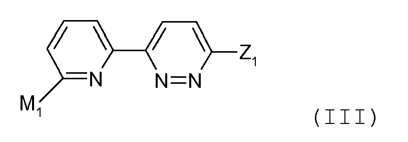 avec un composé de formule 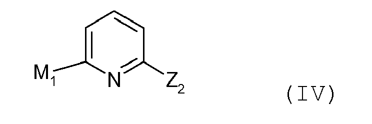 dans lesquelles, Z₁ et Z₂, différents, représentent soit un halogène, soit un groupement stannylé de formule SnB₃, dans lequel B représente une chaîne méthyle, butyle ou phényle.

Le couplage de Stille est une réaction de couplage catalysée par du palladium (0) permettant la création d'une liaison carbone-carbone. De préférence, le palladium (0) est introduit dans le milieu de réaction sous la forme de tétrakistriphénylphosphine de palladium. La réaction est opérée à reflux dans un solvant organique de préférence, du toluène, DMF (diméthylformamide), THF (tétrahydrofurane), HMPA (hexaméthylphosphorotriamide), N-méthylpyrrolidine.

L'avantage de cette voie de synthèse est de permettre l'obtention de composés (bipyridin-2-yl)pyridaziniques dissymétriques par le choix des substituants sur les cycles pyridinyles.

L'introduction de groupements carboxyliques des composés selon l'invention s'effectue ensuite selon le procédé suivant, conduisant à un composé mono ou di-carboxylé. Il s'agit donc d'un procédé **de** préparation des composés de formule 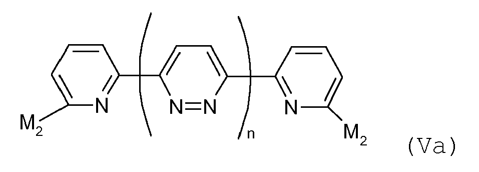 dans laquelle n est un nombre entier compris entre 1 et 4 bornes incluses, au moins un des substituants M₂ est un groupement -COOH, l'autre substituant pouvant être alternativement un hydrogène, un halogène ou une chaîne alkyloxy de 1 à 6 carbones, par oxydation du précurseur méthylé en présence d'un oxydant allylique ou aromatique tel que le dioxyde de sélénium ou l'oxyde de chrome.

Avantageusement, le solvant utilisé est un solvant classique pour les réactions d'oxydation, par exemple, le o-dichlorobenzène. La réaction est menée à des températures préférentielles de l'ordre de 100 à 160°C, de préférence 120 à 140°C.

L'invention propose également un procédé de préparation des composés de formule 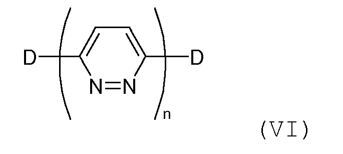 dans laquelle les groupements D représentent un halogène ou une chaîne alkyloxy de 1 à 6 carbones, de préférence méthoxy ou éthoxy et n est un nombre entier compris entre 2 et 4 bornes incluses,
par couplage d'au moins deux halogénopyridazines de formule 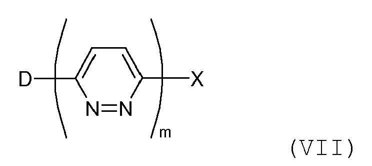 dans lesquelles X représente un halogène, D est tel que défini ci-avant et m est un nombre entier compris entre 1 et 3 bornes incluses,
en présence d'un mélange stoechiométrique de zinc, de dibromobis(triphénylphosphine) de nickel et de iodure de tétrabutylamonium dans du diméthylformamide distillé et dégazé, le couplage étant suivi d'une étape de purification par décomplexation.

Les fonctions halogènes sont les fonctions réactives de ce couplage.

Ces réactions ont lieu entre 50 et 60°C.

Ce procédé correspond à une alternative au procédé proposé par Lehn et al., dans la synthèse de la 6,6'-bis(6-méthyl-pyridin-2-yl)-3,3'-bipyridazine.

Les conditions optimisées sont en particulier la préparation à l'avance du dibromobis(triphénylphosphine) de nickel, et non sa génération *in situ* dans le milieu réactionel ainsi que l'utilisation d'iodure de tétrabutylamonium. Avantageusement, le dibromobis(triphénylphosphine) de nickel et l'iodure de tétrabutylamonium sont introduits dans un rapport 1 :0,3 :1. Dans les exemples ci-après la réaction a été menée à une température de 50 à 70°C.

Ce procédé présente l'avantage de permettre l'incrémentation du nombre de cycles pyridaziniques dans la molécule oligopyridazines.

L'étape de purification est nécessaire afin de décomplexer le produit de la réaction du milieu réactionnel. Ce procédé de purification peut être réalisé selon deux modes opératoires distincts.

Selon un premier mode opératoire, le procédé de purification des composés est opéré par décomplexation desdits composés dans une solution aqueuse saturée de cyanure de potassium ou de sodium à froid pendant 1h30 à 4h, de préférence 2h à 3h.

Par « à froid », on entend des températures allant de 0 à 25°C, de préférence 18 à 20°C.

Selon un second mode opératoire, le procédé de purification des composés est opéré par décomplexation desdits composés dans une solution aqueuse saturée d'halogénure de potassium ou d'halogénure de tétrabutyl ammonium, de préférence du fluorure de potassium, ou dans une solution saturée d'ammoniaque, la phase organique étant ensuite lavée avec de l'hydrogénocarbonate de sodium ou de potassium, puis extraite avec du chloroforme, du dichlorométhane, de l'acétate d'éthyle ou de l'éther...

L'invention vise aussi un procédé de préparation des composés de formule 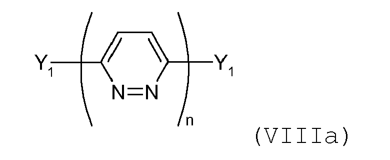
- si n = 1,
   les groupes Y₁, différents, représentent un groupement de formule 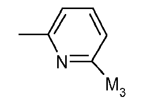 dans lequel M₃ représente un hydrogène, une chaîne alkyle ou alkyloxy de 1 à 6 carbones,
- si n est un entier compris entre 2 et 4, bornes incluses,
   les groupements Y₁, identiques ou différents, représentent une chaîne alkyle ou alkyloxy de 1 à 6 carbones, ou un groupement choisi parmi les groupes suivants : 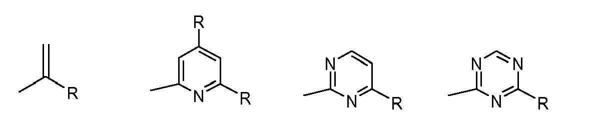 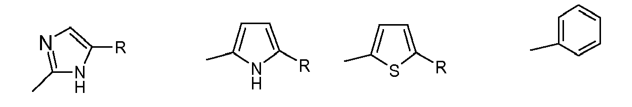 dans lesquels les groupes R, identiques ou différents, représentent un hydrogène, une chaîne alkyle ou alkyloxy de 1 à 6 carbones,
   par couplage de Stille, dans des proportions allant de 1 :2 à 1 :3, entre un composé de formule 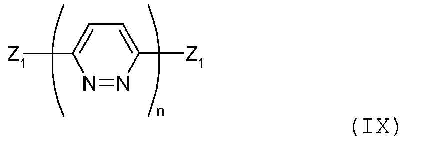 et un composé de formule Y₁-Z₂
   dans laquelle Z₁, Z₂, différents, représentent soit un halogène, soit un groupement stannylé de formule SnB₃, dans lequel B représente une chaîne méthyle, butyle ou phényle.

L'invention propose également deux procédés de préparation inspirés du couplage dit de Negishi.

Il s'agit du procédé de préparation d'un composé : 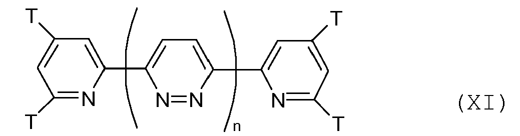 dans lequel n est un entier compris entre 1 et 4, bornes incluses,
les groupes T, identiques ou différents, représentent un hydrogène, une chaîne alkyle de 1 à 6 carbones,
par couplage entre un composé de formule 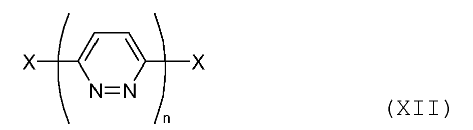 dans lequel les groupes X sont des halogènes, et n tel que défini précédemment et un composé de formule
(XIII)
X, T étant tels que définis précédemment, en présence de butyllithium, d'un solvant, d'un réactif zincique et de palladium (O).

Ce procédé permet un double couplage, tandis que les inventeurs ont également mis au point un procédé de couplage sélectif permettant la préparation d'un composé 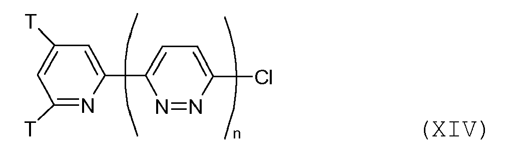 dans lequel n est un entier compris entre 1 et 4, bornes incluses,
les groupes T, identiques ou différents, représentent un hydrogène, une chaîne alkyle de 1 à 6 carbones,
par couplage sélectif entre un composé de formule 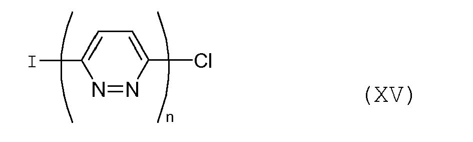 n étant tel que défini précédemment, et un composé de formule 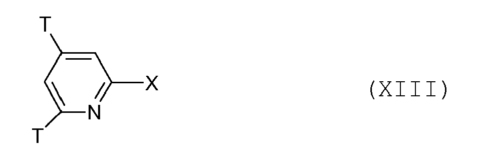 X étant un halogène, en présence de butyllithium, d'un solvant, d'un réactif zincique et de palladium (O).

Avantageusement, dans ces deux procédés, le solvant sera du THF ou de l'éther, le réactif zincique, du ZnCl₂ et le palladium (o) du (Pd(Ph₃)₄) ou du Pd₂dba₃.
L'invention a également permis la préparation d'un nouveau précurseur : la 3-méthoxy-6-(pyridin-2-yl)-pyridazine.

Cette molécule est impliquée dans deux nouveaux procédés : un procédé de préparation de la 3-méthoxy-6-(pyridin-2-yl)-pyridazine par couplage de la 3-chloro-6-méthoxypyridazine avec une 2-trialkylstannyl-pyridine en présence de palladium (0) et un procédé de préparation de la 6-(pyridin-2-yl)-2*H-*pyridazin-3-one par hydrolyse de la 3-méthoxy-6-(pyridin-2-yl)-pyridazine.

L'invention vise également un procédé de préparation de la 6,6'-bis(pyridin-2-yl)-3,3'-bipyridazine par homocouplage de la 3-chloro-6-(pyridin-2-yl)-pyridazine en présence de dibromobistriphénylphosphine.

Alternativement à ces procédés de synthèse de nature purement organique, les inventeurs ont également élaboré des procédés mettant en oeuvre des voies de synthèse électrochimique.

L'invention propose donc un procédé d'homocouplage par voie électrochimique d'un halogénure pyridazinique de formule 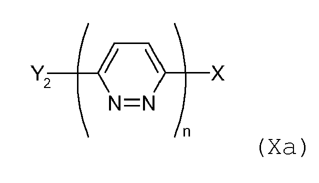 dans laquelle n est un nombre entier compris entre 1 et 2 bornes incluses, X est un halogène et Y₂ représente un halogène, une chaîne alkyle ou alkyloxy de 1 à 6 carbones, un groupement choisi parmi les groupes suivants : 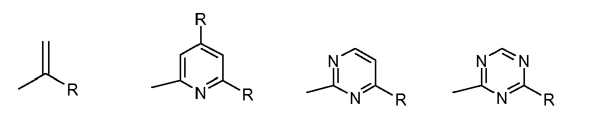 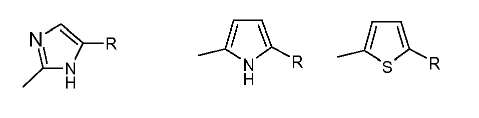 dans lesquels les groupes R, identiques ou différents, représente un hydrogène, une chaîne alkyle ou alkyloxy de 1 à 6 carbones ou un phényle, les conditions de l'électrolyse étant les suivante :
- l'anode est constituée d'au moins 50% de fer,
- le milieu de l'électrolyse comporte du nickel, un élément choisi parmi les halogènes, et de la pyridine ou ses dérivés.

De préférence, l'anode utilisée est une anode Fe/Ni (64/36). Le solvant de la réaction comporte avantageusement au moins 50% de DMF et un co-solvant polaire. Par exemple, on pourra utiliser un mélange de diméthylformamide (DMF) et de pyridine, dans un rapport allant de 90/10 à 50/50, bornes incluses, de préférence, 80/20. Le catalyseur mis en oeuvre est préférentiellement un complexe du nickel, tel qu'un halogénure de nickel hydraté. Lorsque le solvant ne contient pas de pyridine, on pourra utiliser avantageusement comme catalyseur un halogénure de nickel-bipyridine.

L'électrolyte support est de préférence un halogénure de tétrabutylammonium ou un équivalent tel que le tétrafluoroborate de tétrabutyl ammonium, avantageusement dans des quantités allant de 10 à 20% molaire bornes incluses, de préférence 13 à 17%, par rapport au substrat pyridazinique.

L'intensité mise en oeuvre au cours de la réaction est par exemple de l'ordre de 0,05A à 0,2A, bornes incluses, de préférence, 0,06A à 0,1A. La réaction peut être conduite à température ambiante (généralement 20-25°C).

Par ailleurs, l'invention propose également un procédé d'hétérocouplage par voie électrochimique d'un halogénure pyridazinique de formule 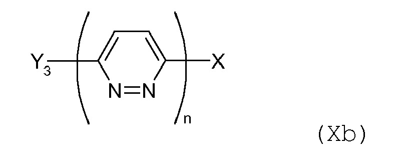 dans laquelle n est un nombre entier compris entre 1 et 2 bornes incluses, X est un halogène et Y₃ représente une chaîne alkyle ou alkyloxy de 1 à 6 carbones ou un groupement choisi parmi les groupes suivants : 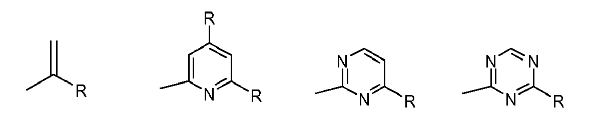 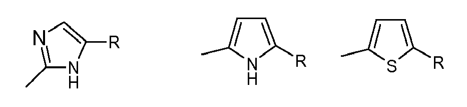 dans lesquels les groupes R, identiques ou différents, représentent un hydrogène, une chaîne alkyle ou alkyloxy de 1 à 6 carbones ou un phényle,
avec un halogénure de cycle aromatique de formule Ar-X dans laquelle, X tel que défini précédemment et Ar représente un cycle aromatique de 5 à 6 chaînons, éventuellement substitué, les conditions de l'électrolyse étant les suivantes :
- l'anode est constituée de fer,
- le catalyseur est choisi parmi les halogénures de nickel bipyridine.

Cette réaction est spécifiquement décrite dans l'exemple 3 ci-après.

De préférence, le solvant utilisé est du DMF tandis que l'électrolyte support est un halogénure de tétrabutylammonium ou un équivalent tel que le tétrafluoroborate de tétrabutyl ammonium dans des quantités allant de 10 à 20% molaire, bornes incluses, de préférence de 13 à 17%, par rapport au substrat pyridazinique. L'intensité mise en oeuvre au cours de la réaction est de 0,15 à 0,35A bornes incluses, de préférence de l'ordre de 0,2A. La réaction peut être conduite à température ambiante (généralement 20-25°C).

Le cycle aromatique est préférentiellement un noyau phényle, pyridinyle ou thiophényle, éventuellement substitué.

Le procédé de réduction des oligopyridazines en oligopyrroles à la base des travaux des inventeurs est décrit ci-dessous. Il fait plus spécialement l'objet des exemples 4 et 5.

Ce procédé permet donc la réduction en pyrrole d'un composé de formule 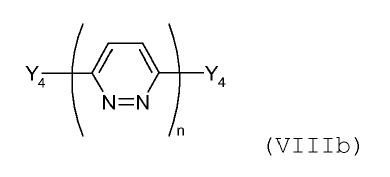 dans laquelle n est un nombre entier compris entre 2 et 4 bornes incluses, les groupements Y₄, identiques ou différents, représentent une chaîne alkyle ou alkyloxy de 1 à 6 carbones ou un groupement choisi parmi les groupes suivants : 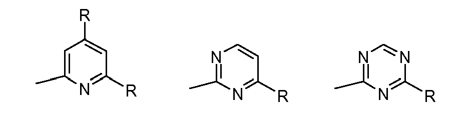 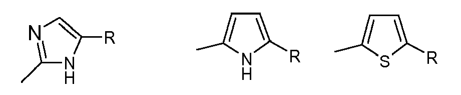 dans lesquels les groupes R, identiques ou différents, représentent un hydrogène, une chaîne alkyle ou alkyloxy de 1 à 6 carbones ou un phényle,
par voie électrochimique, par extrusion d'un atome d'azote sur un ou plusieurs cycle(s) pyridazinique(s), dans lequel les conditions de l'électrolyse sont les suivante :
- l'anode est une électrode à grande surface,
- le milieu de l'électrolyse est un milieu polaire donneur de proton.

A titre d'exemple, le milieu polaire donneur de proton peut être constitué par un solvant polaire organique (tel que du DMF, de l'acétonitrile, etc.) complété par un donneur de proton (tel que le phénol, l'acide acétique, etc.), et, éventuellement, lorsque le milieu résultant n'est pas conducteur, un électrolyte support tel que des sels d'ammonium quaternaire ou un milieu aqueux acide alcoolique.

Avantageusement, les sels d'ammonium quaternaire sont choisis parmi le tétrabutylammonium hexafluorophosphate ou le tétrabutylammonium hydrogénosulfate et le milieu acide alcoolique est constitué par un mélange acide sulfurique ou acide acétique additionné d'éthanol.

De tels milieux sont amplement décrits dans les exemples 4 à 8.

De préférence, la cathode est choisie parmi les électrodes à nappe de mercure de diamètre 4,5 cm, les électrodes de carbone à grande surface ou les électrodes de carbone sérigraphiées.

L'intensité mise en oeuvre est de l'ordre de 10 à 50mA. La réaction est conduite à température ambiante (généralement 20-25°C).

Le potentiel de réduction imposé qui varie selon les substrats étudiés doit être contrôlé, afin de maîtriser la quantité de Coulomb consommée pendant l'électrolyse, c'est-à-dire le nombre d'électrons utilisés : 4 pour le monopyrrole et 8 pour le bipyrrole, etc.

Ce procédé vient compléter les travaux des inventeurs sur la réduction d'un cycle pyridazine en pyrrole (Manh G.T. et al, Electrochimica Acta, 2002, 2833).

De manière inattendue, cette réduction électrochimique effective sur des monopyridazines s'est révélée également applicable, après élaboration des conditions de réaction adéquates, à des composés oligopyridaziniques.

En effet, il n'était pas évident d'obtenir une régression des cycles pyridaziniques du fait de la modification de l'environnement électronique des cycles, cette modification étant principalement due au fait que les cycles pyridaziniques devant réagir dans la molécule se trouvaient à présent adjacents à d'autres cycles identiques susceptibles également de réagir. En outre, cette modification était également susceptible de générer des intermédiaires de synthèse présentants des propriétés d'électroréduction différentes. De plus, dans le cas où la régression était réalisable, il était probable que les étapes de réductions électrochimiques, opérées simultanément sur plusieurs structures pyridaziniques adjacentes, interagissent entre elles et conduisent, par exemple à des dégradations, à des réarrangements internes, à des réductions partielles pour fournir des intermédiaires di ou tétrahydro-pyridaziniques au lieu des enchaînements pyrroliques. Dans le cas où la régression s'avérait séquentielle (cycle pyridazinique par cycle pyridazinique), il s'agissait d'évaluer l'influence de la formation éventuelle d'un premier pyrrole ou d'un intermédaire dihydropyridaziniques sur le potentiel de réduction des systèmes mixtes alors générés.

Par « régression », on entend deux étapes de réduction en milieu acide, la régression étant le résultat mécanistique des réductions électrochimiques.

Les travaux des inventeurs ont permis d'établir un protocole adapté à la réduction de composés oligopyridaziniques et de démontrer le caractère soit séquentiel de cette réduction soit simultané selon le nombre d'électrons et le potentiel appliqué lors de l'électroréduction.

Le procédé de régression a également permis de synthétiser des produits minoritaires, qui sont nouveaux. Il s'agit des produits suivants :
- 6-(4,6-diméthylpyridin-2-yl)-3-(5-(4,6-diméthylpyridin-2-yl)-1*H*-pyrrol-2-yl)-1,4,5,6- tétrahydropyridazine,
- 6-(6-méthylpyridin-2-yl)-3-(5-(6méthylpyridin-2-yl)-1*H-*pyrrol-2-yl)-1,4,5,6- tétrahydropyridazine,
- 6-(pyridin-2-yl)-3-(5-(pyridin-2-yl)-1*H*-pyrrol-2-yl)-1,4,5,6- tétrahydropyridazine.

Dans tous les procédés ci-avant, il a été fait référence à des chaînes alkyles et alkyloxy comportant 1 à 6 carbones. Avantageusement, lesdites chaînes comportent 1 à 3 carbones, de préférence, elles sont méthyle, éthyle, méthoxy ou éthoxy.

Un troisième aspect de l'invention vise à couvrir les multiples applications des composés synthétisés.

Les composés selon l'invention sont particulièrement adaptés à une utilisation comme ligands.

En particulier, ces composés sont des ligands qui complexent particulièrement bien les ions métalliques, notamment les cations de type fer, cuivre, ruthénium, europium, argent et bismuth. Ils peuvent être utilisés seuls ou plusieurs ligands identiques en association les uns avec les autres.

On citera à titre d'exemples de ligands non limitatifs, les métallocaténanes formés à partir de composés selon l'invention.

Les inventeurs ont par ailleurs recherché les éventuelles propriétés biologiques de leurs composés et mis en évidence des propriétés thérapeutiques intéressantes.

L'invention vise donc les composés de formule 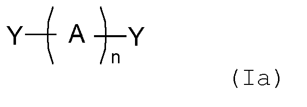 dans laquelle
- si n=1
   - si A représente un groupement de formule 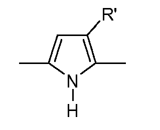 dans laquelle R' représente un hydrogène, une chaîne alkyle, hydroxyalkyle, alkylamine, alkyloxy de 1 à 6 carbones, un groupement -COOH, -COOR1, -CONH₂, -CONHR1, dans lesquels R1 est une chaîne alkyle de 1 à 6 carbones,
   - les groupements Y, identiques ou différents, représentent un groupement de formule 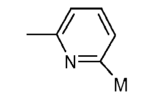 dans lequel M représente un hydrogène, un halogène, une chaîne alkyle, hydroxyalkyle, alkylamine ou alkyloxy de 1 à 6 carbones, un groupement -COOH, -COOR1, -CONH₂, -CONHR1, dans lesquels R1 est tel que défini ci-avant,
   - si A représente un groupement de formule 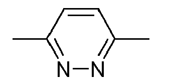
   - les groupements Y, identiques, représentent un groupement de formule 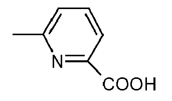 ou, les groupements Y, différents, représentent un groupement de formule 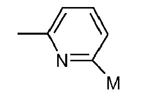 dans lequel M représente un hydrogène, un halogène, une chaîne alkyle, hydroxyalkyle, alkylamine ou alkyloxy de 1 à 6 carbones, un groupement -COOH, -COOR1, -CONH₂, -CONHR1, dans lesquels R1 est tel que défini ci-avant,
- si n est un entier compris entre 2 et 4 bornes incluses,
   - les groupements A, identiques ou différents, représentent un groupement 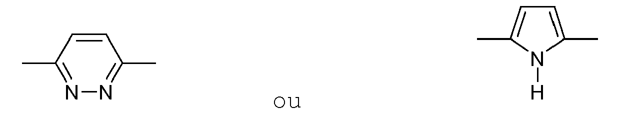
   - les groupements Y, identiques ou différents, représentent un halogène, un hydroxy, un mercapto, une chaîne alkyle, hydroxyalkyle, alkylamine ou alkyloxy de 1 à 6 carbones, éventuellement cyclique, un groupement -COOH, -COOR1, -CONH₂, -CONHR1, dans lesquels R1 est tel que défini précédemment, ou un groupement choisi parmi les groupes suivants : 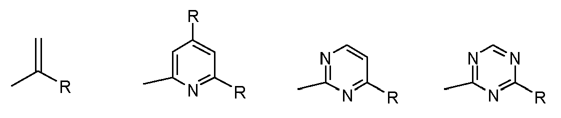 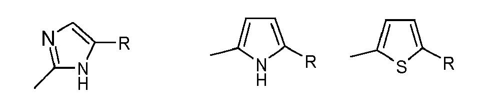 dans lesquels les groupes R, identiques ou différents, représentent un hydrogène, une chaîne alkyle ou alkyloxy de 1 à 6 carbones ou un phényle, un groupement -COOH, -COOR1, - CONH₂, -CONHR1, dans lesquels R1 est tel que défini ci-avant,
pour une utilisation comme médicaments.

De préférence, les chaînes alkyles, hydroxyalkyles, alkylamines ou alkyloxy de 1 à 6 carbones sont des chaînes méthyles, hydroxyméthyles, méthylamines ou méthoxy.

Dans toute la demande, les composés ci-avant listés et les composés préférentiels donnés ci-après seront désignés par les termes « composés utilisables comme médicaments ».

Plus préférentiellement, dans la formule ci-avant, que n est égal à 2 et Y est un groupement 2-pyridinyle éventuellement substitué ou un groupement -C(CH₂)OR1, dans lequel R1 est une chaîne alkyle de 1 à 6 carbones.

En effet, la plupart des composés utilisables comme médicaments sont nouveaux, et pour les autres, leurs propriétés thérapeutiques n'apparaissent pas avoir fait l'objet d'étude.

L'invention couvre plus particulièrement les composés suivants :
- 5,5'-bis(6-méthyl-pyridin-2-yl)-2,2'-bipyrrole,
- 6,6'-di-(1-éthoxyvinyl)-3,3'-bipyridazine,
- 6,6'-bis(pyridin-2-yl)-3,3'-bipyridazine,
- 5,5'-bis(pyridin-2-yl)-2,2'-bipyrrole,
- 3-[5-(6-méthyl-pyridin-2-yl)-pyrrol-2-yl]-6-(6-méthyl-pyridin-2-yl)-pyridazine,
- 6,6'-bis(6-méthyl-pyridin-2-yl)-3,3'-bipyridazine,
- 6,6"'-bis-(6-méthyl-pyridin-2-yl)-[3,3':6',6":3",3"']quaterpyridazine,
- 6-(pyridin-2-yl)-3-((5-pyridin-2-yl)-pyrrol-2-yl)-pyridazine,
- 6,6'-bis(4,6-diméthylpyridin-2-yl)-3,3'-bipyridazine,
- 5,5'-bis(4,6-diméthylpyridin-2-yl)-2,2'-bipyrrole),
- 6-(4,6-dimethylpyridin-2-yl)-3-((5-(4,6-diméthylpyridin-2-yl)-pyrrol-2-yl)-pyridazine).
pour une utilisation comme médicaments.

L'intérêt thérapeutique de ces composés est décrit plus précisément dans l'exemple 42 ci-après. L'invention vise également les compositions thérapeutiques renfermant comme principes actifs les composés utilisables comme médicaments.

L'invention vise également les composés utilisables comme médicaments dans lesquels n=1 et les groupements M représentent un halogène, une chaîne alkyle de 1 à 6 carbones ou un groupement -COOH pour une utilisation comme médicaments.

Avantageusement, les groupements M, identiques ou différents, représentent un halogène, une chaîne alkyle de 1 à 6 carbones ou un groupement -COOH.

L'invention couvre plus particulièrement les composés suivants :
- 3-(2-carboxy-pyridin-6-yl)-6-(pyridin-2-yl)-pyridazine,
- 3,6-bis(2-carboxy-pyridin-6-yl)-pyridazine,
- 3-(6-méthyl-pyridin-2-yl)-6-(pyridin-2-yl)-pyridazine,
- 3-(2-bromo-pyridin-6-yl)-6-(pyridin-2-yl)-pyridazine,
- 2,5-bis(pyridin-2-yl)pyrrole,
pour une utilisation comme médicaments.

Plus précisément, les composés utilisables comme médicament possèdent plusieurs applications biologiques pouvant déboucher sur des applications thérapeutiques.

Selon une première application, ces composés utilisables comme médicaments peuvent complexer sélectivement les acides nucléiques. En particulier, ils peuvent être utilisés comme agents de complexation sélectifs d'ADN et d'ARN, dont celui du VIH. Ils agissent en effet sur la reverse transcriptase des cellules, par inhibition de son amorce sur l'ARN viral. Ils sont donc particulièrement adaptés à la préparation de médicaments anti-viraux. Ces composés peuvent également être utilisés comme des agents de coupure de l'ADN (métallo-nucléase) en particulier lorsque les composés sont complexés à un métal de type Cu.

Selon une seconde application, ces composés utilisables comme médicaments présentent une activité cytotoxique vis-à-vis de cellules cancéreuses. Ils sont donc particulièrement adaptés à la préparation de médicaments anticancéreux.

Préférentiellement, le cancer visé par l'invention est un carcinome, par exemple un carcinome ORL, des poumons, de l'utérus, digestif (oesophage, colon, foie), de la peau, du sein, de la prostate, des ovaires... La 6,6'-di-(1-éthoxyvinyl)-3,3'-bipyridazine et la 6-(pyridin-2-yl)-2H-pyridazin-3-thione, 3,6-bispyridin-2-yl-pyridazine et 3-(6-méthyl-pyridin-2-yl)-6-pyridin-2-yl-pyridazine, en particulier, ont montré une activité cytotoxique très importante dans un modèle cellulaire de cancer : un test *in vitro* sur des cellules KB, Caco, Huh7 et fibroblaste.

Selon une troisième application, ces composés utilisables comme médicaments agissent sur la régulation du transfert de fer dans les bactéries par inhibition de la protéine tonB.

Le fer est indispensable à l'infection bactérienne. Or, les bactéries doivent puiser le fer dans leur environnement. Ce dernier est toujours couplé à des protéines telles que la transferrine, la lactoferrine, l'hémoglobine... Par conséquent, les bactéries disposent de systèmes très élaborés, impliquant la protéine TonB, pour récupérer ce fer.

En particulier, la 6,6'-di-(1-éthoxyvinyl)-3,3'-bipyridazine s'est avérée capable d'inhiber la croissance de *E.coli.*

Ces composés sont donc particulièrement adaptés à la préparation de médicament antibactérien, par exemple pour le traitement de la dysenterie ou de la méningite.

Selon une quatrième application, ces composés utilisables comme médicaments permettent également l'obtention de médicaments destinés à traiter les maladies parasitaires. Les maladies parasitaires visées par l'invention sont en particulier les leishmanioses, les aspergilloses et les candidoses.

Pour cette application thérapeutique spécifique, on utilisera avantageusement les composés de formule 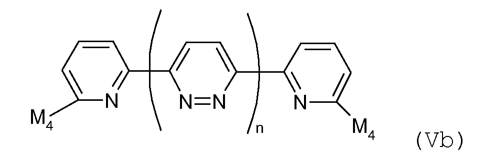 dans laquelle
- n est un entier compris entre 1 et 4 bornes incluses,
- les groupements M₄, identiques ou différents, représentent un hydrogène, une chaîne alkyle de 1 à 6 carbones, un groupement -COOH, -COOR1, dans lesquels R1 est une chaîne alkyle de 1 à 6 carbones,
et plus particulièrement les composés listés dans le groupe suivant :
- 3-(6-méthyl-pyridin-2-yl)-6-(pyridin-2-yl)-pyridazine,
- 3-(2-carboxy-pyridin-6-yl)-6-(pyridin-2-yl)-pyridazine,
- 6,6'-bis(pyridin-2-yl)-3,3'-bipyridazine,
- 6,6'-bis(6-méthyl-pyridin-2-yl)-3,3'-bipyridazine, et,
- 3,6-bis(2-carboxy-pyridin-6-yl)-pyridazine.

Selon une cinquième application, les composés utilisables comme médicaments, sont des vecteurs de métaux radioactifs de grand intérêt dans le cadre de radioligands. Par conséquent, lorsqu'ils sont complexés au métal adéquat, tel que le bismuth ou l'europium, ils permettent l'obtention d'un médicament destiné à la radio-immunothérapie.

Pour cette dernière application thérapeutique, on utilisera préférentiellement, les composés dans la formule desquels les groupements A représentent un groupement de formule 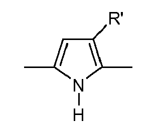 dans laquelle R' représente un hydrogène, une chaîne alkyle, hydroxyalkyl, alkylamine ou alkyloxy de 1 à 6 carbones, un groupement -COOH, -COOR1, -CONH₂, -CONHR1 dans lesquels R1 est une chaîne alkyle de 1 à 6 carbones.

Ces composés sont des ligands tridentés ou tétradentés, mixtes N,O ou N-donneur. Ils sont par conséquent particulièrement appropriés pour complexer des ions métalliques.

Les inventeurs ont également défini des applications dans le domaine de l'environnement, des matériaux et de l'électronique.

En effet, les composés selon l'invention peuvent avantageusement être utilisés pour la dépollution de cations en milieu liquide. Il s'agit en particulier des composés pour lesquels
A représente un groupement 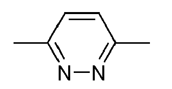 n est compris entre 2 et 4, bornes incluses et Y, identiques ou différents, représente un hydroxy, une chaîne hydroxyalkyle ou alkyloxy de 1 à 6 carbones, éventuellement cyclique, un groupement -COOH, -COOR1 dans lequel R1 est une chaîne alkyle de 1 à 6 carbones, -CONH₂.

Comme nous l'avons déjà mentionné, ces composés sont particulièrement appropriés pour complexer des ions métalliques. Ils peuvent éventuellement être utilisés seuls ou plusieurs ligands identiques en association les uns avec les autres.

Plus préférentiellement encore, le composé sera choisi parmi le groupe suivant :
- 5,5'-bis(6-méthyl-pyridin-2-yl)-2,2'-bipyrrole,
- 6,6'-di-(1-éthoxyvinyl)-3,3'-bipyridazine,
- 6,6'-bis(pyridin-2-yl)-3,3'-bipyridazine,
- 5,5'-bis(pyridin-2-yl)-2,2'-bipyrrole,
- 3-[5-(6-méthyl-pyridin-2-yl)-pyrrol-2-yl]-6-(6-méthyl-pyridin-2-yl)-pyridazine,
- 3-(2-carboxy-pyridin-6-yl)-6-(pyridin-2-yl)-pyridazine,
- 3,6-bis(2-carboxy-pyridin-6-yl)-pyridazine,
- 3-(6-méthyl-pyridin-2-yl)-6-(pyridin-2-yl)-pyridazine, et,
- 3-(2-bromo-pyridin-6-yl)-6-(pyridin-2-yl)-pyridazine.

On utilisera avantageusement le composé selon l'invention en combinaison avec un acide carboxylique, en particulier l'acide α-bromocaprique. Les inventeurs ont en effet constaté une synergie dans l'activité de dépollution lorsque cette combinaison spécifique était mise en oeuvre vis-à-vis des cations actinides particulièrement.

L'invention couvre également les matériaux composés d'une organisation supramoléculaire de composés selon l'invention. En particulier, certains des composés selon l'invention possèdent des propriétés d'auto-assemblages. D'autres peuvent s'auto-assembler autour de cations métalliques.

Ces matériaux présentent en outre des propriétés en optique linéaire avantageuses. Ils permettent en particulier l'élaboration de cristaux liquides, de fibres optiques ...

D'autres caractéristiques et avantages de l'invention apparaîtront dans les exemples qui suivent, avec références aux figures, qui représentent respectivement :
- la figure 1, des voltampérogrammes Voltampérogrammes cycliques au cours de l'électrolyse préparative, milieu H₂SO₄ 0,5mol.L⁻¹, éthanol (1/1), C=6.10⁻³mol/L, v=100mV/s,
- la figure 2, des voltampérogrammes cycliques au cours de l'électrolyse préparative de la 6,6'-bis(6-méthyl-pyridin-2-yl)-3,3'-bipyridazine **2**, ( - avant électrolyse, - - pendant l'électrolyse, - à la fin de l'électrolyse), électrode de carbone vitreux, v=100mV/s,
- la figure 3, des voltampérogrammes cycliques du solvant et de 3,6-bis(pyridin-2-yl)-pyridazines en milieu tampon acétique et éthanol, C = 10⁻³mol.L⁻¹, V = 100mV/s,
- la figure 4, des voltampérogrammes cycliques de la 4-carbométhoxy-3,6-bis(pyridin-2-yl)-pyridazine **84** et de la 3,6-bis(pyridin-4-yl)-pyridazine **81** en milieu tampon acétique et éthanol, C = 10⁻³mol.L⁻¹, V = 100mV/s,
- la figure 5, des voltampérogrammes cycliques au cours de l'électrolyse préparative de la 4-(1-hydroxyéthyl)-3,6-bis(pyridin-2-yl)-pyridazine **82**, ( - avant électrolyse, - - pendant l'électrolyse, - à la fin de l'électrolyse), électrode de carbone vitreux, v=100mV/s),
- la figure 6, des voltampérogrammes de différents dérivés pyrroliques en fin d'électrolyse préparative dans le compartiment cathodique, électrode de carbone vitreux, v = 100mV/s,
- la figure 7 est un voltampérogramme de la réaction d'électrolyse préparative de la 6,6'-bis(pyridin-2-yl)-3,3'-bipyridazine (**19**), blanc = voltampérogramme de référence en absence de produit, elec 0 = voltampérogramme de contrôle au temps 0, elec 1 = voltampérogramme de après consommation de 8 électrons,
- la figure 8 est un voltampérogramme de la réaction d'électrolyse préparative de la 6,6'-bis(4,6-diméthylpyridin-2-yl)-3,3'-bipyridazine (**105**),
- la figure 9 présente les spectres d'absorption UV-Visible et de fluorescence de la 6,6'-bis(pyridin-2-yl)-3,3'-bipyridazine (**19**),
- la figure 10 présente les spectres d'absorption UV-Visible et de fluorescence de la 6,6'-bis(6-méthyl-pyridin-2-yl)-3,3'- bipyridazine (**2**), et,
- la figure 11 présente les spectres d'absorption UV-Visible et de fluorescence de la 6,6'-bis(4,6-diméthylpyridin-2-yl)-3,3'-bipyridazine (**105**).

### Conditions générales et procédures relatives à la partie expérimentale

### Résonance magnétique nucléaire

Les spectres RMN ¹H et ¹³C ont été enregistrés sur un spectromètre Bruker Avance 300. Les fréquences d'irradiation sont respectivement de 300 MHz et 75.5 MHz, les déplacements chimiques sont donnés en partie par million (ppm) avec le tétraméthylsilane comme étalon interne. Les constantes de couplages sont données en Hertz (Hz) et la multiplicité des signaux est décrite comme suit : s (singulet), sl (singulet large), d (doublet), dd (doublet de doublet), t (triplet), q (quadruplet), m (multiplet).

### Analyses UV et fluorescence

Les spectres d'absorption UV-Visible ont été enregistrés sur un spectromètre UV-2401PC Shimadzu. Les spectres de fluorescence ont été enregistrés sur un fluorimètre SPEX Fluoromax. Tous les spectres enregistrés par les appareillages décrits ci-dessus ont été effectués dans une cellule en quartz UV-Visible (1cm).

### Chromatographie phase gazeuse

Les chromatogrammes ont été enregistrés sur l'appareil HP 6890 équipé d'une colonne JW 1701 (30 m x 0,25 mm, phase stationnaire : cyanopropyl-phényl-méthylsilane), d'un détecteur à ionisation de flamme, et l'azote utilisé comme gaz vecteur (débit = 1,3 mL/min). La température du four a été programmée de la manière suivante: 1 minute à 80°C, puis 12°C par minute jusqu'à 280°C.

### Chromatographie sur couche mince

Toutes les réactions sont suivies par chromatographie sur couche mince (Kieselgel 60F₂₅₄ Merck sur feuille d'aluminium). Les plaques sont révélées sous la lumière UV ou par le test de Mohr (FeSO₄ 10% dans l'eau).

### Spectroscopie de masse

Les spectres de masse ont été enregistrés sur un appareil DSQ Thermoelectron par impact électronique (70 eV), par ionisation chimique (ammoniac), par introduction directe ou par couplage GC-MS.

### Solvant

Tous les solvants utilisés sont achetés purs pour synthèse. Le tétrahydrofurane (THF) est fraîchement distillé sur sodium/benzophénone sous argon. Le dichlorométhane (DCM) et le *N,N*-diméthylformamide (DMF) sont fraîchement distillés sur hydrure de calcium sous argon. Le toluène est fraîchement distillé sur sodium sous argon.

### Procédure A: procédure générale de la réaction d'homocouplage de type Ulmann

Dans un ballon, le bromure de tétrabutylammonium, le zinc activé en poudre et le dibromo-bistriphénylphosphine nickel (II) sont ajoutés. L'ensemble est séché sous vide et placé sous argon. Le DMF fraîchement distillé et dégazé est canulé dans le milieu. La solution est agitée à température ambiante jusqu'à l'obtention d'une solution homogène. L'halogénopyridazine est solubilisée dans le DMF fraîchement distillé, dégazé et canulée dans le milieu réactionnel. La solution est agitée pendant 15 heures à 55°C. La solution noirâtre est refroidie à température ambiante, traitée par une solution d'ammoniaque (25 N) et extraite au DCM. Après avoir séché la phase organique sur Na₂SO₄ et évaporé le solvant sous pression réduite, le résidu est ensuite purifié.

### Procédure B: procédure générale de l'hydrolyse acide

Dans un ballon surmonté d'un réfrigérant de la méthoxypyridazine et une solution d'HBr à 33% dans l'acide acétique sont agités 48 heures à 60°C. La solution est refroidie et concentrée sous vide. Le précipité est filtré et lavé à l'acétone. Le solide grisâtre est mis en suspension dans l'eau. La solution est portée à reflux et neutralisée par une solution de NaOH 1M. Le précipité est filtré, lavé à l'eau et séché sous vide.

### Procédure C: procédure générale de la chloration

Dans un ballon surmonté d'un réfrigérant le POCl₃ et la pyridazinone sont chauffés à reflux pendant 18 heures. Après retour à température ambiante l'excès de POCl₃ est éliminé par distillation sous vide et le résidu est hydrolysé par de la glace. La solution est ensuite neutralisée par ajout de soude 1M et extraite au dichlorométhane. La phase organique est séchée sur Na₂SO₄ et concentrée sous pression réduite.

### Procédure D: procédure générale du couplage de Stille

Dans un ballon surmonté d'un réfrigérant sous argon, sont placés les réactifs préalablement séchés (halogénoaryle, stannylpyridine, catalyseur au palladium) et le solvant fraîchement distillé et dégazé est canulé dans le milieu réactionnel. La solution est chauffée et agitée jusqu'à disparition totale du produit de départ. Après retour à température ambiante, le solvant est évaporé sous pression réduite, le résidu est repris dans le DCM. La solution est filtrée sur Célite, et lavée au DCM. La phase organique est ensuite lavée successivement avec une solution concentrée d'ammoniaque (25N) et une solution saturée de KF. La phase organique est séchée sur Na₂SO₄ et concentrée sous pression réduite.

### Procédure E: procédure générale du couplage de Negishi

Dans un ballon tricol surmonté d'un réfrigérant une solution de bromopyridine (1.6eq.) dans le THF fraîchement distillé et dégazé est refroidie à -78°C. Le butyllithium (2.5M dans l'hexane, 1.6eq.) est ajouté lentement et le milieu réactionnel est agité pendant 30 minutes à -78°C. Une solution de chlorure de zinc (préalablement sublimé, 1.6eq.) dans le THF dégazé est canulée à -78°C dans le milieu réactionnel. La solution est agitée à température ambiante pendant 30 minutes puis, une solution de tetrakis(triphénylphosphine)palladium (0) (0.1eq.) et d'halogénopyridazine (1eq.) dans le THF est canulée dans le milieu réactionnel. La solution est agitée pendant 48 heures à une température dépendante du substrat. Le milieu est traité par une solution saturée de NaHCO₃. La solution est filtrée sur Célite, et lavée successivement au DCM avec une solution concentrée d'ammoniaque (25N). La phase organique est séchée sur Na₂SO₄ et concentrée sous pression réduite.

### Procédure F: procédure générale de la contraction de cycle par voie électrochimique

Le composé à réduire est dissous soit dans un système de trois solvants (THF/tampon acétique/CH₃CN: 5/4/1), soit dans une solution d'H₂SO₄ à 0.5M et placé dans le compartiment anodique de la cellule électrochimique. Un système de solvant identique est placé dans le compartiment cathodique et la tension appropriée est imposée jusqu'au passage de 8 électrons. La phase organique est évaporée sous vide si nécessaire. La phase aqueuse est ensuite traitée par une solution saturée de Na₂CO₃ jusqu'à l'obtention d'un pH alcalin. Le milieu est extrait au DCM, la phase organique est séchée sur Na₂SO₄, filtrée et concentrée sous vide. Le résidu est purifié par chromatographie sur colonne de silice (EP/AcOEt: le rapport dépend des composés).

### Exemple 1 : Synthèse de la 6,6'-bis(pyridin-2-yl)-3,3'-bipyridazine 19, de la 6,6'-bis(5-méthyl-pyridin-2-yl)-3,3'-bipyridazine 2, et de la 6'6'-di-picolin-4,4'-di-méthyl-2-yl-[3,3']bipyridazine (105)

### 1. Synthèse de la 6,6'-bis(pyridin-2-yl)-3,3'-bipyridazine 19

Une première voie d'accès classique à la 6,6'-bis(pyridin-2-yl)-3,3'-bipyridazine **19** a été envisagée selon l'analyse rétrosynthétique suivante (Rétrosynthèse 1), qui s'inspire de la stratégie de J. M. Lehn (Baxter, Lehn et al. 2000).

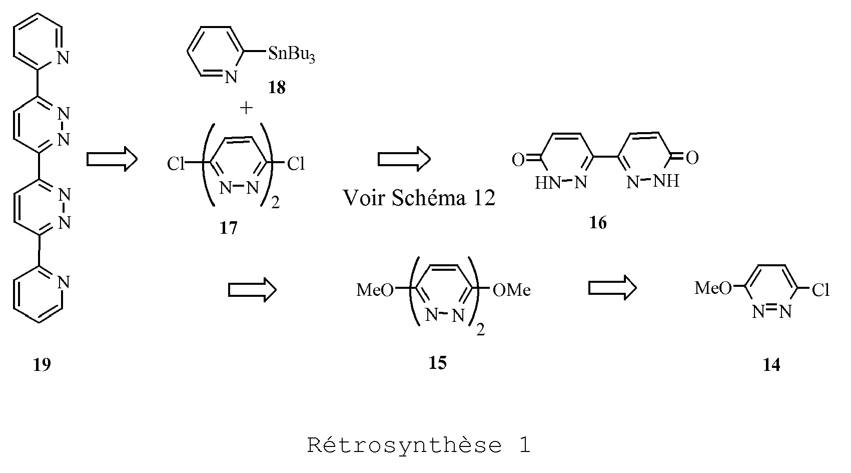

Différentes voies de synthèse ont été étudiées afin d'obtenir la bipyridazine (**19**).

### Voie 1 :

La bi-pyridazine (**15**) est obtenue après une réaction d'homocouplage de la 3-chloro-6-méthoxypyridazine (**14**) en présence d'un catalyseur : le dibromobistriphénylphosphine de nickel (II) avec un rendement de 36%. La bipyridazinone (**16**) est obtenue après hydrolyse acide avec un rendement de 98%. Le composé (**16**) est ensuite chloré, puis mis en présence de stannylpyridine (**18**) dans les conditions de couplage de Stille pour obtenir le produit (**19**) attendu.

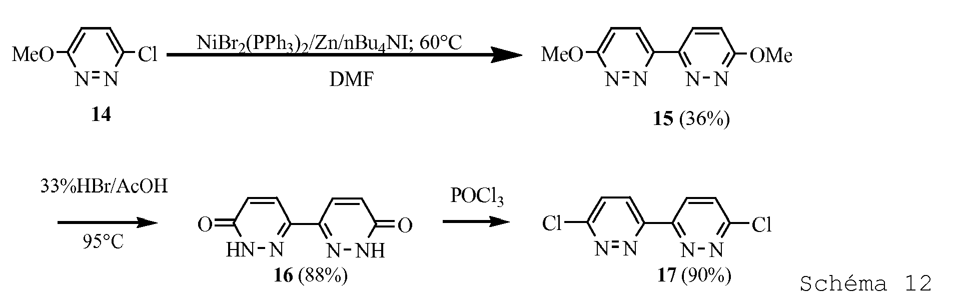

Le motif bi-pyridazine introduit dès la première étape possédant des propriétés de complexation potentiellement gênantes lors des extractions, une seconde synthèse a été envisagée. L'objectif de la deuxième voie de synthèse est d'introduire ce motif à la dernière étape.

### Voie 2 :

La stannylpyridine (**18**) est mise en présence de 3-chloro-6-méthoxypyridazine (**14**) dans les conditions de couplage de Stille. La méthoxypyridazine (**102**) est obtenue avec un rendement de 77%. Après hydrolyse acide et chloration, la pyridazinone (**7**) et la chloropyridazine (**8a**) sont obtenues avec des rendements respectifs de 40% et 77%. La bipyridazine (**19**) est obtenue après une réaction d'homocouplage de la 3-chloro-6-pyridylpyridazine (**8a**) en présence de dibromobistriphénylphosphine de nickel (II) avec un rendement de 12%.

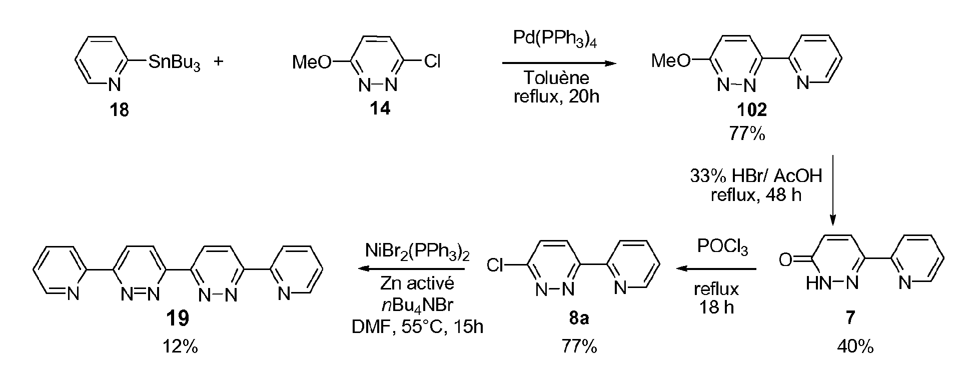

Une troisième voie de synthèse a été développée afin d'éviter l'usage de l'étain, de minimiser le nombre d'étapes.

### Voie 3 :

Cette voie de synthèse utilise une méthode de couplage type Negishi entre un dérivé organozincique et une pyridazine halogénée. La pyridine zincique est formée *in situ* à partir de la 2-bromopyridine en présence de butyllithium et de chlorure de zinc. Une solution de 3-chloro-6-iodopyridazine (**103**) et de tétrakis(triphénylphosphine)palladium (0) est ensuite cannulée pour donner le composé (**8a**) avec un rendement de 62% (étape originale). La bipyridazine (**19**) est obtenue selon une réaction d'homocouplage.

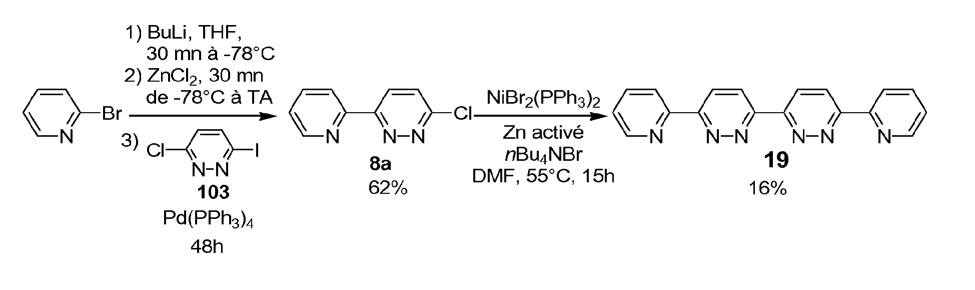

Une étude de la première étape a été effectuée afin d'optimiser le couplage de Negishi. Cette étude a permis d'augmenter les rendements de 14% à 62 % en utilisant la 3-chloro-6-iodo-pyridazine (**103**) au lieu de la 3,6-dichloropyridazine à température ambiante. La réaction donne uniquement le produit de mono-substitution, le produit de di-substitution n'apparaissant que lors du chauffage, de façon minoritaire.

| Température | -78°C à TA | -78°C à 60°C | -78°C à TA | -78°C à TA | -78°C à 60°C |
|---|---|---|---|---|---|
| Temps de réaction | 24h | 24h | 24h | 24h | 24h |
| 2-bromopyridine | 1.6 éq | 0.9 éq | 1.6 éq | 1.6 éq | 1.6 éq |
| nBuLi | 1.6 éq | 0.9 éq | 1.6 éq | 1.6 éq | 1.6 éq |
| ZnCl₂ | 1.6 éq | 0.9 éq | 1.6 éq | 1.6 éq | 1.6 éq |
| dichloropyridazine | 1 éq | 1 éq | 1 éq | - | - |
| Pd(PPh₃)₄ | 0.05 éq | 0.05 éq | 0.10 éq | 0.05 éq | 0.05 éq |
| 3-chloro-6-iodopyridazine | - | - | - | 1 éq | 1 éq |
| Rendement | 18% | 14% | 48 % | 62% | |
| Masse de produit obtenu | 204 mg | 180 mg | 364 mg | 464 mg | Mélange de produits mono et disubstitué non séparables par chromatographie sur gel de silice |

Le nickel utilisé lors de la réaction d'homocouplage peut rendre difficile l'extraction de la molécule au cours de la dernière étape. Une quatrième voie a donc été développée.

### Voie 4 :

Cette voie de synthèse est quasi identique à la voie 1, le couplage de Stille étant remplacé par un couplage de Negishi pour éviter les traces d'étain dans le composé final.

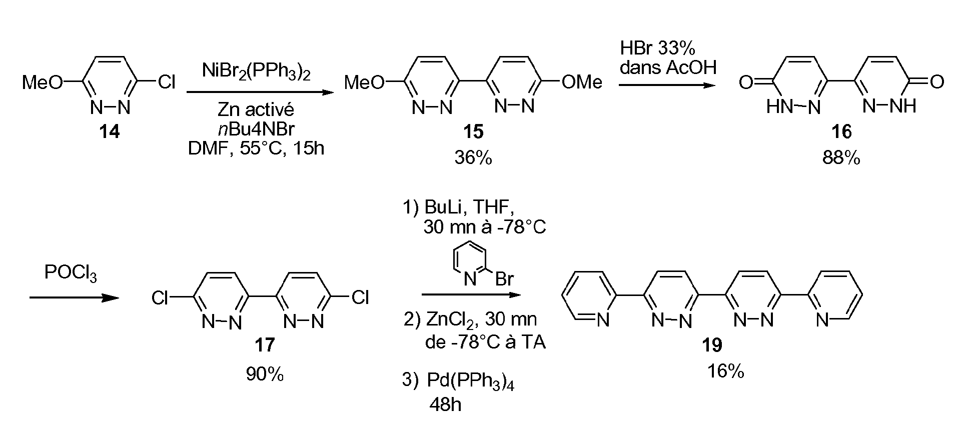

### 2. Synthèse de la 6,6'-bis(5-méthyl-pyridin-2-yl)-3,3'-bipyridazine 2 (appelée également 6,6'-di-picolin-2-yl-[3,3']bipyridazine)

### Voie 1 :

Le protocole opératoire décrit dans le schéma 12 a également été reproduit pour la préparation de la 6,6'-bis(6-méthyl-pyridin-2-yl)-3,3'-bipyridazine **2**, qui a été obtenue avec 37% de rendement. Le couplage de Stille est réalisé dans ce cas à partir de la 6,6'-dichloro-3,3'-bipyridazine **17** en présence de la 6-méthyl-3-tributylstannylpyridine **22** (Schéma 9).

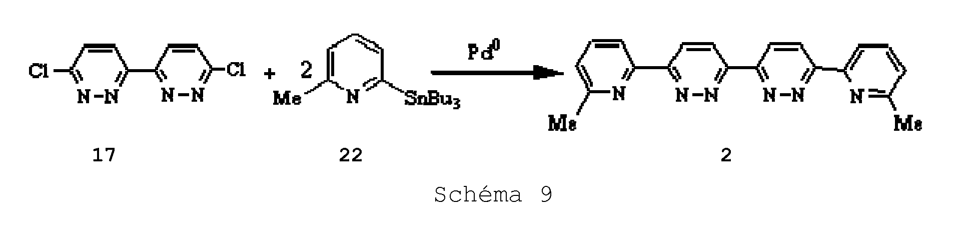

### Voie 2 :

Cette voie de synthèse correspond à la voie 4 décrite précédemment.

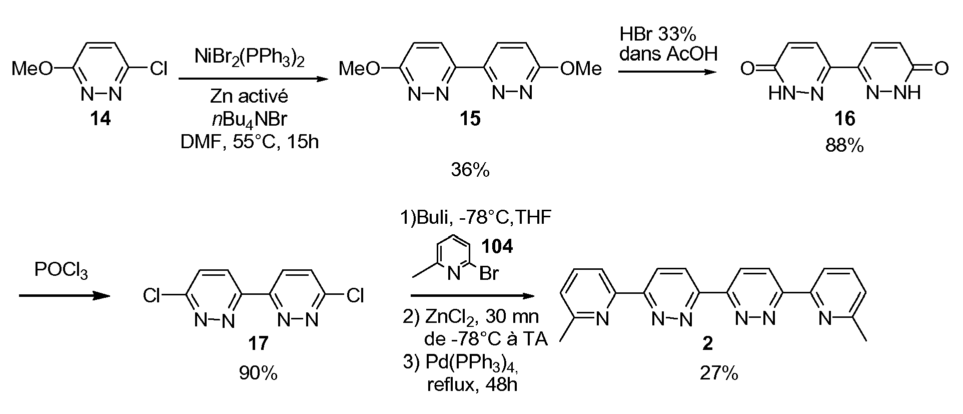

### 3. Synthèse de la 6,6'-di-picolin-4,4'-di-méthyl-2-yl-[3,3']bipyridazine (105)

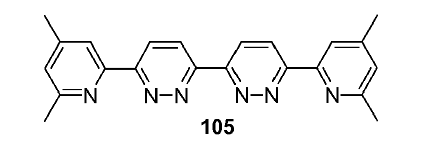

La voie de synthèse utilisée correspond également à la voie 4 du point 1. L'ajout d'un substituant méthyle supplémentaire sur le groupement 6-méthylpyridinyle permet d'augmenter la solubilité du composé et ainsi de faciliter la régression de cycle par voie électrochimique.

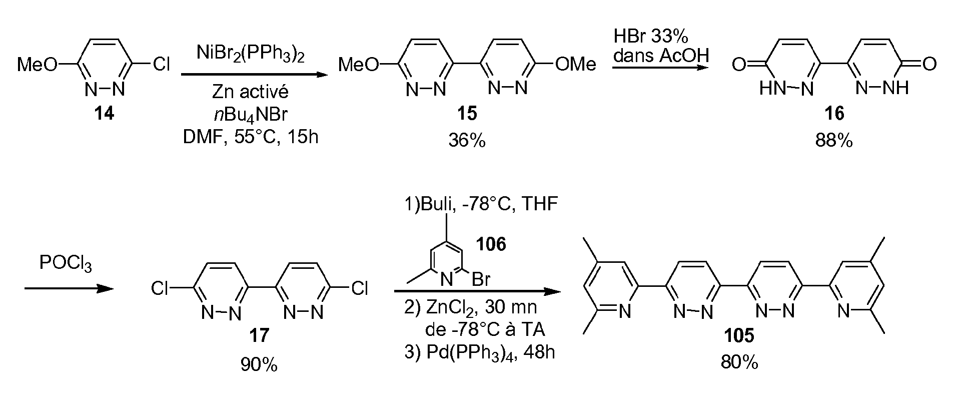

### Exemple 2 : Synthèse de 2,2,6,6'-bis(1-éthoxyvinyl)-3,3'-bipyridazine 24 via la 6,6'-dichloro-3,3'-bipyridazine 17

A partir de la 6,6'-dichloro-3,3'-bipyridazine **17** de nombreuses modifications fonctionnelles sont autorisées dont l'accès à la 6'-bis(1-éthoxyvinyl)-3,3'-bipyridazine **24** qui a été obtenue avec un rendement de 68% en présence de tributyl-(1-éthoxyvinyl)étain et de trans-bis(triphénylphosphine)palladium(II) [Pd(PPh₃)₂Cl₂], dans le DMF à 80°C (schéma 10).

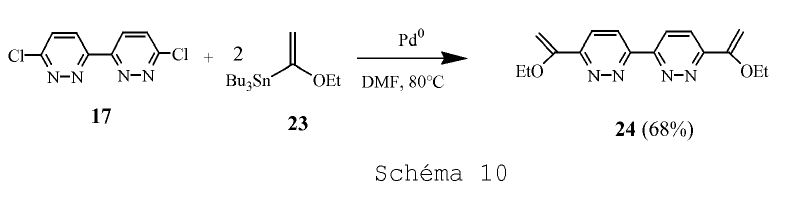

Cette molécule présente un fort potentiel cytotoxique sur des cellules cancéreuses KB avec une IC₅₀ de 0,3 µg/ml et affecte également la protéine tonB intervenant dans le processus du transport du fer utilisé pour la croissance des bactéries.

### Exemple 3 : Synthèse de ligands oligopyridaziniques linéaires par voie électrochimique

Le procédé à anode consommable (J. Chaussard, J.-C. folest, J.-Y. Nédélec, J. Périchon, S. Sibille, M. Troupel, Synthesis, 1990, 369-381) a permis de réaliser des couplages d'halogénures aromatiques et hétéroaromatiques.

### 1. Description du procédé électrochimique employé

Le couplage d'halogénures aromatiques est possible grâce à un procédé d'électrolyse indirecte catalysé par les complexes du nickel. Le procédé employé est le procédé à anode consommable. Le précurseur du catalyseur est introduit sous forme de sels de nickel (NiBr₂, xH₂O) ou par oxydation d'un barreau métallique contenant du nickel (acier inoxydable ou acier Fe/Ni 64/36)

Le matériel utilisé se présente de la façon suivante :
La cellule électrochimique est constituée d'une paroi en verre se terminant dans sa partie basse par un pas de vis sur lequel on visse un socle en bakélite (noir, SVL40) contenant un joint d'étanchéité. Dans sa partie supérieure, quatre entrées de type SVL 15 sont placées autour d'une entrée centrale SVL22 permettant d'adapter un barreau métallique qui jouera le rôle d'anode. La cathode, constituée d'une mousse de nickel (40 cm²), est placée de façon concentrique autour de l'anode.
L'agitation du solvant au sein de la cellule est assurée par un barreau aimanté. Les différentes entrées latérales ont pour fonction de permettre le raccordement électrique de la cathode au moyen d'un fil d'inox, d'autoriser l'entrée et la sortie d'un gaz comme de l'argon assurant une atmosphère inerte dans la cellule électrochimique. La quatrième entrée permet d'effectuer des prélèvements ou de rajouter des réactifs dans le milieu réactionnel en cours d'électrolyse. Une des entrées peut, si utile, servir pour placer une électrode référence pour mesurer l'évolution du potentiel de la cellule en cours de réaction.

La cellule est placée sur un bain d'huile-agitateur magnétique, permettant un éventuel chauffage, si nécessaire. Le DMF est le solvant employé dans le procédé. Le milieu est rendu conducteur grâce à l'introduction d'électrolytes supports tels que des sels d'ammonium quaternaires. L'alimentation électrique de la cellule est assurée par une alimentation stabilisée qui permet de travailler sous un régime intensiostatique de 10 à 300 mA.

Les deux réactions mises en jeu lors d'une électrolyse ont lieu simultanément. La réaction cathodique concerne la réduction de l'espèce la plus facilement réductible qui est dans le cas présent, le précurseur du catalyseur (sels de nickel II). Le nickel (II) est ainsi réduit en nickel (0) stabilisé par des ligands présents dans le milieu (pyridine ou bipyridine). A l'anode la contre-réaction est l'oxydation du barreau métallique, en fer ou d'un alliage Fer/Nickel de composition 64/36. Les sels métalliques générés dans le milieu contribuent ainsi au bon déroulement de la réaction. Le processus mis en jeu autour du nickel (0) est présenté dans les schémas 1 et 2, selon l'intensité imposée : forte (schéma 1) ou faible (schéma 2).

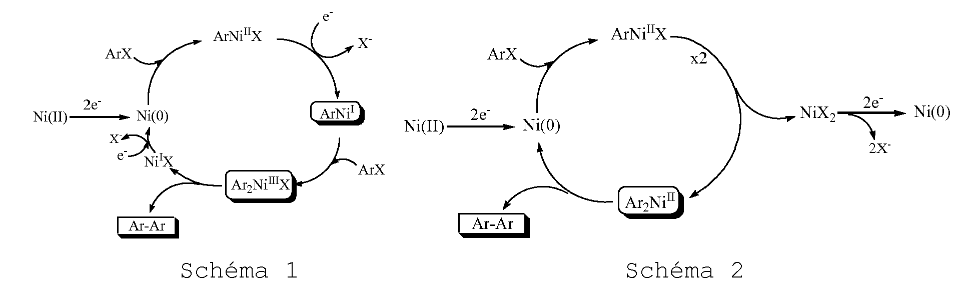

Si l'anode est en fer, le précurseur du catalyseur utilisé est le complexe NiBr₂Bipy (10%), et dans le cas d'une anode Fe/Ni (64/36) le précurseur du catalyseur est NiBr₂ (5 à 10%) et le ligand placé comme co-solvant est la pyridine.

### 2. Homocouplage d'un halogénure pyridazinique par voie électrochimique

La 6,6'-diméthoxy-3,3'-bipyridazine **15** est l'intermédiaire clé de la synthèse de 3,3'-bipyridazine 6,6'-bis substitués. Une synthèse originale simple et efficace de cet intermédiaire a été mise au point par voie électrochimique. Cette synthèse utilise le procédé à anode consommable décrit ci-dessus et met en jeu l'homocouplage de la 3-chloro-6-méthoxypyridazine **14** grâce à une catalyse par les complexes du nickel (Schéma 1').

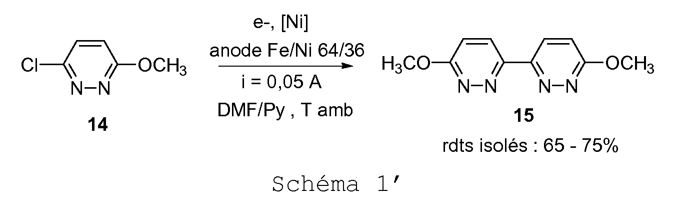

Le matériel employé est celui décrit au paragraphe 1. L'anode est un barreau Fe/Ni (64/36) et la cathode est une mousse de nickel (fournisseur Goodfellow). Le solvant est un mélange DMF/pyridine 50/50 et l'électrolyte support est constitué d'un mélange NBu₄Br/NBu₄I 1/1. La réaction est conduite à la température ambiante sous atmosphère d'argon. Une préélectrolyse en présence de dibromoéthane (300 µL) est réalisée durant 15 min sous une intensité de 0,1 A en l'absence de nickel (NiBr₂, xH₂O 10%) et du réactif (3-chloro-6-méthoxypyridazine). Ces derniers sont ensuite ajoutés et l'électrolyse est poursuivie sous une intensité de 0,05 A. L'évolution de la réaction est suivie par analyse CPG de prélèvements du milieu réactionnel qui sont hydrolysés (mélange solution aqueuse saturée d'EDTA/CH₂Cl₂) et ceci jusqu'à disparition totale de l'halogénure d'aryle (durée : environ 15 à 19h). Le solvant est évaporé sous pression réduite. Le résidu est repris dans un mélange (solution aqueuse saturée d'EDTA et de dichlorométhane) et placé sous agitation magnétique pendant une heure. La phase organique est séparée de la phase aqueuse et cette dernière est extraite avec du CH₂Cl₂ (4 fois 100 mL). Les phases organiques rassemblées sont séchées sur sulfate de sodium anhydre, filtrées et concentrées sous pression réduite. Le résidu obtenu est purifié par chromatographie sur alumine neutre (élution 100% CH₂Cl₂) .

### Données expérimentales :

*6,6'-diméthoxy-3,3'-bipyridazine **1**, CAS RN [24049-46-5] :*
*Cristaux blancs ; **Masse obtenue** = 695 mg ; **Rendement** =* 64 % ; *(purification sur gel d'alumine, éluant : dichlorométhane 100%).* ***Point* de** ***fusion** : 238-239°C (litt. : 237-238°C)*
***RMN ¹H (CDCl₃, 300 MHz**,* δ ***ppm)** : 8, 60 (d, 2H, J = 9,3 Hz) ; 7,11 (d, 2H, J = 9,3 Hz), 4,19 (s, 6H, OCH₃).*
***RMN ¹³C (CDCl₃,* 75 *MHz,*** δ ***ppm)** : 165,47 ; 152,47 ; 127,41 ; 118,21 ; 55,07.*
***SM (EI) M*/*****Z (%)** : 219 (13), 218 (100), 217 (57), 189 (32), 175 (33), 147 (31), 119 (12).*

De même, la préparation de la 6,6'-dichloro-3,3'-bipyridazine **17** peut être réalisée par homocouplage électrochimique de la 3,6-dichloropyridazine **1'** selon la réaction suivante (Schéma 2') :

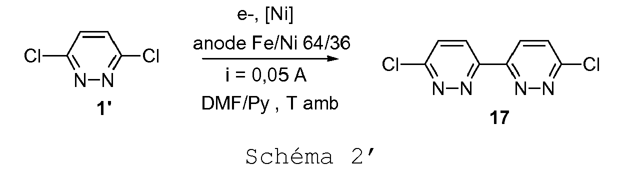

### 3. Hétérocouplage d'un halogénure pyridazinique par voie électrochimique

La réaction mise en oeuvre est la suivante (Schéma 4') :

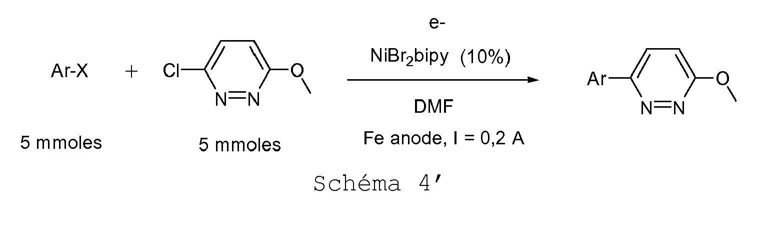

Les hétérocouplages ont été conduits, à la différence des homocouplages, sous une intensité de 0,2 A. Le précurseur du catalyseur est le complexe NiBr₂bipy ajouté en quantité catalytique (10%). L'anode est en fer (XC10, 0,1% de carbone). Le solvant est le DMF.

Les résultats obtenus sont rassemblés dans le tableau ci-dessous (tableau 1)

**Tableau 1 : hétérocouplage d'halogénures aromatiques avec la 3-chloro-6-méthoxypyridazine.**

| Entrée | ArX | Produit | Rdt isolé (%) |
|---|---|---|---|
| 1 | 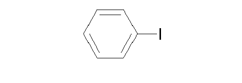 | 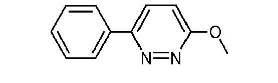 | 60 |
| 2 | 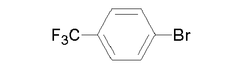 | 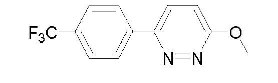 | 62 |
| 3 | 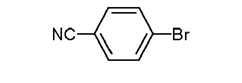 | 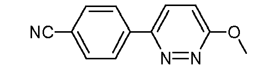 | 58 |
| 4 | 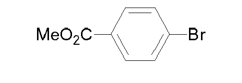 | 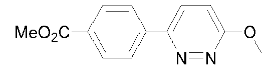 | 70* |
| 5 | 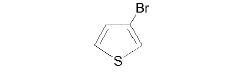 | 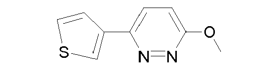 | 56* |
| 6 | 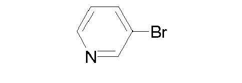 | 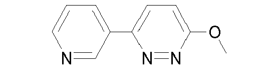 | 33 |

| | | | |
|---|---|---|---|
| *intensité 0,05 A | | | |

Les rendements obtenus sont de l'ordre de 60% sauf pour la 3-bromopyridine (entrée 6) où une diminution est observée (33%). Pour le para-bromobenzoate de méthyle (entrée 4) et le 3-bromothiophène (entrée 5), les réactions ont été conduites à 0,05 A.

Selon le schéma rétrosynthétique présenté ci-dessous (Rétrosynthèse 1'), il est donc possible d'envisager deux approches vers les bipyridazines par couplages électrochimiques.

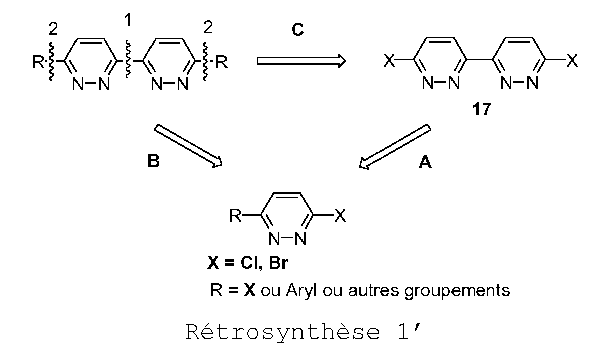

Pour la voie rétrosynthétique A, un couplage permettant d'obtenir la 6,6'-dichloro-3,3'-bipyridazine **17** par homocouplage de la 3,6-dichloropyridazine **1'** est envisagé (voir ci-avant).

A partir de **17**, de nombreuses structures bipyridaziniques sont accessibles par voie chimique ou électrochimique pour lesquelles R est variable.

Concernant la voie rétrosynthétique B, la synthèse de 6,6'-bis(pyridin-2-yl)-3,3'-bipyridazines **19** et **2** est réalisée en deux étapes électrochimiques selon le Schéma 7' *via* l'intermédiaire **2**'.

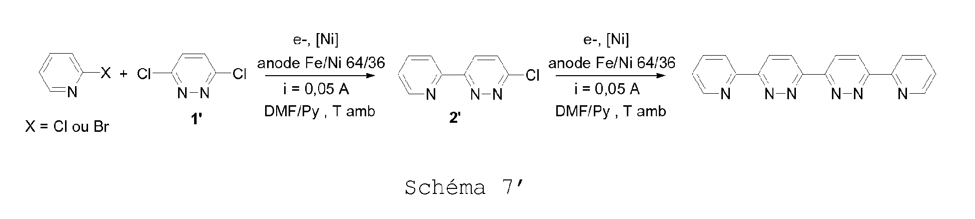

De plus, cette voie d'étude permet d'obtenir des analogues bipyridaziniques non symétriques pour lesquels R et R' sont variables (Schéma 8').

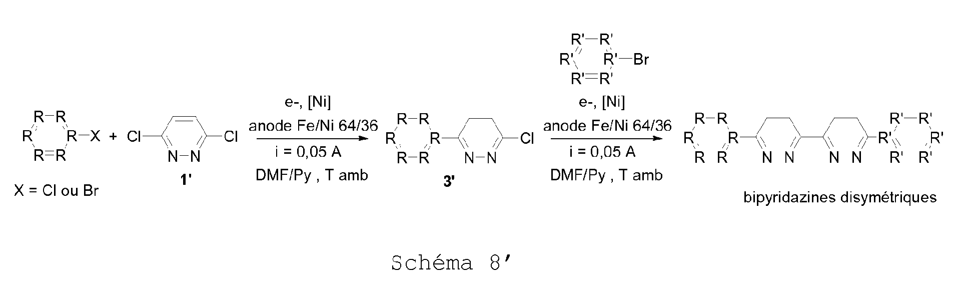

### Exemple 4 : Synthèse d'enchaînements bipyrroliques par électrosynthèse

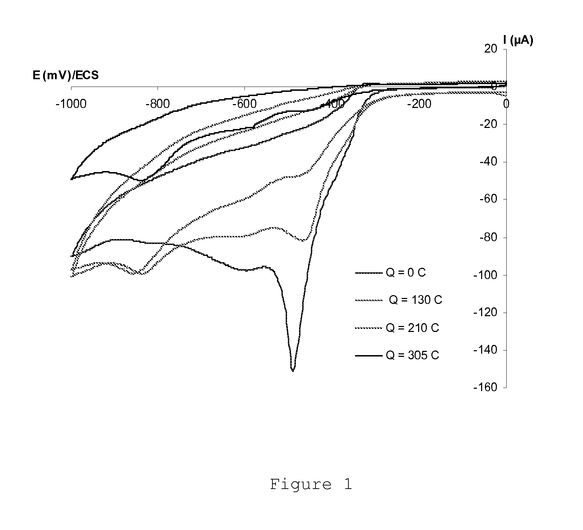

Quelques structures bipyridaziniques ont été soumises aux conditions de régression de cycle par voie électrochimique. L'expérience d'électroréduction a été réalisée en milieu acide sulfurique car les 3,3'-bipyridazines **2** et **19** sont difficilement solubles dans le tampon acétique. Les conditions étaient les suivantes : électrode de carbone vitreux, v=100mV/s. Le voltampérogramme de la 6,6'-bis(6-méthyl-pyridin-2-yl)-3,3'-bipyridazine **2** indique clairement une vague de réduction très marquée à un potentiel de -0,5V/ECS et un léger épaulement vers -0,6V/ECS (Fig. 1)

Selon les premières études, il était concevable que la première vague puisse correspondre à un potentiel d'une réduction simultanée à deux électrons (par cycle pyridazinique), des deux groupements pyridaziniques symétriques du dimère **2** (4 électrons au total). La formation d'un intermédiaire bis-dihydropyridazinique **68** pouvait donc être envisagée et sa régression en dipyrrole **71** devait logiquement nécessiter encore un apport d'au moins quatre électrons par mole (deux électrons par cycle dihydro), pour un total de huit électrons/mol à partir de la bipyridazine **2** (Schéma 121).

L'électrolyse préparative de ce composé a ainsi été réalisée en milieu sulfurique en appliquant un potentiel de travail de -0,5V/ECS pendant une heure (Q=210C) puis celle-ci a été poursuivie à un potentiel de -0,6V/ECS jusqu'à consommation presque totale du précurseur (Q= 305C). Le suivi de cette électrolyse préparative a été réalisé par voltampérométrie cyclique directement dans le compartiment cathodique sur électrode de carbone à différents stades de l'électrosynthèse (Fig. 1 : - avant électrolyse, - - pendant l'électrolyse, - à la fin de l'électrolyse). Une diminution de l'intensité de la vague de réduction du composé bipyridazine **2** sur les différents voltampérogrammes montre bien la réduction de celui-ci au cours de l'électrolyse préparative. Cette dernière a été arrêtée après disparition totale de cette vague (4h30 d'expérience, pendant lesquelles au moins quatre électrons ont donc été consommés).

A la fin de l'électrolyse, la quantité de Coulomb consommée dans cette expérience ne correspond en fait qu'à seulement 4,78 électrons par mole de substrat bipyridazine (pour une valeur théorique de Qₜ = 440C = 8e⁻).

Cette électroréduction (Schéma 121) à 4 électrons conduit selon cette procédure majoritairement à la formation de la 3-[5-(6-méthyl-pyridin-2-yl)-pyrrol-2-yl]-6-(6-méthyl-pyridin-2-yl)-pyridazine **70**.

La réduction sélective d'un seul noyau pyridazinique sur les deux est donc possible. Le mécanisme implique soit une première réduction à 4 électrons, du dimère pyridazinique qui conduirait à des bis-1,2-dihydropyridazines **68** soit deux étapes successives de réduction à 2 électrons via l'intermédiaire **69**. Cette dernière hypothèse a tout d'abord été suggérée par l'identification dans le milieu de l'intermédiaire 3-[6-(6-méthyl-pyridin-2-yl)-dihydropyridazin-3-yl]-6-(6-méthyl-pyridin-2-yl)-pyridazine **69** résultant d'une première réduction à 2 premiers électrons.

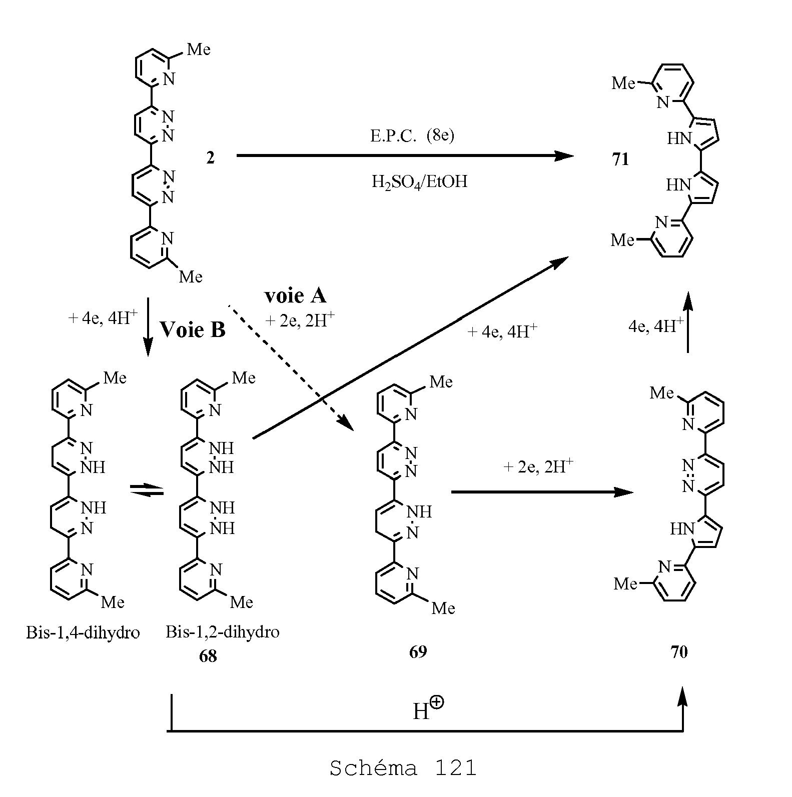

L'identification de deux composés a été confirmée par leur spectre de masse, respectivement m/z = [M-H] 341 pour le dihydro **69** et m/z = [M] 327 pour le composé pyrrolique **70.** L'analyse du spectre RMN ¹H du pyrrole **70** indique la présence d'un NH à δ=11 ppm et de 2 protons pyrroliques à δ= 6,77 et 6,78 ppm (³*J* = 3,9 Hz). Ces derniers couplent également avec le NH du pyrrole avec des constantes de couplage respectives ⁴*J* de 2,4 Hz et 2,7 Hz.

Cette alternative offre la possibilité d'accès à des systèmes alternés pyridazine-pyrrole.

Le mécanisme privilégié semble en fait devoir s'opérer vers la formation d'une bis-1,2-dihydropyridazine **68** (résultante d'une réduction à 4 électrons) qui se réarrange en milieu acide en système mixte pyrrole-pyridazine **70.** Par la suite, le bipyrrole **71** résulte d'une nouvelle réduction à 4 électrons de la pyridazine restante.

En effet, selon le protocole expérimental qui a été retenu pour cette expérience d'électroréduction, dans laquelle seulement 4,78 électrons par mole ont été consommés, la formation du bipyrrole **71,** qui demande 8 électrons par mole de bipyridazine **2,** ne pouvait pas être optimum. Sa présence a pourtant été mise en évidence dans ces conditions mais en faible proportion. En effet, la RMN ¹H du brut réactionnel indique sans ambiguïté le pic NH des pyrroles à δ=9,65 ppm pour cette molécule symétrique et son spectre de masse est conforme (m/z = [M+1] 313).

***RMN ¹H (300MHz, CDCl₃) de 71** :9.65 (sl, 2H, NH); 7.45 (t, 2H, ³J = 7.5Hz, 2H4'pyridinyl); 7.31 (d, 2H, ³J = 7.5Hz, 2H3'pyridinyl); 7.31 (d, 2H, ³J = 7.5Hz, 2H5'pyridinyl); 6.66 (m, 2H, 2H pyrrole) ; 6.41 (m, 2H, 2H pyrrole) ; 2.49 (s, 6H, CH3)*

### Electrolyse préparative de la bipyridazine 2, réduction à huit électrons

L'étude a été reprise en déplaçant progressivement le potentiel d'électroréduction vers des valeurs plus négatives de -0.7V/ECS jusqu'à disparition totale du substrat (soit une consommation de 8,15 électrons par mole de substrat). On a observé la disparition de la vague principale lors de l'expérience précédente coïncidant avec l'apparition d'une autre vague à un potentiel d'environ -0,8V/ECS (Fig. 1). Elle correspond à la réduction de la 3-[5-(6-méthyl-pyridin-2-yl)-pyrrol-2-yl]-6-(6-méthyl-pyridin-2-yl)-pyridazine **70** en bipyrrole correspondant **71.**

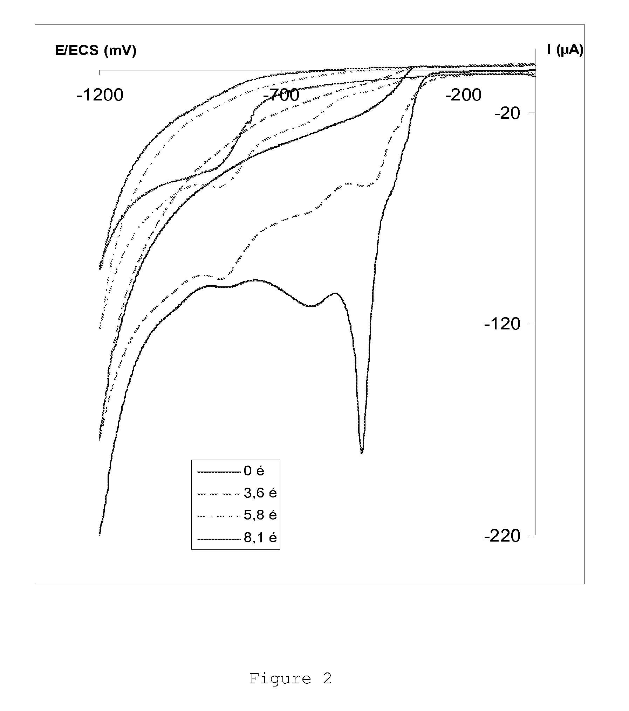

Comme dans l'électrolyse précédente, le suivi a été effectué par voltampérométrie cyclique (Fig. 2).

Sur la figure 2, on peut observer qu'après le passage de quatre électrons, il reste encore du produit de départ, caractérisé par les trois premières vagues toujours présentes. On peut également noter l'apparition d'une nouvelle vague plus cathodique qui est caractéristique du produit final de la réaction.

Le traitement, identique à l'essai précédent, donne lui aussi un mélange de produits de réaction et l'analyse RMN du brut réactionnel est très fournie en pics. Cependant, deux fractions majoritaires ont pu être isolées par chromatographie: l'une contenant une 1,2,3,4-tétrahydropyridazinyl-pyrrole **72,** et l'autre le bipyrrole attendu **71.** (Schéma 122)

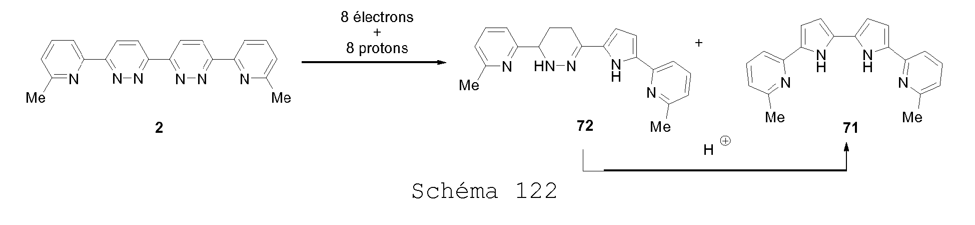

La présence de ce produit de réaction tétrahydropyridazine-pyrrole **72,** laisse conclure que les 8 électrons servent bien à réduire la pyridazine, puisque **72** représente l'intermédiaire de synthèse du bipyrrole **71.**

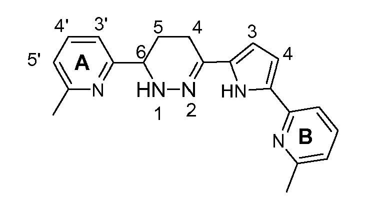

***RMN ¹H (400MHz, CDCl₃) 72:**10.07 (sl, 1H, NH pyrrole);7.62 (t, 1H, ³J = 7. 7Hz, H4'pyridinylA) ; 7.41 (t, 1H, ³J = 7.7 Hz, H4'pyridinylB) ; 7.34 (d, 1H, ³J = 7.7Hz, H3'pyridinylB); 7.26 (d, 1H, ³J = 7.7Hz, H3'pyridinylA); 7.09 (d, 1H, ³J = 7.7Hz, H5'pyridinylA) ; 6.88 (d, 1H, ³J = 7.7Hz, H5'pyridinylB); 6. 64 (m, 1H, H4 pyrrole) ; 6.33 (m, 1H, H3 pyrrole) ; 6.02 (sl, 1H, NH tétrahydropyridazine); 4.30 (dd, 1H, ³J = , 9.9, 3.09, H6 tétrahydropyridazine); 2.70 (m, 1H, H4 tétrahydropyridazine); 2. 65 (m, 1H, H4 tétrahydropyridazine); 6.64 (s, 6H, CH3); 2.54 (m, 1H, H5 tétrahydropyridazine); 2.16 (m, 1H, H5 tétrahydropyridazine);*

***RMN ¹³C (400MHz, CDCl₃) 72:**160.4 (C2' pyridinylA);* 158.2 *(C6' pyridinylA);* 157.9 *(C6' pyridinylB); 149.7 (C2' pyridinylA); 138.5 (Cq pyrrole) ;* 136.7 *(C4' pyridinylA); 136.5 (C4' pyridinylB);* 132. 6 *(Cq pyrrole) ;* 122.2 *(C3' pyridinylA); 121.0 (C3' pyridinylB);* 120.1 *(C5' pyridinylA);* 117.5 *(C5' pyridinylB);* 108.2 *(C4 pyrrole) ; 107.5 (C3 pyrrole) ; 57.5 (C6 tétrahydropyridazine); 26.0 (C5 tétrahydropyridazine); 24.6 (2 CH3) 22.1 C4 tétrahydropyridazine).*

Cependant, une amélioration du procédé peut porter soit sur le potentiel à appliquer (jusqu'à 0,85 V/ECS), soit en laissant simplement la réaction se poursuivre. Les hypothèses sont qu'à -0,7 V/ECS, on réduit la dihydropyridazine **2** en **72** et que le réarrangement en bipyrrole **71** en milieu acide requiert simplement un temps de réaction plus long.

### Exemple 5: Réduction de la 6,6'-bis(pyridin-2-yl)-3,3'-bipyridazine par électrolyse préparative à potentiel contrôlé

L'électrolyse préparative est effectuée en milieu acide sulfurique (0,5mol.L-1)/ éthanol (proportion: 0,5/0,5) dans une cellule à deux compartiments séparés par un verre fritté. Dans le compartiment anodique est placée l'anode, une plaque d'inox de surface 15 cm². Dans le compartiment cathodique sont introduits la cathode une nappe de mercure de surface 16 cm² et l'électrode de référence, l'électrode au calomel saturée. Le volume de solvant dans les deux compartiments est de 90mL Le substrat est introduit dans le compartiment cathodique (194 mg soit 5,7 10-4 mol) et le potentiel appliqué en début d'électrolyse est de -0,5V/ECS (potentiel de réduction du substrat), l'intensité correspondante est de 35 mA. Après 1 heure d'électrolyse, celle-ci est poursuivie à -0,6V/ECS jusqu'à disparition du substrat (4H30), l'intensité en fin d'électrolyse est de 8 mA. Le suivi de l'électrolyse est réalisé par voltampérommétrie cyclique sur électrode de carbone (S=3,2 mm2) directement dans le compartiment cathodique). La quantité de coulomb consommée pendant l'électrolyse est de 305C soit 5,57 électrons par mole de substrat. Le milieu réactionnel du compartiment cathodique est évaporé pour éliminer l'éthanol, puis neutralisé par NaHCO₃. Après extraction par le dichorométhane, la phase organique est séchée sur Na₂SO₄ puis évaporée. Le composé monopyrrolique 3-[5-(pyridin-2-yl)-pyrrol-2-yl]-6-(6-méthyl-pyridin-2-yl)-pyridazine est purifié par chromatographie sur gel de silice.

### Obtention du 5,5'-bis(pyridin-2-yl)-2,2'bipyrrole 112

Afin d'optimiser l'électrosynthèse du bipyrrole, la réalisation des électrolyses dans les mêmes conditions opératoires (solvant, électrodes), mais en déplaçant progressivement le potentiel d'électroréduction vers des valeurs plus négatives (E=-0,85V/ECS) conduit à la formation du système bipyrrolique.

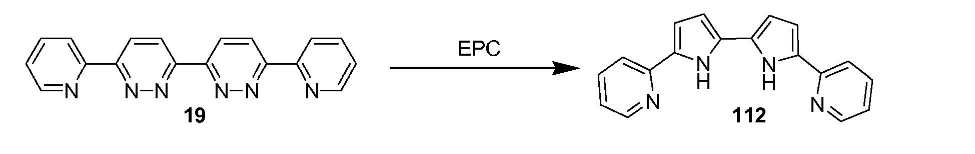

Les difficultés de solubilisation du composé **19** n'ont pas permis d'effectuer la régression électrochimique dans les conditions classiques (EtOH/Tampon acétique), seule une solubilisation dans l'acide sulfurique 0,5M est possible. Les voltampérogrammes enregistrés dans l'acide sulfurique 0,5M ont mis en évidence trois vagues de réduction successives à des potentiels E_{c} = -0,363V, -0,500V et -0,673V. Dans les mêmes conditions, le composé pyrrolique montre une vague de réduction correspondant à la réduction des pyrroles avec un potentiel E_{c} = -0.818V.

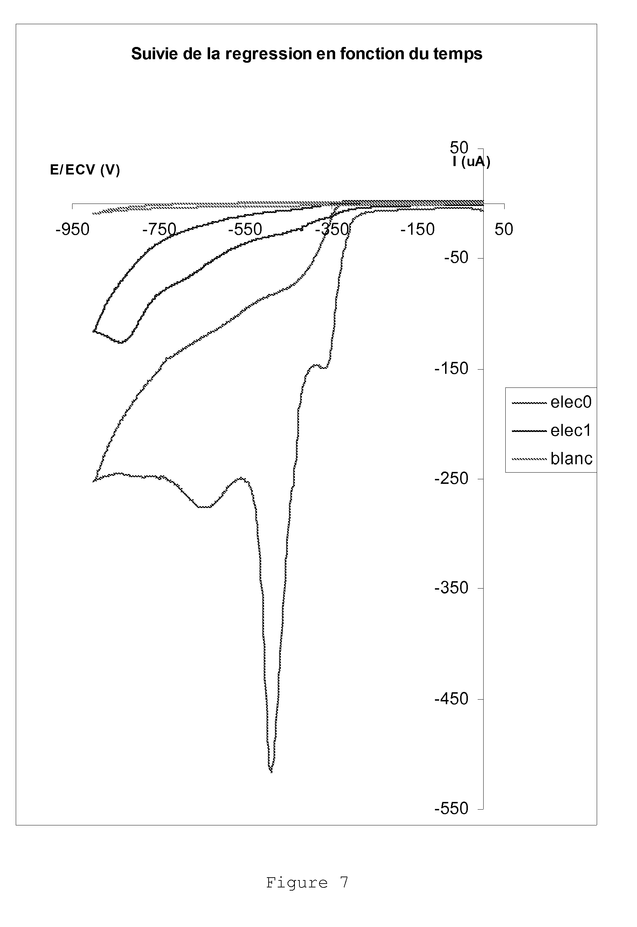

La réaction d'électrolyse préparative de la bipyridazine **(19)** par voie électrochimique a été effectuée en milieu H₂SO₄ 0,5M. Un potentiel Eₜ = -0,650V/ECS a été appliqué à l'électrode de travail (nappe de mercure) jusqu'à consommation de 8 électrons. Le suivi de la réaction est effectué par voltampérométrie cyclique et CCM (voir Fig.7).

Le composé bipyrrolique **112** a été obtenu avec un rendement voisin de 10%, ainsi que le composé monopyrrolique **107** et le composé tétrahydropyridazinique **108.** Le faible rendement obtenu pour le bipyrrole s'explique par sa dégradation partielle en milieu H₂SO₄ concentré et aux difficultés de purification par flash chromatographie sur gel de silice.

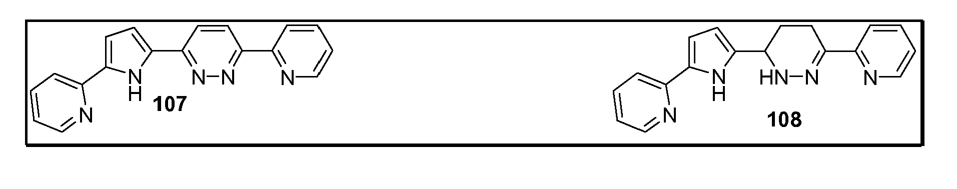

### Exemple 6: Etudes analytiques de la réduction des monopyridazines polycycliques

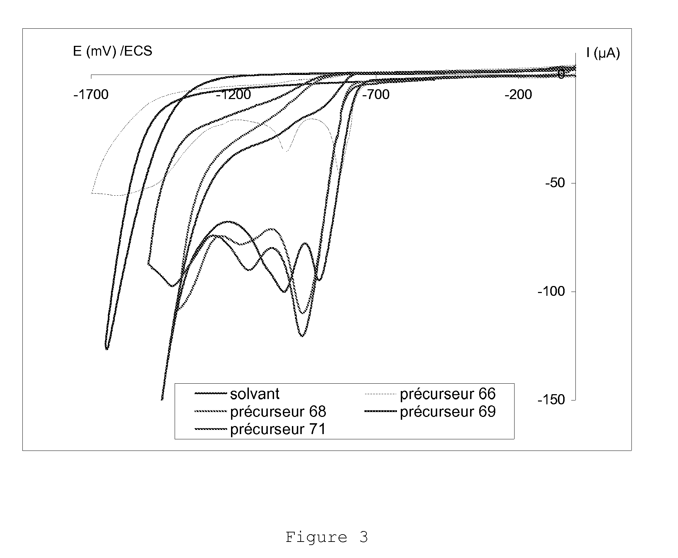

Les études analytiques montrent que les 3,6-bis(pyridin-2-yl)-pyridazines **80, 82, 83** et **85** présentent des voltampérogrammes similaires à leurs analogues 3,6-dicarbométhoxy-pyridazines. Ainsi, les premiers pics à quatre électrons sont bien définis et apparaissent à des potentiels compris entre -0.9 V et -1.1 V (Fig. 3, voltampérogramme cyclique du solvant et des précurseurs **80, 82, 83** et **85** en milieu acétique et éthanol, C = 10⁻³mol.L⁻¹, V = 100mV/s).

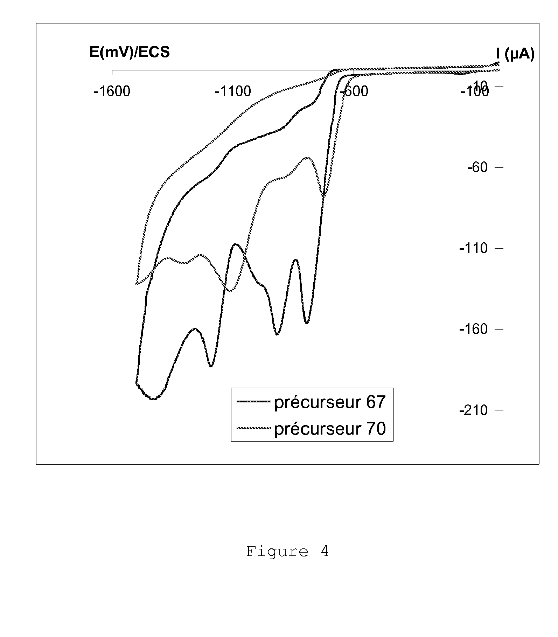

Les voltampérogrammes des précurseurs 4-carbométhoxy-3,6-bis(pyridin-2-yl)-pyridazines **84** et 3,6-bis(pyridin-4-yl)-pyridazine **81** sont plus complexes et plusieurs vagues de réduction apparaissent à partir de potentiels plus positifs (- 0.7 V) (Fig. 4, voltampérogrammes cycliques des précurseurs **81** et **84** en milieu tampon acétique + éthanol, C = 10⁻³mol.L⁻¹, V = 100mV/s).

Les valeurs de potentiels des pics Ep des deux premiers pics de réduction de ces différents précurseurs sont rassemblées dans le tableau 4.

**Tableau 4**

| Précurseur | E_{P}(pic I) | E_{P}(pic II) |
|---|---|---|
| 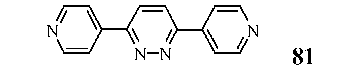 | -0.79 V | -0.91 V |
| 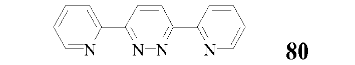 | -0.87 V | -1.06 V |
| 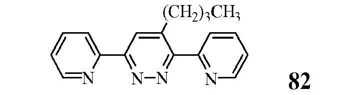 | -0.89 V | -1.08 V |
| 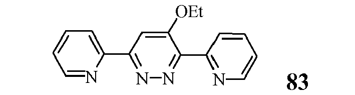 | -0.90 V | -1.02 V |
| 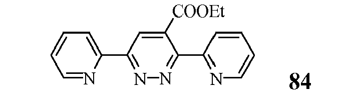 | -0.70 V | -1.11 V |
| 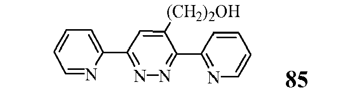 | -0.99 V | -1.18 V |

La détermination du nombre d'électrons mis en jeu par vague de réduction a été effectuée en voltampérométrie cyclique par ajout d'une référence interne (couple Red/Ox dont le nombre d'électrons est connu) dans la solution. On a utilisé comme référence interne le ferrocèneméthanol (Fc) dont le potentiel de pic d'oxydation est de 0,28V/ECS dans les conditions analytiques de l'expérience (Schéma 91)

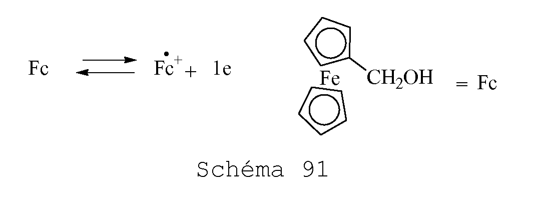

Les résultats analytiques confirment que l'électroréduction de pyridinyl-pyridazines, en pyrroles requiert quatre électrons. Le premier transfert biélectronique permet de générer les intermédiaires dihydropyridaziniques (1,2 ou 1,4-dihydropyridazines selon leur stabilité relative) et le deuxième transfert biélectronique provoque l'extrusion d'atome d'azote par dégagement d'ammoniac. Le potentiel à imposer pour les électrolyses préparatives doit donc se situer au niveau des vagues de réduction correspondant au minimum à une réduction à quatre électrons.

### Exemple 7: Electrolyse préparative de 3,6-bis(pyridinyl)-pyridazines

Les électrolyses préparatives des différentes pyridazines **(80-85)** ont été réalisées en se plaçant respectivement à un potentiel de travail correspondant au potentiel de la deuxième vague de réduction des précurseurs. Celles-ci ont été poursuivies jusqu'à la disparition totale des précurseurs et jusqu'à la consommation d'une quantité de charges (nombre de Coulombs) correspondant à la quantité minimum d'électrons nécessaire pour induire leur réarrangement en pyrrole (quatre électrons par mole du substrat réduit).

En partant pratiquement de la même concentration initiale (10⁻⁴ M), le temps moyen de la disparition totale des précurseurs est de l'ordre de 5 à 6 heures. Les produits pyrroliques **86, 87, 88, 89** et **90,** ont été obtenus avec des rendements variables allant de 60 à 90% (Schéma 92, Tableau 5).

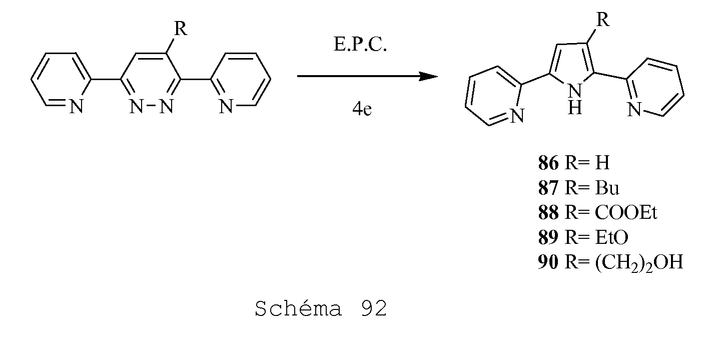

**Tableau 5**

| précurseur | E_{P}(vagueI) | E_{P}(vagueII) | E_{imposé} | ne^{-*} | t(h) | Rdt E.C.P. (pyrroles) | Rdt** chimique |
|---|---|---|---|---|---|---|---|
| **80** | -0.87 V | -1.06 V | -1.00 V | 4.06 | 4 | **86** (82%) | 22% |
| **82** | -0.89 V | -1.08 V | -1.00 V | 4.53 | 6 | **87** (85%) | 18% |
| **83** | -0.90 V | -1.02 V | -1.00 V | 4.5 | 5.20 | **88** (75%) | 30% |
| **84** | -0.71 V | -1.12V | -1.05 V | 5.66 | 6.25 | **89** (60%) | 32% |
| **85** | -0.99 V | -1.18 V | -1.10V | 4.5 | 5.33 | **90** (92%) | 25% |

| | | | | | | | |
|---|---|---|---|---|---|---|---|
| ^{*-}nombre d'électrons consommés /mol. de substrat réduit. ** Zn/AcOH | | | | | | | |

Comme l'indiquent les résultats, dans tous les cas, la régression de cycle des pyridazines en milieu tampon acétique apparaît nettement plus efficace selon le procédé électrochimique que par la voie chimique (Zn/AcOH) préconisée dans la littérature.

Ces résultats sont confirmés dans le cas de la pyridin-4-yl-pyridazine **81** dont la transformation en pyrrole **91** s'effectue avec 85% de rendement au lieu de 30% par voie chimique (Tableau 6).

**Tableau 6**

| précurseur | E_{P}(vagueI) | E_{P}(vagueII) | E_{imposé} | ne^{-*} | t(h) | Rdt E.C.P. (pyrroles) | Rdt** chimique |
|---|---|---|---|---|---|---|---|
| **81** | -0.79 V | -0.91 V | -0.95 V | 3.77 | 4.6 | **91** (85%) | 30% |

| | | | | | | | |
|---|---|---|---|---|---|---|---|
| *⁻nombre d'électrons consommés /mol. de substrat réduit. ** Zn/AcOH | | | | | | | |

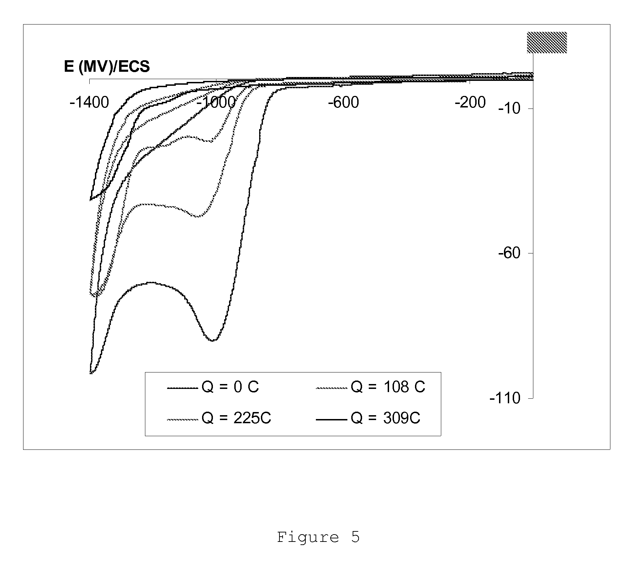

L'avancement des réactions par électrolyse préparative a été contrôlé dans chaque cas par voltampérométrie cyclique directement dans le compartiment cathodique sur électrode de carbone vitreux, comme l'illustrent les voltampérogrammes enregistrés au cours de l'électrolyse du précurseur **82** (Fig. 5, voltampérogrammes cycliques au cours de l'électrolyse préparative du précurseur **82,** - avant électrolyse, - - pendant l'électrolyse, - à la fin de l'électrolyse, électrode de carbone vitreux, v=100mV/s))

La diminution de l'intensité de la vague de réduction (-1.08V) du précurseur au cours de l'électrolyse préparative est corrélée à la transformation au cours de l'électrosynthèse. De plus, celle-ci disparaît totalement en fin d'électrolyse permettant ainsi de vérifier la consommation totale du précurseur.

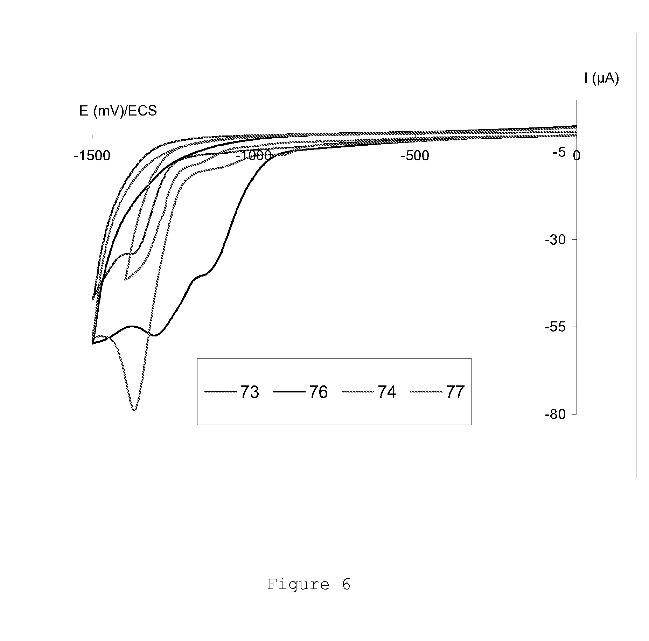

A la fin de l'électrolyse, des voltampérogrammes ont été également contrôlés (Fig. 6, voltampérogrammes des différents dérivés pyrroliques en fin d'électrolyse préparative dans le compartiment cathodique, électrode de carbone vitreux, v = 100mV/s). Ils montrent effectivement la disparition des deux vagues de réduction, ce qui confirme l'hypothèse mécanistique proposée. La première vague est attribuée aux réductions biélectroniques des pyridazines en dihydropyridazines et la deuxième correspond à l'extrusion d'azote pour conduire aux cycles pyrroliques.

### Exemple 8: Electrolyse préparative de la 6,6'-di-picolin-4,4'-di-méthyl-2-yl-[3,3']bipyridazine (105)

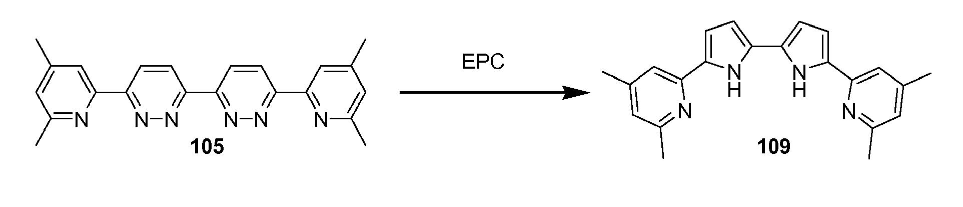

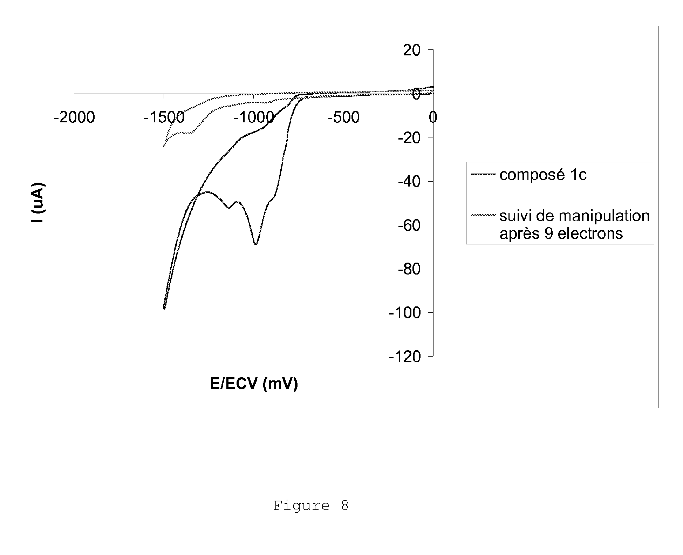

La solubilité de la bipyridazine a été améliorée par l'ajout de chaînes alkyles aux substituants pyridiniques (composé **105).** Il est alors possible de solubiliser cette bipyridazine **105** dans des conditions plus douces que les milieux H₂SO₄ ou tampon acétique: un mélange de solvants THF/Tampon acétique (pH=4.6) /Acétonitrile. Les voltampérogrammes enregistrés dans ces conditions ont mis en évidence trois vagues de réduction successives à des potentiels E_{c} = -0,92V, -1,03V et -1,16V (voir Fig.8).

L'électrolyse préparative de la bipyridazine **(105)** à E_{T} = -1.05 eV a permis d'isoler le bipyrrole **(109)** avec un rendement de 35% (tableau ci-dessous, entrée 1). Les résultats obtenus confirment l'hypothèse selon laquelle le faible rendement obtenu lors de l'électrolyse préparative de la bipyridazine **(105)** est dû à une dégradation du composé bipyrrolique dans l'acide sulfurique.

| | | | |
|---|---|---|---|
| 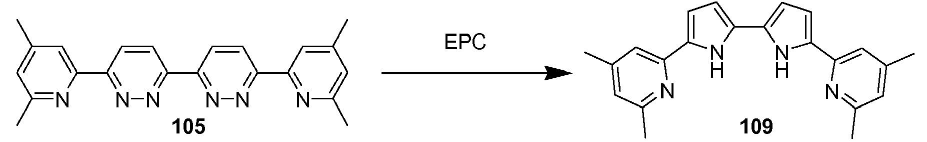 | | | |

| Milieu | | Potentiel appliqué | résultats |
|---|---|---|---|
| THF/tampon acétique | 1 | Eₜ=-1,05V, 10,3 e | Rendement = 35% |
| pH=4,6/Acétonitrile: 50/45/5 Polarogramme | 2 | Eₜ=-1,15V, 8 e Arrêts pour volta traces. | Mélange de 2 composés non séparés dont du bipyrrole **(109)** |
| Ec=-0,92V,-1,03V, - 1,16V | 3 | Eₜ=-1,15V, 10,1 e Sans s'arrêter + 1h de pause à la fin avant extraction **(111)** 20% | pyrrole-pyridazine **(110)** 20% pyrrole-tétrahydropyridazine |
| | 4 | Eₜ=-0,9V, 3,78 e, pause 40mn Eₜ=-0,97V, 4 e, pause 40mn Eₜ=-1,15V, 3,1 e, laissé tourné la nuit | RMN brut produits de dégradation: poubelle |

Plusieurs expériences ont été réalisées à différents potentiels et dans différentes conditions (tableau ci-dessus, entrée 2 à 4). Seule une modification (entrée 3) s'est montrée intéressante puisqu'elle permet d'isoler deux intermédiaires: le monopyrrole-pyridazine **(110)** et le monopyrrole-tétrahydropyridazine **(111)** avec des rendements respectifs de 20%.

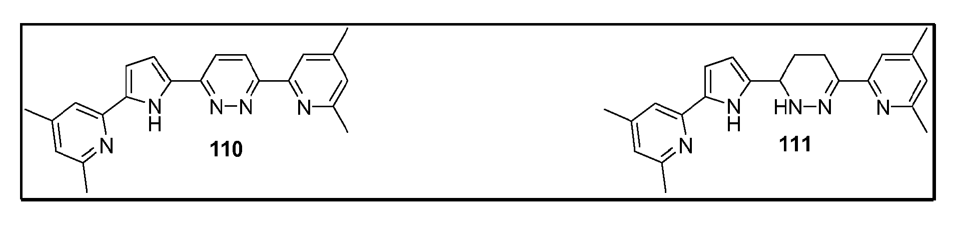

### Exemple 9: Préparation de la 3-(2-bromo-pyridin-6-yl)-6-(pyridin-2-yl)-pyridazine 43

Le dérivé chloropyridazinique **8a** traité en présence de distributylstannyle et de tétrakistriphénylphosphine de palladium conduit à un mélange de produits **39, 40** et **41.**

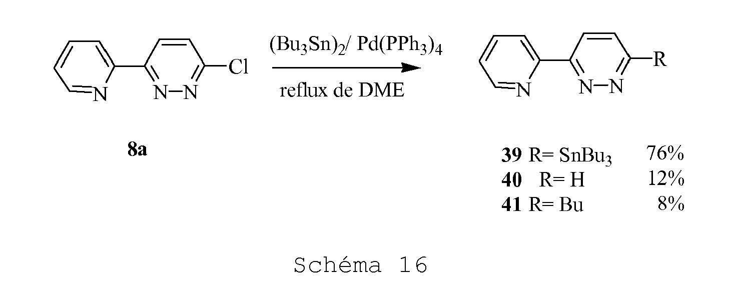

L'intermédiaire monosubtitué, 3-(2-bromo-pyridin-6-yl)-6-(pyridin-2-yl)-pyridazine **43,** est isolé de façon majoritaire avec un rendement de 72%, en partant d'un mélange équimolaire de la pyridazine stannylée **39** et de la dibromopyridinyle **42** en présence de tétrakistriphénylphosphine de palladium dans le toluène (Schéma 17).

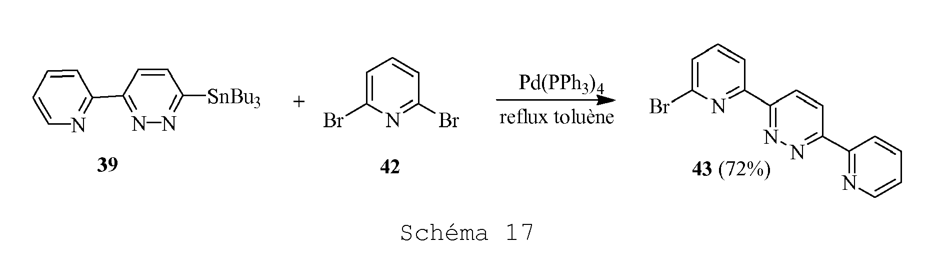

### Exemple 10: Synthèse de la 3-(2-carboxy-pyridin-6-yl)-6-(pyridin-2-yl)-pyridazine 45

Pour introduire la monofonctionnalisation, la chloro(pyridyl)-pyridazine **8a** a été choisie comme précurseur dans le couplage Stille pour réagir avec la stannylpyridine méthylée **22** (Schéma 19), en présence équimolaire de tétrakistriphénylphosphine de palladium, pour fournir exclusivement le produit de couplage **44** avec un rendement de 90%.

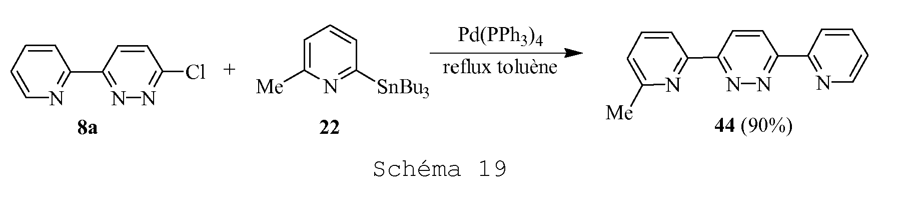

Il est à noter que dans ce cas, aucune trace de produit d'homocouplage bispyridinique n'est observée dans le mélange réactionnel.

L'oxydation du méthyle aromatique en a de l'azote pyridinique a été effectué à l'aide du dioxyde de sélénium, à 150°C dans l'*o*-dichlorobenzène, et la 3-(2-carboxy-pyridin-6-yl)-6-(pyridin-2-yl)-pyridazine **45** a été obtenue avec un rendement de 74% (Schéma 20).

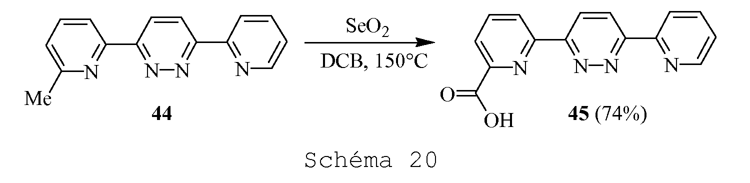

### Exemple 11: Synthèse du diacide bis-tridenté: 3,6-bis-(2-carboxyl-pyridin-6-yl)-pyridazine 48

Selon le même principe, le ligand pyridazinique diacide **48** peut être obtenu par oxydation de la bis(diméthylpyridyl)-pyridazine **47** (Schéma 21). Ce dernier a été préparé *via* un double couplage de Stille entre la 6-méthyl-2-tributhylstannyl-pyridine **22** et la 3,6-dichloropyridazine **46** en présence de tétrakistriphenylphosphine de palladium. Dans ce cas, un léger excès de pyridine stannylée (3 eq.) a été utilisé. Dans les mêmes conditions d'oxydation précédentes, on isole l'acide 3,6-bis(2-carboxyl-pyridin-6-yl)-pyridazine **48** avec 68% de rendement.

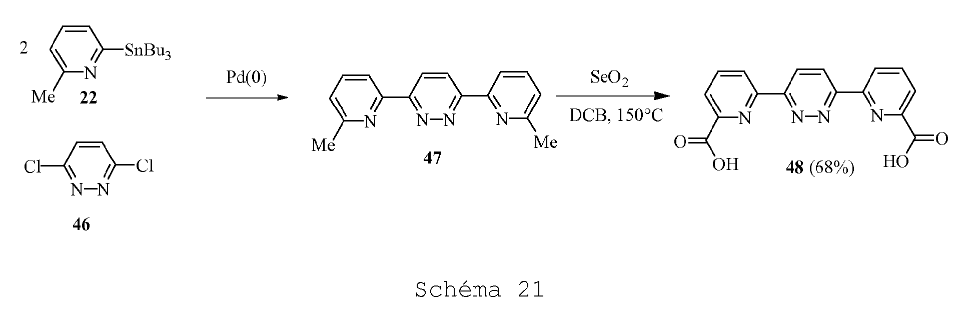

Le diacide pyridazinique **48** obtenu est un ligand bis-tridenté: *N*-donneur (pyridine et pyridazine) et *O*-donneurs (diacide). Dans cet exemple, la présence de deux atomes d'azote adjacents dans le cycle pyridazinique contribue à générer deux sites de coordination distincts.

### Exemple 12: 6,6'-bis(6-méthyl-pyridin-2-yl)-3,3'-bipyridazine 2

| | |
|---|---|
| | **C₂₀H₁₆N₆** |
| 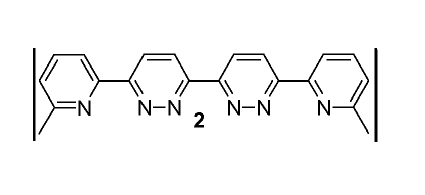 | **M = 340.38** |
| | **Rendement=27%** |
| | **Poudre jaune pâle** |

- Ce composé est synthétisé selon la procédure E à partir de la 2-bromo-6-méthylpyridine **(104)** et la 6,6'-dichloro-[3,3']bipyridazine **(17).** Le résidu est recristallisé à chaud dans l'AcOEt pour donner le produit désiré avec un rendement 27%.

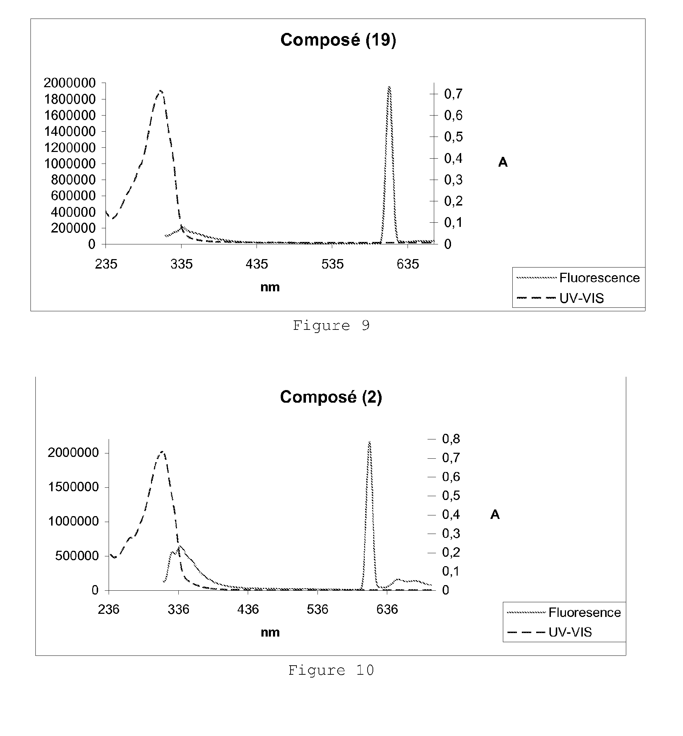

**¹H RMN** (CDCl₃) δ(ppm): 7.45 (m, 2H, H_{pyridine}), 7.94 (dt, *J* = 7.2, 1.8, 2H, H_{pyridine}), 8.79 (m, 6H, 2H_{pyridazine,} 4H_{pyridine}), 8.80 (d, *J* = 9.0, 2H, H_{pyridazine})_{,} 9.01 (d, *J* = 9.0, 2H, H_{pyridazine}).
**¹³C RMN (CDCl₃)** δ(ppm): 121.8, 125.0, 125.4, 125.5, 137.3, 149.6, 153.1, 156.0, 159.2.
**MS, m/z** (I%): 340 (M⁺, 100%), 312 (M⁺-N₂, 49%).
**UV/Fluorescence (DCM):** voir Fig. 10

### Exemple 13: 6-(pyridin-2-yl)-2H-pyridazin-3-one 7

| | |
|---|---|
| | **C₉H₇N₃0** |
| 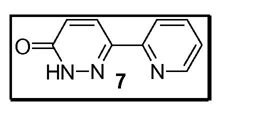 | **M = 173.17** |
| | **Rendement = quantitatif** |
| | **Poudre blanche** |

Ce composé est préparé selon la procédure B avec la 3-méthoxy-6-(pyridin-2-yl)-pyridazine **(102)** (0.29g, 1.55mmol) et une solution d'HBr (33% dans l'acide acétique, l.2mL). Le produit obtenu sous la forme d'un solide blanc de façon quantitative.

**¹HRMN (DMSO-d6**) δ(ppm): 7.01 (d, *J* = 9. 6, 1H, H_{pyridazine}), 7.42-7.46 (m, 1H, H_{pyridine}), 7.91-7.93 (m, 1H, H_{pyridine}), 8.04 (d, *J* = 8.1, 1H, H_{pyridine}), 8.27 (d, *J* = 9.9, 1H, H_{pyridazine}), 13.32 (bs, 1H, NH).
**¹³C RMN (DMSO-d6)** δ(ppm): 118.8, 123.5, 129.3, 130.1, 136.8, 142.9, 148.5, 151.3, 160.1.
**MS, m/z (I%):** 173 (M⁺, 71%), 145 (M⁺-N₂, 10%), 117 (M⁺-CCONH₂, 100%).

### Exemple 14: 3-chloro-6-(pyridin-2-yl)-pyridazine 8a

| | |
|---|---|
| 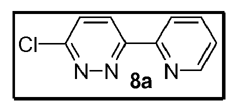 | **C₉H₆ClN₃** |
| | **M = 191.62** |
| | **Poudre brunâtre** |

- Ce composé est préparé selon la procédure C à partir de la 6-(pyridin-2-yl)-2H-pyridazin-3-one **(7)** (4.3g, 24.8mmol) et du POCl₃ (30mL). La 3-chloro-6-(pyridine-2-yl)-pyridazine **(8a)** est obtenue sous la forme d'un solide brunâtre (5.84mg, quantitative).
- Ce composé est préparé selon la procédure E à partir de la 2-bromopyridine (0.604ml, 6.33mmol), de chlorure de zinc (1.05g, 6.33 mmol), de buthylithium (6.33 mmol) et de la 3-chloro-6-iodo-pyridazine **(103)** (942mg, 3.95mmol) et le tétrakis(triphénylphosphine)palladium (0) (450mg, 0.39mmol). Le produit désiré est obtenu avec un rendement de 62%.

**RMN ¹H (CDCl₃)** δ ppm: 7.37-7.42 (ddd, *J* = 7.5, 4.5, 1.2, 1H, H*_{pyridine}*), 7.63 (d, *J* = 9.0, 1H, *H_{pyridazine}*)_{,} 7.89 (dt, *J* = 7.8, 1.8, 1H, H_{pyridine}), 8.55 (d, *J* = 9.0, 1H, H_{pyrlazin}), 8.64 ( d, *J* = 8.7, 1H, H_{pyridine}), 8.71 ( d, *J* = 4.5, 1H, H_{pyridine}).
**RMN ¹³C (CDCl₃)** δ ppm: 121.5, 124.9, 126.9, 128.6, 137.2, 149.4, 152.3, 156.8, 157.8.
**MS, m/z (I%):** 191 (M⁺, 28%), 163 (M⁺-N₂, 5%), 128 8 (M⁺-(N₂+Cl), 100%).

### Exemple 15: 3-bromo-6-(pyridin-2-yl)-pyridazine 8b

| | |
|---|---|
| 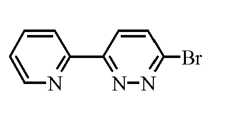 | **C₉H₆BrN₃** |
| | **M = 236.07** |
| | **Rdt = 75 %** |
| | **Cristaux jaunâtres.** |

1 g (5.78 mmol) de 6-(pyridin-2-yl)-2*H*-pyridazin-3-one **7** et un excès (5 g) d'oxybromure de phosphore sont chauffés au reflux pendant 12 heures. Le mélange réactionnel est versé dans 100 mL d'eau glacée puis neutralisé en goutte à goutte par une solution aqueuse saturée de NaHCO₃. Après extraction au dichlorométhane (3x30mL), la phase organique est séchée sur MgSO₄ puis concentrée sous pression réduite, pour conduire à la pyridazine-bromée **8b** avec un rendement de 75 %.

**R.M.N. ¹H (CDCl₃)** δppm: 7.39-7.43 (ddd, 1H, *J* = 0.9, 6.0, 7.5, H_{pyridine}); 7.76 (d, 1H, *J* = 8.7, H_{pyridazine}) ; 7.88 (dt, 1H, *J* = 1.8, 5.2, H_{pyridine}) ; 8.44 (d, 1H, *J* = 8.7, H_{pyridazine}) ; 8.63 (d, 1H, *J* = 8.1, H_{pyridine}) ; 8.70 (m, 1H, H_{pyridine}).
**R.M.N. ¹³C (CDCl₃)** δppm 121.55, 125.08, 126.61, 132.01, 137.27, 148.35, 149.47, 152.48, 160.00.
**SM, m/z (I%):** 237 (M⁺+H, 23%), 235 (M⁺-H, 22%), 128 (M⁺-(Br + N₂), 100%).

### Exemple 16: 6-(pyridin-2-yl)-2H-pyridazin-3-thione 10

| | |
|---|---|
| 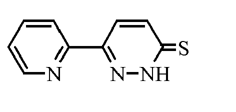 | **C₉H₇N₃S** |
| | **M = 189.24** |
| | **Rdt = 92 %** |
| | **Poudre vert-jaune.** |

500 mg (2.9 mmol) de pyridazinone **7** et 772 mg (3.5 mmol) de pentasulfure de phosphore sont dissous dans 20 mL de pyridine anhydre. Le mélange réactionnel est porté au reflux pendant 18 h, puis refroidis à température ambiante. Il est alors versé dans 200 mL d'eau et le précipité ainsi formé est filtré puis lavé avec de l'eau glacée. La pyridazinethione **10** est obtenue avec un rendement de 92 %.

**R.M.N. ¹H (CDCl₃)** δppm: 7.37-7.39 (m, 1H, H_{pyridine}); 7.81-7.84 (m, 2H, H_{Pyridine}, H_{pyridazine}) ; 8.15 (d, 1H, *J* = 7.5, H_{pyridane}); 8.23 (d, 1H, *J* = 8.7, H_{Pyridazine}) ; 8.68 (d, 1H, *J* = 4.2, H_{Pyridine}) ; 12.34 (sl, 1H, NH).
**R.M.N. ¹³C (CDCl₃)** δppm: 120.08, 120.53, 120.55, 124.85, 137.14, 137.16, 137.53, 141.53, 149.36.
**SM, m/z (I%) :** 189 (M⁺, 100%), 160 (M⁺, 35%).

### Exemple 17: 6,6'-diméthoxy-3,3'-bipyridazine 15

| | |
|---|---|
| 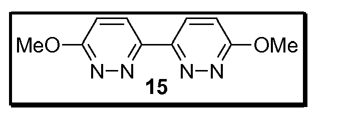 | **C₁₀H₁₀N₄O₂** |
| | **M= 218,21** |
| | **Rendement = 36%** |
| | **Poudre blanchâtre** |

Ce composé est synthétisé selon la procédure A, avec le bromure de tétrabutylammonium (4.291g, 13.31mmol), du zinc activé en poudre (870mg, 13.31mmol), le dibromobistriphénylphosphine nickel (II) (2.963g, 3.99mmol), et la 3-chloro-6-méthoxypyridazine (1.924g, 13.31mmol). En fin de réaction une solution d'ammoniaque (25 N) est additionnée lentement et le milieu est extrait au DCM. La phase organique est séchée sur Na₂SO₄, filtrée et concentrée sous vide. Le composé est recristallisé à chaud dans l'éthanol pour donner des cristaux blanchâtres (494 mg) avec 36% de rendement.

**¹H RMN (CDCl₃)** δ (ppm) : 4.16 (s, 6H, OCH₃); 7.10 (d, *J* = 9.3, 2H, H_{pyridazine}); 8.59 (d, *J* = 9.3, 2H, H_{pyridazine}).
**¹³C RMN (CDCl₃)** δ (ppm): 54.9, 118.0, 127.2, 152.4, 165.3.
**MS, m/z (I%):** 218 (M⁺, 100%), 189 (M⁺-N₂, 22%), 175 (M⁺-(N₂+CH₃), 31%).

### Exemple 18: 6,6'-(2,2'-H)-bipyridazin-3,3'-dione 16

| | |
|---|---|
| 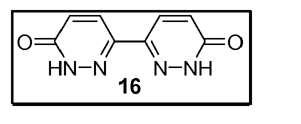 | **C₈H₆N₄O₂** |
| | **M = 190,16** |
| | **Rendement = 88%** |
| | **Poudre grise** |

Ce composé est synthétisé selon la procédure B, avec la 6,6'-diméthoxy-3,3'-bipyridazine **(15)** (712mg, 3.26mmol) et une solution d'HBr à 33% dans l'acide acétique (4mL). Le produit est obtenu sous la forme d'une poudre grisâtre (541mg, 88%).
**¹H RMN (TFA-d₁)** δ (ppm): 7.35 (d, *J* = 9.9, 2H, H_{pyridazine}), 8.38 (d, *J* = 9.9, 2H, H_{pyridazine}).
**¹³C RMN (TFA-d₁)** δ (ppm): 130.7, 134.5, 146.1, 166.3.
**MS, m/z (I%) :** 190 (M⁺, 100%), 175 (M⁺-N₂, 57%).

### Exemple 19: 6,6'-dichloro-3,3'-bipyridazine 17

| | |
|---|---|
| 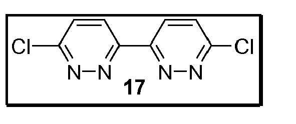 | **C₈H₄Cl₂N₄** |
| | **M = 227,05** |
| | **Rendement = quantitatif** |
| | **Poudre brunâtre** |

Ce composé est synthétisé selon la procédure C, avec 5mL de POCl₃ et la 1H,1'H-[3,3']Bipyridazinyl-6,6'-dione **(16)** (541 mg) pour donner la 6,6'-dichloro-[3,3']bipyridazine **(17)** sous la forme d'un solide brunâtre (584mg, quantitatif).
**RMN ¹H (CDCl₃)** δ ppm: 7.73 (d, *J* = 8.7, 2H, H_{pyridazine}); 8.77 (d, *J* = 8.7, 2H, H_{pyridazine}).
**RMN ¹³C (CDCl₃)** δ ppm: 126.9, 127.6, 129.2, 130.8.
**MS, m/z (I%):** 228 (M⁺+H, 27%), 227 (M⁺, 8%), 226 (M⁺-H, 41%), 163 (M⁺-(N₂+Cl), 100%).

### Exemple 20: 6,6'-bis(pyridin-2-yl)-3,3'-bipyridazine 19

| | |
|---|---|
| 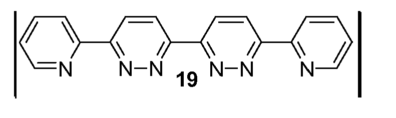 | **C₁₈H₁₂N₆** |
| | **M = 312.33** |
| | **Rendement=16%** |
| | **Poudre jaune pale** |

- La 6,6'-bis(pyridine-2-yl)-3,3'-bipyridazine **(19)** est préparée selon la procédure D. La 6,6'-dichloro-[3,3']bipyridazine **(17),** la 2-tributylstannylpyridine **(18),** le tétrakis(triphénylphosphine)palladium (0) et le DMF fraîchement distillé et dégazé sont chauffés à 80°C pendant 24h. Le résidu est recristallisé à chaud dans l'AcOEt pour donner le produit désiré avec un rendement 15%.
- Ce composé est obtenu selon la procédure A à partir de la 3-chloro-6(pyridin-2-yl)-pyridazine **(8a)** avec un rendement de 12%.
- La 6,6'-bis(pyridine-2-yl)-3,3'-bipyridazine **(19)** est préparée selon la procédure E. La 6,6'-dichloro-[3,3']bipyridazine **(17),** la 2-bromopyridine, le chlorure de zinc, le tétrakis(triphénylphosphine)palladium (0) et le DMF fraîchement distillé et dégazé sont chauffés à 80°C pendant 48h. Le résidu est recristallisé à chaud dans l'AcOEt pour donner le produit désiré avec un rendement 16%.

**¹H RMN (CDCl₃**) δ(ppm): 7.45 (m, 2H, H_{pyridine}), 7.94 (dt, *J* = 7.2, 1.8, 2H, H_{pyridine}), 8.79 (m, 6H, 2H_{pyridazine,} 4H_{pyridine}), 8.80 (d, *J* = 9.0, 2H, H_{pyridazine}), 9.01 (d, *J* = 9.0, 2H, H_{pyridazine}).
**¹³C RMN (CDCl₃)** δ(ppm): 121.8, 125.0, 125.4, 125.5, 137.3, 149.6, 153.1, 156.0, 159.2.
**MS, m/z (I%):** 312 (M+, 100%), 284 (M⁺-N₂)_{,} 255 (M⁺-2N₂, 55%), 91 (PyCH⁺, 89%).
**UV-vis/Fluorescence (CH₂Cl₂):** voir Fig. 9

### Exemple 21: 6,6'-di-(1-éthoxyvinyl)-3,3'-bipyridazine 24

| | |
|---|---|
| 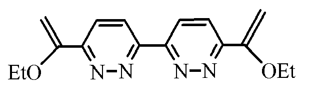 | **C₁₆H₁₈N₄O₂** |
| | **M = 298.34** |
| | **Rdt = 66 %** |
| | **Poudre jaunâtre.** |
| | **F = 214°C** |

Dans un ballon de 100 mL muni d'un barreau magnétique et d'un réfrigérant,on introduit 500 mg (2.21 mmoles ) de dichlorobipyridazine (), 1.591 g (4.42 mmoles ) de 1-ethoxyvinyl)tri(n-butyl)stannique, 77.4 mg (0.11 mmoles ) de dichlorobis(triphénylphosphine) palladium (II) et 50 ml de DMF fraîchement distillé sont introduit. Le milieu réactionnel est agité au reflux durant 24 h. Après refroidissement à température ambiante, le milieu réactionnel est dilué avec 80 ml de dichlorométhane puis versé dans une solution saturée en KF, après filtration, le filtrat est lavé avec une solution aqueuse saturée en NaHCO₃, la phase organique est séché sur MgSO₄ et concentrée sous pression réduite. Le résidu obtenu est chromatographié sur un gel de silice (éluant un mélange de acétate d'éthyle/éther de pétrole (20/80). 6,6'-di-(1-éthoxyvinyl)-3,3'-bipyridazine (18) est isolé avec un rendement de 66 %.

**R.M.N. ¹H (CDCl₃)** δppm : δ1.47 (t, 6H, *J* = 6. 9 Hz, CH₃); 4.03 (q, 4H, *J* = 6.9 Hz, OCH₂); 4.57(d, 2H, *J* = 2.4 Hz, H_{vinyl}); 5.85(d, 2H, *J* = 2.1 Hz, H_{vinyle}); 8.00(d, 2H, *J* = 9.0 Hz, H_{pyridazine}); 8.81(d, 2H, *J* = 8.7 Hz, H_{pyridazine}).

### Exemple 22: 6-(pyridin-2-yl)-3-(tridutylstannyl)-pyridazine 39

| | |
|---|---|
| 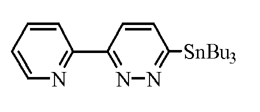 | **C₂₁H₃₃N₃Sn** |
| | **M = 446.22** |
| | **Rdt = 76 %** |
| | **Huile jaune.** |

Dans un tube de Schlenk surmonté d'un réfrigérant, 1 g (5.24 mmol) de chloropyridazine **8a** et 190 mg (0.79 mmol) de tétrakistriphénylphosphine palladium (0) sont solubilisés dans 40 mL de DME fraîchement distillé. Le milieu est dégazé à froid et sous vide. Après retour à température ambiante, on additionne 3.04 g (5.24 mmol) d'hexabutylditin. La solution est chauffée au reflux pendant 18 heures. Le solvant est évaporé sous pression réduite, et le résidu est purifié par chromatographie sur un gel d'alumine neutre (éluant: éther de pétrole / acétate d'éthyle = 95/5), la pyridazine stannylé **39** est obtenue avec un rendement de 76 %.
**R.M.N.¹H (CDCl₃)** δppm : 0.84-0.92 (m, 9H, CH₃); 1.19-1.37 (m, 12H, CH₂); 1.56-1.62 (m, 6H, CH₂); 7.33-7.37 (m, 1H, H_{pyridine}) ; 7.63 (d, 1H, *J* = 8.4, H_{Pyridazine}); 7.84 (dt, 1H, *J* = 1.8, 7.6, H_{pyridine}) ; 8.35 (d, 1H, *J* = 8.4, H_{Pyridazine}) ; 8.67-8.72 (m, 2H, 2H_{pyridine}).
**R.M.N. ¹³C (CDCl₃)** δppm : 9.99, 13.52, 27.16, 28.87, 121.28, 121.36, 124.35, 134.12, 136.98, 149.15, 154.08, 156.38, 174.85.

### Exemple 23: 3-(6-bromo-pyridin-2-yl)-6-(pyridin-2-yl)-pyridazine 43

| | |
|---|---|
| 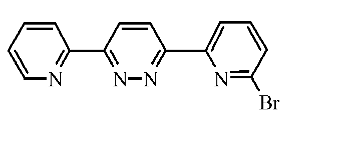 | **C₁₄H₉BrN₄** |
| | **M = 313.15** |
| | **Rdt = 72 %** |
| | **Poudre blanche.** |

La 3-(6-bromo-pyridin-2-yl)-6-(pyridin-2-yl)-pyridazine **43** a été préparée selon la méthode générale de couplage Stille, en partant d'un mélange de 1.6 g (6.74 mmol) de 2,6-dibromobipyridine **42,** 3 g (6.74 mmol) de 6-(pyridin-2-yl)-3-(tributylstannyl)-pyridazine **39,** 546 mg (0.47 mmol) de tétrakis(triphénylphosphine) palladium **(0)** et 50 mL de toluène fraîchement distillé. Le mélange est chauffé au reflux pendant 24 h. Le résidu obtenu est chromatographié sur un gel de silice (éluant: acétate d'éthyle /éther de pétrole = 1/9). On isole le produit bromé **43** avec un rendement de 72 %.

**R.M.N.¹H (CDCl₃)** δppm : 7.40-7.44 (m, 1H, H_{pyridine}); 7.59 (dd, 1H, *J* = 7.5, *J* = 0.9, H_{pyridazine}) ; 7.76 (t, 1H, *J* = 7.5, H_{pyridine}) ; 7.88-7.94 (m, 1H, H_{pyridine}) ; 8.64-8.76 (m, 5H, 4H_{Pyridine}, 1H_{pyridazine}).
**R.M.N.** ¹³**C (CDCl₃) δ**ppm **:** 121.36, 121.90, 126.92, 124.97, 125.00, 125.48, 129.19, 133.12, 137.47, 139.50, 143.13, 149.36, 149.38.
**SM, m/z (I%) :** 314 (M⁺+2, 92%), 312 (M⁺, 89%), 284 (M⁺- N₂, 35%), 205 (M⁺-(N₂+Br), 100%).

### Exemple 24 : 3-(6-méthyl-pyridin-2-yl)-6-(pyridin-2-yl)-pyridazine 44

| | |
|---|---|
| 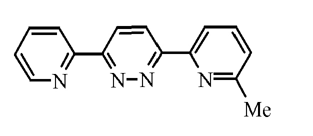 | **C₁₅H₁₂N₄** |
| | **M = 248.28** |
| | **Rdt = 90 %** |
| | **Poudre blanche.** |
| | **F = 107°C** |

La 3-(6-méthyl-pyridin-2-yl)-6-(pyridin-2-yl)-pyridazine **44** a été préparée selon la méthode générale de couplage Stille, en partant d'un mélange de 2 g (5.23 mmol) de 6-méthyl-2-tributylstannylpyridine **22**, 666 mg (3.49 mmol) de 3-chloro-6-(pyridin-2-yl)-pyridazine **8a,** 208 mg (0.18 mmol) de tétrakis(triphénylphosphine) palladium (**0**) et 50 mL de toluène fraîchement distillé. Le milieu réactionnel est agité au reflux durant 18 h. Le résidu obtenu est chromatographié sur un gel de silice (éluant **:** acétate d'éthyle /éther de pétrole **=** 2/8). On isole le produit de couplage **44** avec un rendement de 90 %.

**R.M.N.** ¹**H (CDCl₃)** δppm **:** 2.62 (s, 3H, CH₃) ; 7.22 (d, 1H, *J* = 7.8, H_{pyrlaazlne} ) ; 7.37 (ddd, 1H, *J* **=** 0**.**9, 4**.**8, 7**.**5, H_{pyridine} ) ; 7.76 (t, 1H, *J* **=** 8**.**1, H_{Pyridine}) **;** 7.68 (dt, 1H, H_{Pyridine}) **;** 8.52 (d, 1H, *J* = 7.8, H_{pyridazine}) **;** 8.61-8.75 (m, 4H, 4H_{Pyridine}).
**R.M.N. ¹³C (CDCl₃)** δppm : 24.49, 118.69, 121.66, 124.34, 124.69, 124.99, 125.19, 137.21, 137.35, 149.33, 152.60, 153.42, 157.89, 158.27, 158.33.
**SM, m/z (I%) :** 248 (M⁺**,** 94%), 220 (M⁺- N₂**,** 100%), 205 (M⁺-(N₂+CH₃)**,** 35%)**.**
**SMHR**
**Masse exacte calculée [M+H] = 249.1140**
**Masse exacte trouvée [M+H] = 249.1141**

### Exemple 25 : 3-(2-carboxy-pyridin-6-yl)-6-(pyridin-2-yl)-pyridazine 45

| | |
|---|---|
| 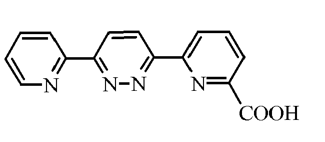 | **C₁₅H₁₀N₄O₂** |
| | **M = 278.27** |
| | **Rdt = 74 %** |
| | **Poudre rouge-orange.** |
| | **F = 224°C** |

Dans un ballon de 100 mL muni d'un réfrigérant, sont introduits 400 mg (2.42 mmol ) de pyridazine **44,** 177 mg (1.60 mmol) de dioxyde de sélénium, et 7 mL d'*o*-dichlorobenzène. Le mélange est chauffé à 150°C pendant 4 heures, puis refroidis à température ambiante. Un excès d'eau est additionné au précipité formé qui est filtré et lavé à l'eau. Le solide obtenu est séché, pour conduire à l'acide **45** avec un rendement de 74 %.

**R.M.N. ¹H (DMSO-d₆)** δppm : 7.59 (ddd, 1H, *J* = 1.2, 4.8, 7.5, H_{Pyridine}) ; 8.07 (dt, 1H, *J* = 2.1, 8.1, H_{Pyridine}) ; 8.1-8.3 (m, 2H, H_{Pyridine} ) ; 8.64 (d, 1H, *J* = 8.4 , H_{Pyridine} ) ; 8.72 (d, 1H, *J* = 9.0, H_{Pyridazine}) ; 8.79 (m, 1H, H_{Pyridine}) ; 8.82-8.85 (m, 2H, H_{Pyridine}, H_{Pyridazine}) ; 9.71 (sl, 1H, COOH).
**R.M.N**. ¹³**C (DMSO-d₆)** δppm : 121.1, 123.98, 125.09, 125.28, 125.48, 125.72, 137.65, 139.11, 148.32, 149.70, 152.53, 152.65, 157.12, 157.96, 165.64.
**SM, m/z (I%)** : 279 (M⁺, 39%), 278 (M⁺-H, 100%), 250 (M⁺-N₂, 32%), 205 (M⁺- (N₂+COOH), 80%).
**SMHR**
**Masse exacte calculée [M] = 278.0804**
**Masse exacte trouvée [M] = 278.0781**

### Exemple 26 : 3,6-bis(6-méthyl-pyridin-2-yl-)-pyridazine 47

| | |
|---|---|
| | **C₁₆H₁₄N₄** |
| 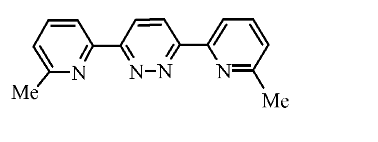 | **M = 262.31** |
| | **Rdt = 52 %** |
| | **Cristaux jaunâtres.** |
| | **F = 141°C** |

La 3,6-bis(6-méthyl-pyridin-2-yl)-pyridazine **47** est obtenue selon la procédure générale de Stille, en partant d'un mélange réactionnel de 1.55 g (4.06 mmol) de 6-méthyl-2-tributylstannyl-pyridine **22**, 300 mg (2.03 mmol) de 3,6-dichloropyridazine **46**, 231 mg (0.20 mmol) de tétrakis(triphénylphosphine) palladium (0) et 50 mL de toluène fraîchement distillé. Le milieu réactionnel est agité à reflux durant 18 h et le résidu obtenu est purifié par chromatographie sur gel de silice (éluant : acétate d'éthyle /éther de pétrole = 3/7). On isole le produit disubstitué **47** avec un rendement de 52 %.

**R.M.N. ¹H (CDCl₃)** δppm : 2.65 (s, 6H, CH₃) ; 7.24 (d, *J* = 6.3, 2H, H_{Pyridine}) ; 7.77 (t, *J* = 7.8, 2H, H_{Pyridine} ) ; 8.54 (d, *J* = 8.7, 2H, H_{Pyridine}) ; 8.68 (s, 2H, H_{Pyridazine}).
**R.M.N. ¹³C (CDCl₃)** δppm : 24.35, 118.60, 124.18, 124.96, 137.14, 137.22, 152.78, 158.17, 158.21.
**SM, m/z (I%)** : 262 (M⁺, 100%), 234 (M⁺-N₂, 85%), 142 (M⁺-(N₂+Py-CH₃), 48%).
**SMHR**
**Masse exacte calculée [M+H] = 263.1297**
**Masse exacte trouvée [M+H] = 263.1317**

### Exemple 27 : 3,6-bis(2-carboxy-pyridin-6-yl)-pyridazine 48

| | |
|---|---|
| | **C₁₆H₁₀N₄O₄** |
| 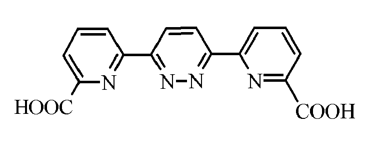 | **M = 322.28** |
| | **Rdt = 68 %** |
| | **Poudre orange.** |
| | **F > 250°C** |

Le diacide **48** est obtenu suivant une procédure similaire à la préparation de l'acide **45**, à partir d'un mélange de 230 mg (0.88 mmol) de pyridazine **47**, 126 mg (1.14 mmol) de dioxyde de sélénium, et 7 mL d' o-dichlorobenzène. Le mélange est chauffé à 150°C pendant 12 heures, pour conduire au diacide **48** avec un rendement de 68 %.

**R.M.N. ¹H (DMSO-d₆)** δppm : 8.21-8.29 (m, 4H, H**_{Pyridine}**) 8.82-8.85 (m, 2H, H_{Pyridine}, H_{Pyridazine}) ; 13.41 (ls, 2H, COOH).
**R.M.N. ¹³C (DMSO-d₆)** _{.}δppm : 124.13, 125.53, 125.83, 139.15, 148.38, 152.64, 157.38, 165.64.
**SM, m/z (I%) :** 322 (M⁺, 100%), 294 (M⁺-N₂, 41%), 278 (M⁺-COOH, 47%).
**SMHR**
**Masse exacte calculée [M-H] = 321.0624**
**Masse exacte trouvée [M-H] = 321.0623**

### Exemple 28 : 6-méthyl-2-tributylstannyl-pyridine 22

| | |
|---|---|
| | **C₁₈H₃₃NSn** |
| 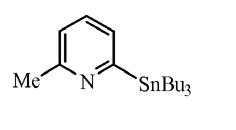 | **M = 382.17** |
| | **Rdt = 82 %** |
| | **Huile incolore.** |

Dans un tube de Schlenk à -10°C, 1.7 mL (2.6 mmol) de butylllithium (1.5M dans l'hexane) sont ajoutés goutte à goutte à une solution diisopropylamine 0.4 mL (2.6 mmol) fraîchement distillée dans du THF anhydre (50 mL). Après 5 min, on ajoute 0.70 mL (2.6 mmol) d'hydrure de tributylétain. L'agitation est maintenue 30 min à 0°C. On obtient une solution vert pâle de tributylstannyllithium, qui sera refroidie à -78°C, avant d'ajouter goutte à goutte 294.5 µl (2.6 mmol) de 2-bromo-6-méthylpyridine. Le mélange est maintenu deux heures à -78°C. Après retour à température ambiante, le solvant est évaporé sous vide. Le résidu est repris au dichlorométhane puis lavé à l'eau. La phase organique est séchée sur MgSO₄, et évaporée à sec. Le produit est purifié par chromatographie sur colonne d'alumine (éluant : acétate d'éthyle/ éther de pétrole = 0.5/9.5), en isolant la pyridine stannylée **22** sous forme d'un huile jaune, avec un rendement de 82 %.

**R.M.N. ¹H (CDCl₃)** δppm : 0.86-0.91 (m, 9H, CH3) ; 1.07-1.12 (m, 12H, CH2) ; 1.28-1.36 (m, 12H, CH2) ; 1.44-1.59 (m, 12H, CH2) ; 2.54 (s, 3H, CH₃) ; 6.95 (d, 1H, *J* = 7.8, H_{pyridine}) ; 7.18 (d, 1H, *J* = 7.5, H_{pyridine}) (t, 1H, *J* = 7.5, H_{Pyridine}).
**R.M.N. ¹³C (CDCl₃)** δppm : 13.58, 13.67, 27.30, 27.81, 29.04, 120.63, 121.46, 129.32, 133.23, 158.53.

### Exemple 29 : Dibromo-bistriphénylphosphine nickel (II) :

| | |
|---|---|
| | **C₃₆H₃₀Br₂NiP₂** |
| 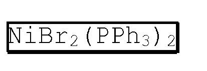 | **M = 743,07** |
| | **Rendement = 60%** |
| | **Poudre verdâtre** |

Le bromure de Nickel monohydraté (4,37g, 20mmol) et la triphénylphosphine finement broyée (10.48g, 40mmol) sont dissous séparément dans du n-butanol (50mL chacun). Les solutions sont portées à reflux jusqu'à dissolution total des réactifs. Les solutions sont ensuite mélangées à chaud. Un précipité verdâtre se forme et le milieu réactionnel est agité à reflux pendant 45 min et 1 heure à température ambiante. La solution est filtrée et le précipité est lavé avec 70 mL de n-butanol, 70 mL d'éthanol et 70 mL d'éther diéthylique. Après séchage sous vide, une poudre verdâtre est obtenue (8.85g) avec un rendement de 60%.

### Exemple 30 : Tetrakis(triphenylphosphine)palladium (0) :

| | |
|---|---|
| | **C₇₂H₆₀P₄Pd** |
| 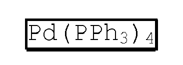 | **M = 1155.56** |
| | **Rendement = 90%** |
| | **Poudre jaune brillante** |

Dans un ballon surmonté d'un réfrigérant sous argon sont placés le chlorure de palladium (2) (0.9g, 5.09mmol) et la triphénylphosphine finement broyée (6.66g, 25.42mmol) préalablement séché sous vide. Le DMF (60mL) fraîchement distillé et dégazé est canulé dans le milieu réactionnel. La solution est agitée à 140°C jusqu'à ce qu'elle devienne translucide. La solution est refroidie à 120°C et l'hydrazine (0.99mL, 20.43mmol) est ajoutée. Une émission d'azote est immédiatement observée en même temps que la formation d'un précipité du complexe de palladium (0). Après retour à température ambiante, le précipité est filtré sous vide et argon, lavé avec de l'éthanol et de l'éther diéthylique, puis séché sous vide.

### Exemple 31 : 2-bromopicoline (104):

| | |
|---|---|
| | **C₆H₆BrN** |
| 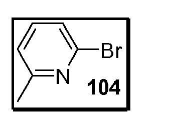 | **M = 172.02** |
| | **Rendement = 80 %** |
| | **Liquide incolore** |

Dans un ballon sous agitation mécanique la 2-amino-picoline (37.35g, 0.346 mol) est additionnée en plusieurs fois dans une solution d'acide bromhydrique (48% dans l'eau, 187mL) en maintenant la température entre 20 et 30°C. Après dissolution complète du réactif, le milieu réactionnel est refroidi à - 20°C pendant l'ajout de dibrome (49mL, 0.966mol) au goutte à goutte pendant 30 min. La température de la solution est maintenue à -20°C pendant 90 minutes. Une solution de nitrite de sodium (63.5g, 6mol) dans l'eau (100mL) est additionnée au goutte à goutte. La température de la solution est ensuite portée à 15°C en 1 heure et agitée 45 minutes à cette température. Le milieu est refroidi à -20°C et traité par une solution de soude (249g, 400mL H₂O) en maintenant la température inférieur à -10°C pendant l'addition. Après retour à température ambiante la solution est agitée 1 heure puis, extraite à l'AcOEt. La phase organique est séchée sur Na₂SO₄, filtrée et concentrée sous vide. Le résidu est distillé sous vide et la 2-bromopicoline (**104**) est obtenue sous la forme d'une huile incolore avec un rendement de 80%.
**bp** 129-132°C (2.6mbar)
**¹H RMN (CDCl₃)** δ(ppm): 2.49 (s, 3H, H₇) , 7.08 (d, *J* = 7.6, 1H, H₅), 7.24 (d, *J* = 7. 6, 1H, H₃), 7.41 (t, 1 H, H₄).

### Exemple 32 : 2-tributylstannylpyridine (18):

| | |
|---|---|
| | **C₁₇H₃₁NSn** |
| 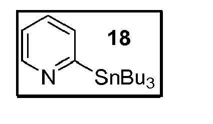 | **M = 368.14** |
| | **Rendement = 94%** |
| | **Huile jaune pale** |

Du butyllithium (2.5M dans l'hexane, 6.33mmol) est ajouté à une solution de 2-bromopyridine (1g, 6.33mmol) dans THF (12mL) fraîchement distillé et dégazé à -78°C. La solution rougeâtre est agité 30 minutes à -78°C. Du chlorure de tributylétain (1.7ml, 6.33mmol) est alors ajouté, la solution est agité 1 heure à -78°C puis 1 heure à température ambiante. Le mélange est traité par une solution saturée de NH₄Cl et extraite à l'éther diéthylique. La phase organique est lavée par une solution saturée de NaCl, séchée sur MgSO₄ et concentrée sous pression réduite. Le résidu est chromatographié sur colonne d'alumine (hexane/AcOET : 20/1) pour donner le produit pur avec un rendement de 94%.
**¹H RMN (CDCl₃)** δ(ppm): 8.73 (ddd , *J* = 4.9, 1.9, 1.0, 1H, H₆), 7.48 (dt, *J* = 7.4, 1.8, 1H, H₅), 7.39 (dt, *J* = 7.4, 1.6, 1H, H₃), 7.10 (ddd, *J* = 6.9, 4.9, 1.7, 1H, H₄), 1.70-1.05 (m, 18H, CH₂), 0.85 (t, 9H, *J* = 7.3, CH₃).

### Exemple 33 : 3-méthoxy-6-(pyridin-2-yl)-pyridazine (102):

| | |
|---|---|
| | **C₁₀H₉N₃0** |
| 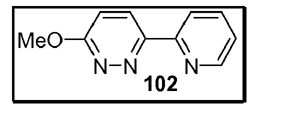 | **M** = **187.2** |
| | **Rendement = 77%** |
| | **Poudre blanchâtre** |

Ce composé est préparé selon la procédure D. La 2-tributylstannylpyridine (**18**) (1.24g, 3.36mmol), la 3-chloro-6-méthoxypyridazine (0.37g, 2.58mmol), le tétrakis(triphénylphosphine)palladium (0) (0.15g, 0.13mmol) et le toluène fraîchement distillé et dégazé (19mL) sont placé dans un ballon sous argon et chauffé à reflux pendant 20 heures. Après retour à température ambiante, le milieu réactionnel est traité par une solution d'HCl à 15% (2x30mL), lavé à l'éther diéthylique, puis une solution saturée de Na₂CO₃ est ajoutée jusqu'à l'obtention d'un pH alcalin. La solution est extraite au DCM, la phase organique est ensuite séchée sur Na₂SO₄, filtrée et concentrée sous vide. Le résidu est purifié par chromatographie sur colonne d'alumine (EP) pour donner le produit désiré avec un rendement de 77%.
**¹H RMN (CDCl₃)** δ(ppm): 4.2 (s, 3H, CH₃), 7.08 (d, *J* = 9.7, 1H, H_{pyridazine}), 7.34 (m, 1H, H_{pyridine}) , 7.83 (td, *J* = 8.8, 1.7, 1H, H_{pyridine}), 8.47 (d, *J* = 9. 7, 1H, H_{pyridazine}) 8.57 (d, *J* =8.2, 1H, Hpyridine), 8.67 (d, *J* = 5.5, 1H, H_{pyridine}).

### Exemple 34 : 2-bromo-4-méthylpicoline (106):

| | |
|---|---|
| | **C₇H₈BrN** |
| 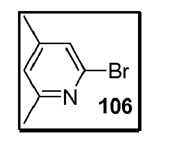 | **M = 158.02** |
| | **Rendement** = **55%** |
| | **Liquide incolore** |

La procédure utilisée est identique à celle de la 2-bromopicoline (**104**) appliquée à la 2-amino-4-méthylpicoline (10g, 81.97mmol).

**¹H RMN (CDCl₃)** δ(ppm): 2.28 (s, 3 H, CH₃), 2.48 (s, 3 H, CH₃), 6.91 (s, 1 H), 7.13 (s, 1 H).
**¹³C RMN (CDCl₃)** δ (ppm): 20.5, 23.9, 123.3, 125.6, 141.3, 150.2, 159.4.
**MS, m/z** (**I**%): 185 (M⁺, 20%), 106 (M⁺-Br, 100%), 79 (Br+, 68%).

### Exemple 35 : 3-Chloro-6-iodo-pyridazine (103):

| | |
|---|---|
| | **C₄H₂ClIN₂** |
| 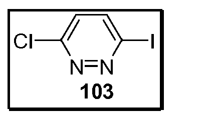 | **M = 240,43** |
| | **Rendement = quantitatif** |
| | **Poudre jaunâtre** |
| | **Rt = 9.632 mn** |

Un mélange de 3,6-dichloropyridazine (**46**) (2g, 13.42mmol), d'iodure de sodium (2g, 13.42mmol), et d'acide iodhydrique (10mL) sous atmosphère d'argon est chauffé à 40°C pendant 4h.

Après retour à température ambiante le milieu réactionnel est versé dans la glace, une solution de soude concentrée est ajoutée et l'ensemble est agité pendant 10 minutes. La solution est ensuite extraite au DCM. La phase organique lavée à l'eau, séchée sur Na₂SO₄, filtrée et concentrée sous vide. La 3-chloro-6-iodo-pyridazine (**103**) est obtenue sous la forme d'une poudre jaunâtre (3.20g, quantitatif).

**¹H RMN (CDCl₃)** δ(ppm): 7.81 (d, 1H, *J* = 9.0, H*_{pyridazine}*), 7.20 (d, 1H, *J* = 9.0, H*_{pyridazine}*).
**¹³C RMN (CDCl₃)** δ (ppm): 122.9, 129.2, 139.2, 157.1.
**MS, m/z** (**I**%): 240 (M⁺, 18%), 127 (I⁺, 100%).

### Exemple 36: 6,6'-bis(4,6-diméthylpyridin-2-yl)-3,3'-bipyridazine ou 6,6'-di-picolin-4,4'-di-méthyl-2-yl-[3,3']bipyridazine (105):

| | |
|---|---|
| | **C₂₂H₂₀N₆** |
| 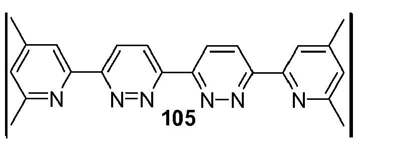 | **M = 368.43** |
| | **Rendement = 80%** |
| | **Poudre jaune pâle** |

Ce composé est préparé selon la procédure E à partir de la 2-bromo-4-méthylpicoline (**106**) et la 6,6'-dichloro-[3,3']bipyridazine (**17**). Le produit attendu est obtenu avec un rendement de 80% après une recristallisation à chaud dans l'AcOEt.

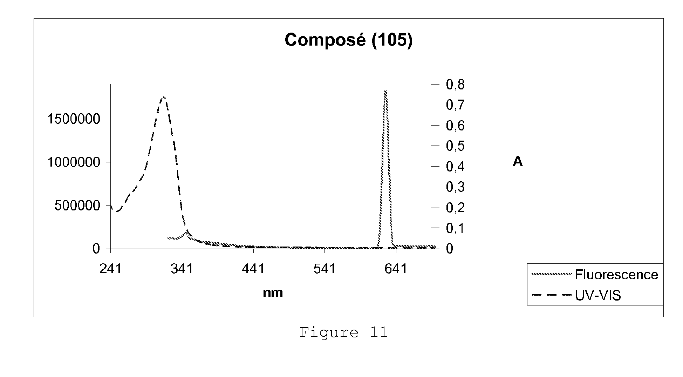

**¹H RMN (CDCl₃)** δ(ppm): 2.46 (s, 6H, CH₃), 2.63 (s, 6H, CH₃), 7.13 (s, 2H, H_{pyridine}), 8.79 (d, *J* = 8.9, 2H, H_{pyridazine}), 8.97 (d, *J* = 8.9, 2H, H_{pyridazine}).
**MS, m/z (I%):** 368.1 (M⁺, 100%), 340.1 (M⁺-N₂, 90%),
**UV/Fluorescence (DCM):** voir Fig. 11

### Exemple 37 : 5,5'-bis(pyridine-2-yl)-2,2'-bi(1H-pyrrole) (112):

| | |
|---|---|
| | **C₁₈H₁₄N₄** |
| 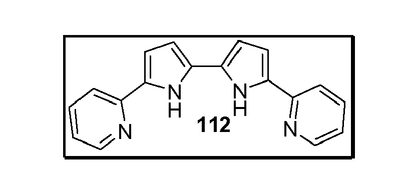 | **M** = **286.33** |
| | **Rendement** = **10** % |
| | **Huile jaunâtre** |

Ce composé est préparé selon la procédure F à partir de la 6,6'-bis(pyridine-2-yl)-3,3'-bipyridazine (**19**) et dans une solution d'H₂SO₄ 0.5M comme solvant. Le composé est obtenu après purification sur plaques préparatives de silice (AcOEt) , à l'état de trace.

**RMN (CDCl₃)** δ(ppm): 6.43(d, *J* = 3.5, 2H, H_{pyrrole}), 6.68 (d, *J* = 3.5, 2H, H_{pyrrole}), 6.97-7.01 (m, 2H, H_{pyridine}), 7.43-7.61(m, 4H, H_{pyridine}), 8.42 (d, *J* = 7.8, 2H, H_{pyridine}).

### Exemple 38 : 6-(pyridin-2-yl)-3-((5-pyridin-2-yl)-1H-pyrrol-2-yl)-pyridazine (107):

| | |
|---|---|
| | **C₁₈H₁₃N₅** |
| 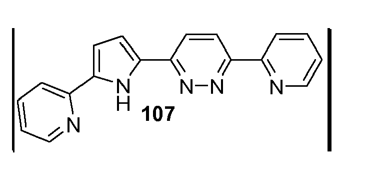 | **M = 299.12** |
| | **Yield** = **4%** |

**¹H RMN (CDCl₃)** δ(ppm): 6.73 (m, 1H, H_{pyrrole}), 6.84 (m, 1H, H_{pyrrole}), 7.06 (m, 1H, H_{pyridine}), 7.29 (m, 1H, H_{pyridine}), 7.51-7.62 (m, 2H, H_{pyridine}), 7.72 (d, *J* = 9.8, 1H, H_{pyridazine}), 8.43 (d, *J* = 9.8, 1H, H_{pyridazine}), 8.46 (m, 2H, H_{pyridine}), 8.59-8. 69 (m, 2H, H_{pyridine}), 10.89 (sb, 1H, NH)

### Exemple 39 : 5,5'-bis(3-méthylpicoline-2-yl)-2,2'-bi(1H-pyrrole) (109):

| | |
|---|---|
| | **C₂₂H₂₂N₄** |
| 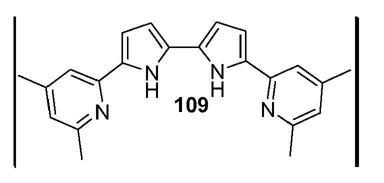 | **342.18** |
| | **Rendement** = **35**% |
| | **Poudre jaune** |

Ce composé est obtenu à partir de la 6,6'-bis(4,6-diméthylpyridine-2-yl)-3,3'-bipyridazine (**105**) avec le système de solvant THF/Tampon acétique/CH₃CN: 5/4/1 (E = -1.05V/ECS). Le résidu est chromatographié sur silice (AcOEt), le produit désiré est obtenu avec un rendement de 35%.

**¹H RMN (CDCl₃)** δ(ppm): 2.32 (s, 6H, CH₃), 2.50 (s, 6H, CH₃), 6.45 (d, *J* =, 2H, H_{pyrrole}), 6.69 (d, 2H, H_{pyrrole}), 6.73 (s, 1H, H_{pyridine}), 7.18 (s, 2H, H_{pyridine}).
**¹³C RMN (CDCl₃)** δ (ppm): 20.9, 24.3, 29.7, 106.6, 108.4, 116.7, 121.1.
**MS, m/z (I%):** 342 (M⁺, 100%), 343 (M⁺+1, 100%)

### Exemple 40 : 6-(4,6-méthylpyridin-2-yl)-3-((5-(4,6-méthylpyridin-2-yl)-1H-pyrrol-2-yl)-pyridazine (110):

| | |
|---|---|
| | **C₂₂H₂₁N₅** |
| 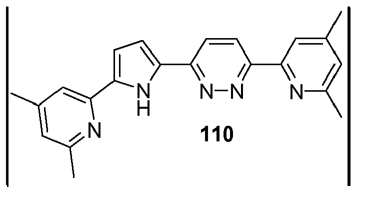 | **M = 355.44** |
| | **Yield = 20%** |
| | **Poudre jaune** |

**¹H RMN (THF-d8)** δ(ppm): 2.21 (s, 3H, CH₃), 2.30 (s, 3H, CH₃), 2.40 (s, 3H, CH₃), 2.43 (s, 3H, CH₃), 6.72 (m, 1H, H_{pyrrole}), 6.75 (s, 1H, H_{pyridine}), 6.85 (m, 1H, H_{pyrrole}), 6.98 (s, 1H, H_{pyridine}), 7.28 (s, 1H, H_{pyridine}), 7.79 (d, *J* = 9, 1H, H_{pyridazine}), 8.23 (s, 1H, H_{pyridine}), 8.37 (d, *J* = 9, 1H, H_{pyridazine}), 10 . 87 (bs, 1H, NH).
**¹³C RMN (THF-d8)** δ (ppm): 19.5, 19.7, 23.0, 29.2, 108.4, 110.7, 117.9, 118.6, 121.1, 121.3, 123.5, 123.8, 128.2, 129.6, 130.2, 134.7, 138.2, 147.6, 148.8, 151.4, 152.8, 155.5, 157.1.
**MS, m/z (I%):** 356.2(M+1, 23%), 355.2 (M⁺, 89%).

### Exemple 41 : 6-(4,6-méthylpyridin-2-yl)-3-(5-(4,6méthylpyridin-2-yl)-1H-pyrrol-2-yl)-1,4,5,6-tétrahydropyridazine (111):

| | |
|---|---|
| | **C₂₂H₂₅N₅** |
| | **M = 359.2** |
| 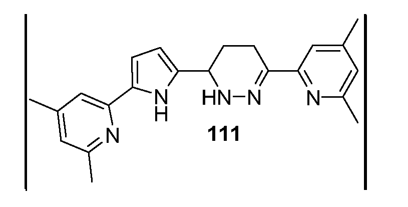 | **Yield = 20%** |
| | **Poudre jaune** |

**¹H RMN (THF-d8)** δ(ppm): 2.14 (s, 3H, CH₃), 2.17 (s, 3H, CH₃), 2.32 (S, 6H, 2CH₃), 2.14-2.49 (4H, H_{térahydropyridazine}), 4.08 (m, 1H, H_{térahydropyridazine}) 6.07 (m, 1H, H_{pyrrole}), 6.48 (m, 1H, H_{pyrrole}), 6.63 (s, 1H, H_{pyridine}), 6.78 (s, 1H, H_{pyridine}), 6.96 (s, 1H, H_{pyridine}), 7.12 (s, 1H, H_{pyridine}), 9.88 (bs, 1H, NH).
**¹³C RMN (THF-d8)** δ (ppm):
**MS, m/z (I%):** 359.2 (M⁺, 45%) , 355.2 (M⁺-2H₂, 78%).

### Exemple 42 : Activité thérapeutique des composés selon l'invention

| | | Cellules KB | | | | **Parasitologie IC50 ou 80** | | | | | |
|---|---|---|---|---|---|---|---|---|---|---|---|
| N° du composé | Structure | Cytotoxicité | | | | *Leishmaniamexicana* | | *Candidaalbicans* | | *Aspergillusfumigatus* | |
| | | %inhibition à [10⁵] | | **IC50 µM** | **IC50 µg/mL** | µM | µg/mL | µM | µg/mL | µM | µg/mL |
| 10 | 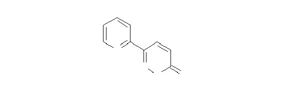 | 84 | 74 | | | | | | | | |
| 24 | 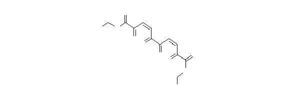 | **96** | **95** | **1,23** | **0,37** | | | | | | |
| 68 | 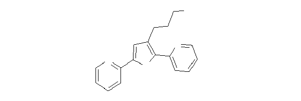 | 43 | | | | | | | | | |
| 45 | 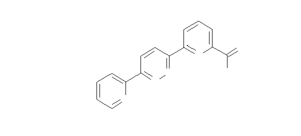 | 9 | 3 | | | 14 | 3,9 | >100 | | | |
| 44 | 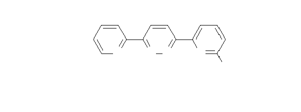 | 11 | 21 | | | **<1** | **<0,25** | **0,6** | **0,15** | | |
| 2 | 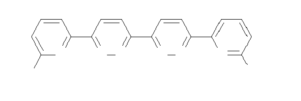 | 23 | 12 | | | **2,2** | **0,91** | 62 | 25,9 | | |
| 48 | 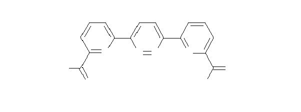 | 21 | | | | **3** | **0,96** | >100 | 32,2 | | |
| 19 | 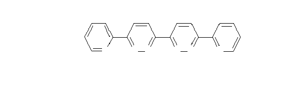 | 16 | | | | | | | | | |

### Exemple 43: Activité thérapeutique des composés selon l'invention

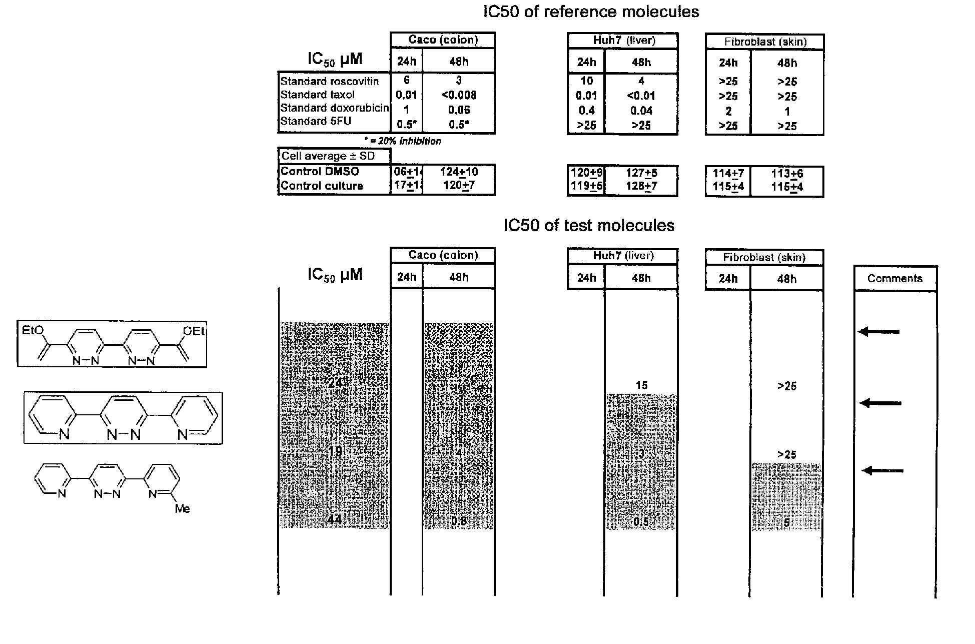

## Revendications

1. Composé de formule 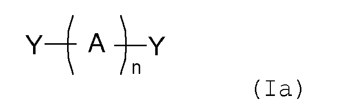 dans laquelle
• si n=1
- si A représente un groupement de formule 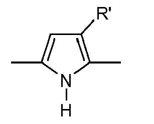 dans laquelle R' représente un hydrogène ; une chaîne alkyle, hydroxyalkyle, alkylamine, alkyloxy de 1 à 6 carbones ; un groupement -COOH, -COOR1, -CONH₂, -CONHR1, dans lesquels R1 est une chaîne alkyle de 1 à 6 carbones,
- les groupements Y, identiques ou différents, représentent un groupement de formule 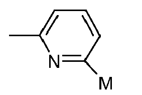 dans lequel M représente un hydrogène ; un halogène ; une chaîne alkyle, hydroxyalkyle, alkylamine ou alkyloxy de 1 à 6 carbones ; un groupement -COOH, -COOR1, -CONH₂, -CONHR1, dans lesquels R1 est tel que défini ci-avant,
- si A représente un groupement de formule 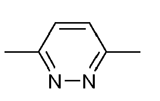
- les groupements Y, identiques, représentent un groupement de formule 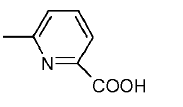 ou, les groupements Y, différents, représentent un groupement de formule 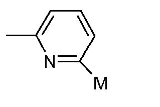 dans lequel M représente un hydrogène ; un halogène ; une chaîne alkyle, hydroxyalkyle, alkylamine ou alkyloxy de 1 à 6 carbones ; un groupement -COOH, -COOR1, -CONH₂, -CONHR1, dans lesquels R1 est tel que défini ci-avant,
• si n est un entier compris entre 2 et 4 bornes incluses,
- les groupements A, identiques ou différents, représentent un groupement 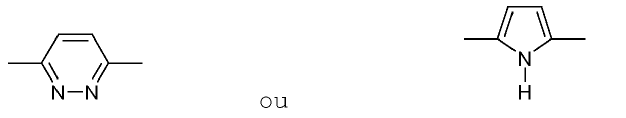
- les groupements Y, identiques ou différents, représentent un halogène ; un hydroxy ; un mercapto ; une chaîne alkyle, hydroxyalkyle, alkylamine ou alkyloxy de 1 à 6 carbones ; éventuellement cyclique ; un groupement -COOH, -COOR1,-CONH₂, -CONHR1, dans lesquels R1 est tel que défini précédemment; ou un groupement choisi parmi les groupes suivants : 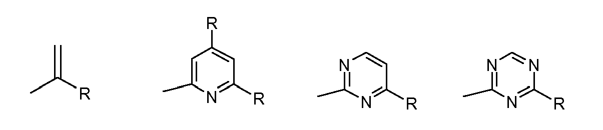 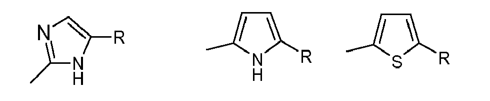 dans lesquels les groupes R, identiques ou différents, représentent un hydrogène ; une chaîne alkyle ou alkyloxy de 1 à 6 carbones ; un groupement -COOH, -COOR1, -CONH₂, -CONHR1, dans lesquels R1 est tel que défini ci- avant, à l'exception des composés suivants :
- 2,5-bis(pyridin-2-yl)pyrrole,
- 6,6'-bis(6-méthyl-pyridin-2-yl)-3,3'-bipyridazine,
- 6,6"'-bis-(6-méthyl-pyridin-2-yl)-[3,3':6',6":3",3"']quaterpyridazine,
- 6,6'-diméthoxy-3,3'-bipyridazine,
- 6,6'-dichloro-3,3'-bipyridazine.

2. Composé selon la revendication 1, **caractérisé en ce que**
• n=1, M représente un hydrogène ; un halogène ; une chaîne alkyle de 1 à 6 carbones ; ou un groupement -COOH ; de préférence
- 3-(2-carboxy-pyridin-6-yl)-6-(pyridin-2-yl)-pyridazine,
- 3,6-bis(2-carboxy-pyridin-6-yl)-pyridazine,
- 3-(6-méthyl-pyridin-2-yl)-6-(pyridin-2-yl)-pyridazine,
- 3-(2-bromo-pyridin-6-yl)-6-(pyridin-2-yl)-pyridazine. ou
- •n=2 et les groupements Y sont un groupement 2-pyridinyle éventuellement substitué, de préférence
- 5,5'-bis(6-méthyl-pyridin-2-yl)-2,2'-bipyrrole,
- 6,6'-di-(1-éthoxyvinyl)-3,3'-bipyridazine,
- 6,6'-bis(pyridin-2-yl)-3,3'-bipyridazine,
- 5,5'-bis(pyridin-2-yl)-2,2'-bipyrrole,
- 3-[5-(6-méthyl-pyridin-2-yl)-pyrrol-2-yl]-6-(6-méthyl-pyridin-2-yl)-pyridazine,
- 6-(pyridin-2-yl)-3-((5-pyridin-2-yl)-pyrrol-2-yl)-pyridazine,
- 6,6'-bis(4,6-diméthylpyridin-2-yl)-3,3'-bipyridazine,
- 5,5'-bis(4,6-diméthylpyridin-2-yl)-2,2'-bipyrrole),
- 6-(4,6-dimethylpyridin-2-yl)-3-((5-(4,6-diméthylpyridin-2-yl)-pyrrol-2-yl)-pyridazine).

3. Procédé de préparation des composés de formule 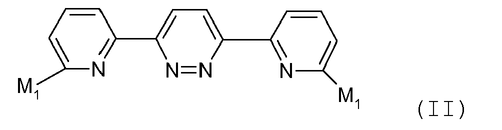 dans laquelle les substituants M₁, identiques ou différents, représentent un hydrogène ; un halogène ; une chaîne alkyle ou alkyloxy de 1 à 6 carbones ;
par couplage organocatalysé entre un composé de formule 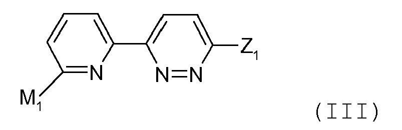 avec un composé de formule 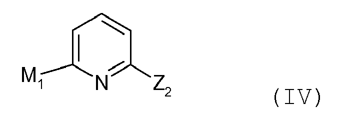 dans lesquelles, Z₁ et Z₂, différents, représentent soit un halogène, soit par exemple un groupement stannylé de formule SnB₃, dans lequel B représente une chaîne méthyle, butyle ou phényle; soit un borane; ou un dérivé zincique.

4. Procédé de préparation des composés de formule 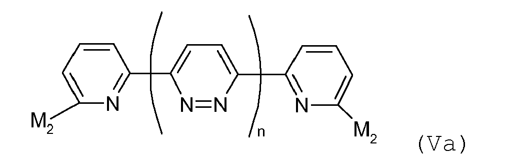 dans laquelle n est un nombre entier compris entre 1 et 4 bornes incluses ; au moins un des substituants M₂ est un groupement -COOH ; l'autre substituant pouvant être alternativement un hydrogène ; un halogène ; ou une chaîne alkyle ou alkyloxy de 1 à 6 carbones ; par oxydation du précurseur méthylé en présence d'un oxydant allylique ou aromatique tel que le dioxyde de sélénium ou l'oxyde de chrome.

5. Procédé de préparation des composés de formule 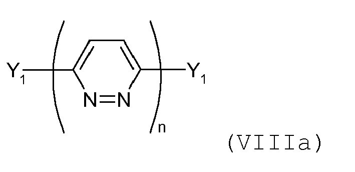
• si n = 1,
les groupes Y₁, différents, représentent un groupement de formule 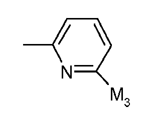 dans lequel M₃ représente un hydrogène ; une chaîne alkyle ou alkyloxy de 1 à 6 carbones,
• si n est un entier compris entre 2 et 4, bornes incluses,
les groupements Y₁, identiques ou différents, représentent une chaîne alkyle ou alkyloxy de 1 à 6 carbones ; ou un groupement choisi parmi les groupes suivants : 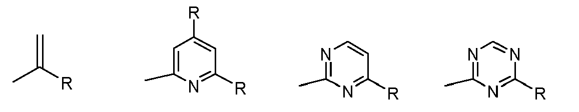 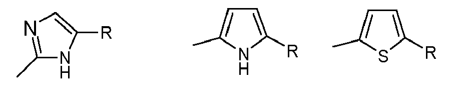 dans lesquels les groupements R, identiques ou différents, représentent un hydrogène ; une chaîne alkyle ou alkyloxy de 1 à 6 carbones ; ou un phényle,
par couplage organocatalysé, par exemple de Stille (Sn), de Nigishi (Zn), ou de Suzuki (B) dans des proportions allant de 1 :2 à 1 :3, entre un composé de formule 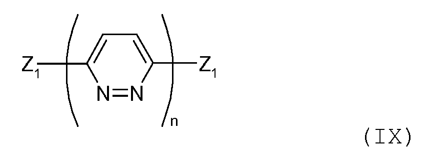 et un composé de formule Y₁-Z₂
dans laquelle Z₁, et Z₂, identiques ou différents, représentent soit un halogène, soit par exemple un groupement stannylé de formule SnB₃, dans lequel B représente une chaîne méthyle, butyle ou phényle, soit un borane; ou un dérivé zincique.

6. Procédé de couplage par voie électrochimique d'un halogénure pyridazinique **caractérisé en ce qu**'il s'agit
- d'un homocouplage d'halogéno pyridazines de formule 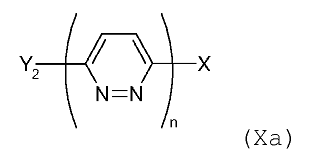 dans laquelle n est un nombre entier compris entre 1 et 2 bornes incluses, X est un halogène et Y₂ représente un halogène ; une chaîne alkyle ou alkyloxy de 1 à 6 carbones ; ou un groupement choisi parmi les groupes suivants : 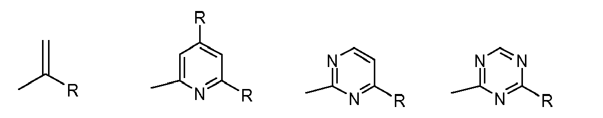 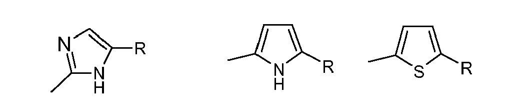 dans lesquels les groupes R, identiques ou différents, représentent un hydrogène ; une chaîne alkyle ou alkyloxy de 1 à 6 carbones ; ou un phényle ; les conditions de l'électrolyse étant les suivante :
- l'anode est constituée d'au moins 50% de fer,
- le milieu de l'électrolyse comporte du nickel, un élément choisi parmi les halogènes, et de la pyridine ou ses dérivés, ou
- d'un hétérocouplage par voie électrochimique d'un halogénure pyridazinique de formule 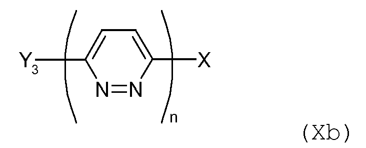 dans laquelle n est un nombre entier compris entre 1 et 2 bornes incluses, X est un halogène et Y₃ représente une chaîne alkyle ou alkyloxy de 1 à 6 carbones, ou un groupement choisi parmi les groupes suivants : 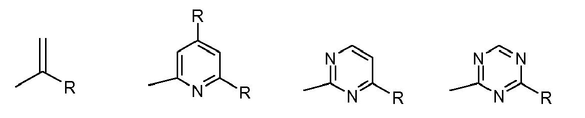 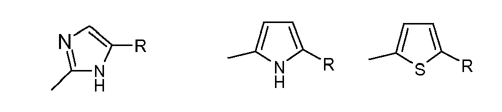 dans lesquels les groupes R, identiques ou différents, représentent un hydrogène, une chaîne alkyle ou alkyloxy de 1 à 6 carbones ou un phényle,
avec un halogénure de cycle aromatique de formule Ar-X dans laquelle, X tel que défini précédemment et Ar représente un cycle aromatique de 5 à 6 chaînons, éventuellement substitué, les conditions de l'électrolyse étant les suivantes :
- l'anode est constituée de fer,
- le catalyseur est choisi parmi les halogénures de nickel bipyridine.

7. Procédé de réduction en pyrrole d'un composé de formule 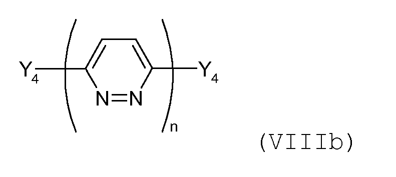 dans laquelle n est un nombre entier compris entre 2 et 4 bornes incluses, les groupements Y₄, identiques ou différents, représentent une chaîne alkyle ou alkyloxy de 1 à 6 carbones ; ou un groupement choisi parmi les groupes suivants : 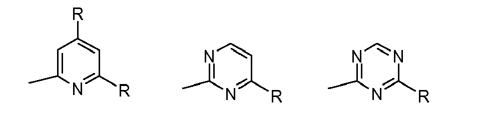 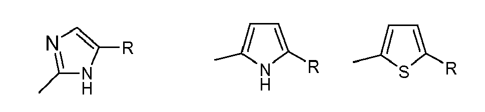 dans lesquels les groupes R, identiques ou différents, représentent un hydrogène, une chaîne alkyle ou alkyloxy de 1 à 6 carbones ou un phényle,
par voie électrochimique, par extrusion d'un atome d'azote sur un ou plusieurs cycle(s) pyridazinique(s), dans lequel les conditions de l'électrolyse sont les suivante :
- l'anode est une électrode à grande surface,
- le milieu de l'électrolyse est un milieu polaire donneur de proton.

8. Utilisation des composés selon l'une quelconque des revendications 1 à 3, comme ligands, pour la dépollution de cations en milieu liquide, pour former un matériau composé d'une organisation supramoléculaire, en optique linéaire.

9. Composé de formule 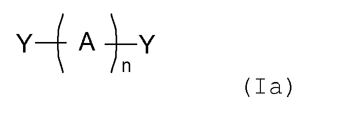 dans laquelle
• si n=1
- si A représente un groupement de formule 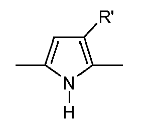 dans laquelle R' représente un hydrogène ; une chaîne alkyle, hydroxyalkyle, alkylamine, alkyloxy de 1 à 6 carbones ; un groupement -COOH, -COOR1, -CONH₂, -CONHR1, dans lesquels R1 est une chaîne alkyle de 1 à 6 carbones,
- les groupements Y, identiques ou différents, représentent un groupement de formule 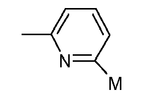 dans lequel M représente un hydrogène ; un halogène ; une chaîne alkyle, hydroxyalkyle, alkylamine ou alkyloxy de 1 à 6 carbones ; un groupement -COOH, -COOR1, -CONH₂, -CONHR1, dans lesquels R1 est tel que défini ci-avant,
- si A représente un groupement de formule 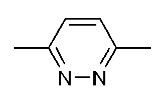
- les groupements Y, identiques, représentent un groupement de formule 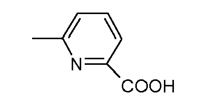 ou, les groupements Y, différents, représentent un groupement de formule 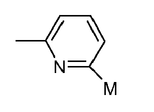 dans lequel M représente un hydrogène ; un halogène ; une chaîne alkyle, hydroxyalkyle, alkylamine ou alkyloxy de 1 à 6 carbones ; un groupement -COOH, -COOR1, -CONH₂, -CONHR1, dans lesquels R1 est tel que défini ci-avant,
- si n est un entier compris entre 2 et 4 bornes incluses,
- les groupements A, identiques ou différents, représentent un groupement 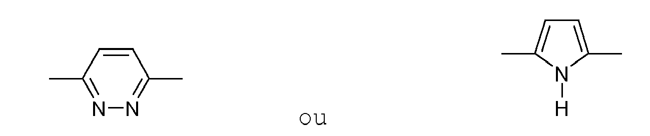
- les groupements Y, identiques ou différents, représentent un halogène ; un hydroxy ; un mercapto ; une chaîne alkyle ; hydroxyalkyle, alkylamine ou alkyloxy de 1 à 6 carbones ; éventuellement cyclique ; un groupement -COOH, -COOR1, -CONH₂, -CONHR1, dans lesquels R1 est tel que défini précédemment ; ou un groupement choisi parmi les groupes suivants : 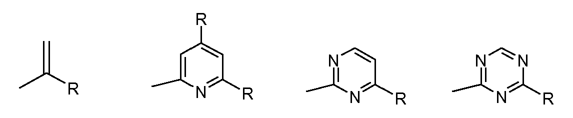 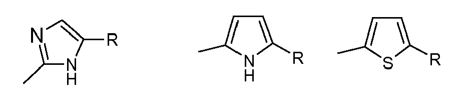 dans lesquels les groupes R, identiques ou différents, représentent un hydrogène ; une chaîne alkyle ou alkyloxy de 1 à 6 carbones ; un phényle ; un groupement -COOH, -COOR1,
- CONH₂, -CONHR1, dans lesquels R1 est tel que défini ci-avant,
pour une utilisation comme médicament.

10. Composé selon la revendication 9, **caractérisé en ce que** n est égal à 2 et Y est un groupement 2-pyridinyle éventuellement substitué ; ou n=1 et les groupements M représentent un halogène ; une chaîne alkyle de 1 à 6 carbones ; ou un groupement -COOH.

11. Composé selon la revendication 10, **caractérisé en ce qu**'il est choisi parmi le groupe constitué de :
- 5,5'-bis(6-méthyl-pyridin-2-yl)-2,2'-bipyrrole,
- 6, 6'-di-(1-éthoxyvinyl)-3,3'-bipyridazine,
- 6,6'-bis(pyridin-2-yl)-3,3'-bipyridazine,
- 5,5'-bis(pyridin-2-yl)-2,2'-bipyrrole,
- 3-[5-(6-méthyl-pyridin-2-yl)-pyrrol-2-yl]-6-(6-méthyl-pyridin-2-yl)-pyridazine,
- 6,6'-bis(6-méthyl-pyridin-2-yl)-3,3'-bipyridazine,
- 6,6"'-bis-(6-méthyl-pyridin-2-yl)-[3,3':6',6":3",3'"]quaterpyridazine,
- 6-(pyridin-2-yl)-3-((5-pyridin-2-yl)-pyrrol-2-yl)-pyridazine,
- 6,6'-bis(4,6-diméthylpyridin-2-yl)-3,3'-bipyridazine,
- 5,5'-bis(4,6-diméthylpyridin-2-yl)-2,2'-bipyrrole),
- 6-(4,6-diméthylpyridin-2-yl)-3-((5-(4,6-diméthylpyridin-2-yl)-pyrrol-2-yl)-pyridazine),
- 3-(2-carboxy-pyridin-6-yl)-6-(pyridin-2-yl)-pyridazine,
- 3,6-bis(2-carboxy-pyridin-6-yl)-pyridazine,
- 3-(6-méthyl-pyridin-2-yl)-6-(pyridin-2-yl)-pyridazine,
- 3-(2-bromo-pyridin-6-yl)-6-(pyridin-2-yl)-pyridazine,
- 2,5-bis(pyridin-2-yl)pyrrole,

12. Composé selon l'une quelconque des revendications 10 ou 11, pour utilisation comme médicament destiné à traiter le cancer, les maladies parasitaires, les infections bactériennes, ou comme médicament destiné à la radio-immunothérapie.

13. Composé comme médicament antiparasitaire **caractérisé en ce qu**'il s'agit de la 6,6'-bis(6-methyl-pyridin-2-yl)-3,3'-bipyridazine ou de la 6,6'-bis(pyridn-2-yl)-3,3'-bipyridazine pour traiter les Leishmanies, notamment dues à *L. mexicana.*
